# EUROPEAN PATENT SPECIFICATION

(11) **EP 0 904 275 B1**
(45) Date of publication and mention of the grant of the patent: **17.05.2006**
(21) Application number: 96935220.2
(22) Date of filing: 18.10.1996
(51) Int. Cl.: C07H 21/00, C07F 9/6584, C07F 9/141

(54) **PREPARATION OF PHOSPHOROTHIOATE OLIGOMERS**
VERFAHREN ZUR HERSTELLUNG VON PHOSPHOROTHIOAT-OLIGOMEREN
PREPARATION D'OLIGOMERES DE PHOSPHOROTHIOATE

(30) Priority: 20.10.1995 US 546198
(43) Date of publication of application: 31.03.1999
(73) Proprietor: McGill University, Montreal, Quebec H3A 2A7 (CA)
(72) Inventor: JUST, George, Ile Cadieux, Quebec J7V 8P3 (CA); XIN, Zhili, Montreal, Quebec H2C 3G6 (CA); MARSAULT, Eric, Montreal, Quebec H2J 3B1 (CA); JIN, Yi, Montreal, Quebec H2X 2C1 (CA); WANG, Jianchao, Montreal, Quebec H2X 2E6 (CA)
(74) Representative: Hallybone, Huw George
(86) International application number: PCT/IB1996/001185
(87) International publication number: WO 1997/014710

(56) References cited:
- WO-A-88/02004
- US-A- 3 846 374
- JOURNAL OF ORGANIC CHEMISTRY, vol. 60, no. 15, 1995, EASTON US, pages 4767-4773, XP000617206 Y.HUANG ET AL.: "Study of the Conformational Equilibria of 2-Z-Methyl-1,3,2-oxazaphosphorinanes. Steric and Stereoelectronic Influences on the Orientation of the Me2N Substituent on Three-Coordinate Phosphorus."
- JOURNAL OF ORGANOMETALLIC CHEMISTRY, vol. 336, no. 1-2, 1987, LAUSANNE CH, pages 237-247, XP000616536 E.E.NIFANTYEV ET AL.: "1,3,2-Oxazaphosphorinanes in Rhodium (I) Complexes."
- PHOSPHORUS AND SULFUR, vol. 7, 1979, pages 235-240, XP000617346 G.HALL ET AL.: "Platinum (II) Complexes of P(III) Cyclophosphamide Derivatives."
- CHEMICAL ABSTRACTS, vol. 90, no. 22, 28 May 1979 Columbus, Ohio, US; abstract no. 177623q, I.NURETDINOV ET AL.: "Nuclear Quadrupole Resonance of Chlorides and Amides of Phosphorus (III) Cyclic Acids." page 612; column 1; XP002024459 & ZH. FIZ. KHIM., vol. 53, no. 1, 1979, pages 126-129,
- ANGEWANDTE CHEMIE INTERNATIONAL EDITION., vol. 33, 18 April 1994, WEINHEIM DE, pages 709-722, XP000576146 W.STEC ET AL.: "Stereocontrolled Synthesis of Oligo(nucleoside phosphorothioate)s." cited in the application
- TETRAHEDRON LETTERS, vol. 37, no. 7, 1996, OXFORD GB, pages 969-972, XP000617272 Z.XIN ET AL.: "Diastereoselective Synthesis of Phosphite Triesters"
- TETRAHEDRON LETTERS, vol. 37, no. 7, 1996, OXFORD GB, pages 973-976, XP000617271 Y.JIN ET AL.: "A Stereoselective Synthesis of Dinucleotide Phosphorothioates, using Chiral Phosphoramidites as Intermediates."
- TETRAHEDRON LETTERS, vol. 37, no. 7, 1996, OXFORD GB, pages 977-980, XP000617270 E.MARSAULT ET AL.: "Diastereoselective Synthesis of Phosphite Triesters Through a New Bicyclic Intermediate."

## Description

### FIELD OF THE INVENTION

This invention relates to methods for the preparation of diastereomerically enriched phosphorothioate linked oligonucleotides, and to intermediates useful in their preparation. This invention also relates to sequence-specific phosphorothioate oligonucleotides having chiral phosphorus linkages and to a novel chemical synthesis of these and other oligonucleotides.

### BACKGROUND OF THE INVENTION:

It is well known that most of the bodily states in multicellular organisms, including most disease states, are effected by proteins. Such proteins, either acting directly or through their enzymatic or other functions, contribute in major proportion to many diseases and regulatory functions in animals and man. Classical therapeutics has generally focused upon interactions with such proteins in efforts to moderate their disease-causing or disease-potentiating functions. In newer therapeutic approaches, modulation of the actual production of such proteins is desired. By interfering with the production of proteins, the maximum therapeutic effect might be obtained with minimal side effects. It is the general object of such therapeutic approaches to interfere with or otherwise modulate gene expression which would lead to undesired protein formation.

One method for inhibiting specific gene expression is with the use of oligonucleotides. Oligonucleotides complementary to a specific target messenger RNA (mRNA) sequence are used. Several oligonucleotides are currently undergoing clinical trials for such use.

Transcription factors interact with doublestranded DNA during regulation of transcription. Oligonucleotides can serve as competitive inhibitors of transcription factors to modulate the action of transcription factors. Several recent reports describe such interactions (*see,* Bielinska, *et. al., Science* **1990,** *250,* 997-1000; and Wu, *et al., Gene* **1990,** *89,* 203-209.)

Oligonucleotides also have found use in diagnostic tests. Such diagnostic tests can be performed using biological fluids, tissues, intact cells or isolated cellular components. As with the above gene expression inhibition, diagnostic use can take advantage of an oligonucleotide's ability to hybridize with a complementary strand of nucleic acid. Hybridization is the sequence specific hydrogen bonding of oligonucleotides via Watson-Crick and/or Hoogsteen base pairs to RNA or DNA. The bases of such base pairs are said to be complementary to one another.

Oligonucleotides are also widely used as research reagents. They are useful for understanding the function of many other biological molecules as well as in the preparation of such other biological molecules. One particular use, the use of oligonucleotides as primers in the reactions associated with polymerase chain reaction (PCR), has been the cornerstone for the establishment of an ever expanding commercial business. The use of such PCR reactions has seemingly "exploded" as more and more use of this very important biological tool is made. The uses of PCR have extended into many areas in addition to those contemplated by its Nobel laureate inventor. Examples of such new areas include forensics, paleontology, evolutionary studies and genetic counseling to name just a few. Primers are needed for each of these uses. Oligonucleotides, both natural and synthetic, serve as the primers.

Oligonucleotides also are used in other laboratory procedures. A number of these uses are described in common laboratory manuals such as *Molecular Cloning, A Laboratory Manual,* Second Ed., J. Sambrook, *et al.,* Eds., Cold Spring Harbor Laboratory Press, **1989**; and *Current Protocols In Molecular Biology,* F. M. Ausubel, *et. al.,* Eds., Current publications, 1993. Such uses include Synthetic Oligonucleotide Probes, Screening Expression Libraries with Antibodies and Oligonucleotides, DNA Sequencing, In Vitro Amplification of DNA by the Polymerase Chain Reaction and Site-directed Mutagenesis of Cloned DNA from Book 2 of *Molecular Cloning, A Laboratory Manual, ibid.* and DNA-Protein Interactions and The Polymerase Chain Reaction from Vol. 2 of *Current Protocols In Molecular Biology, ibid..*

To supply the users of oligonucleotides, many scientific journals now contain advertisements for either oligonucleotide precursors or for custom-synthesized oligonucleotides. This has become an important commercial use of oligonucleotides. Oligonucleotides can be synthesized to have properties that are tailored for the desired use. Thus, a number of chemical modifications have been introduced into oligonucleotides to increase their usefulness in diagnostics, as research reagents, and as therapeutic entities. These modifications are designed, for example, to increase binding to a target nucleic acid strand, to assist in identification of the oligonucleotide or an oligonucleotide-target complex, to increase cell penetration, to provide stability against nucleases and other enzymes that degrade or interfere with the structure or activity of the oligonucleotides, to provide a mode of disruption (terminating event) once sequence-specifically bound to a target, or to improve the pharmacokinetic properties of the oligonucleotides.

Since they exist as diastereomers, phosphorothioate, methylphosphonate, phosphotriester, phosphoramidate and other phosphorus oligonucleotides synthesized using known, automated techniques result in mixtures of Rp and Sp diastereomers at the individual phosphorothioate, methylphosphonate, phosphotriester, phosphoramidate or other phosphorus linkages. Thus, a 15-mer oligonucleotide containing 14 asymmetric linkages has 2¹⁴, i.e. 16,384, possible stereoisomers. It is possible that oligomers having diastereomerically enriched linkages could possess advantages in hybridizing to a target mRNA or DNA. Accordingly, there is a need for such oligomers.

Miller, P.S., McParland, *K.B.,* Jayaraman, K., and Ts'o, P.O.P (1981), *Biochemistry,* 20:1874, found that small di-, tri- and tetramethylphosphonate and phosphotriester oligonucleotides hybridize to unmodified strands with greater affinity than natural phosphodiester oligonucleotides. Similar increased hybridization was noted for small phosphotriester and phosphoramidate oligonucleotides; Koole, L.H., van Genderen, M.H.P., Reiners, R.G., and Buck, H.M. (1987), *Proc. K. Ned. Adad. Wet.,* 90:41; Letsinger, R.L., Bach, S.A., and Eadie, J.S. (1986), *Nucleic Acids Res.,* 14:3487; and Jager, A., Levy, M.J., and Hecht, S.M. (1988), *Biochemistry,* 27:7237. The effects of the diastereomers of undefined stereochemistry on hybridization becomes even more complex as chain length increases.

Bryant, F.R. and Benkovic, S.J. (1979), *Biochemistry,* 18:2825 studied the effects of diesterase on the diastereomers of ATP. Published patent application PCT/US88/03634 discloses dimers and trimers of 2',5'- linked diastereomeric adenosine units. Niewiarowski, W., Lesnikowski, Z.J., Wilk, A., Guga, P., Okruszek, A., Uznanski, B., and Stec, W. (1987), *Acta Biochimica Polonia,* 34:217, synthesized dimers of thymidine having high diastereomeric excess, as did Fujii, M., Ozaki, K., Sekine, M., and Hata, T. (1987), *Tetrahedron,* 43:3395.

A study of the conformations of a series of 1,3,2-oxazaphosphorinanes have been reported by Y. Huang *et al.,* J. Org. Chem. (1995) 60, 4767-4773.

Various 1,3,2-oxazaphosphorinanes have been discussed by E.E. Nifantyev *et al*., J. Organomet. Chem. (1987) 336 (1-2), 237-247, and by I. A. Nuretdinov *et al*., Chem. Abs. 90, 177623q.

Stec, W.J., Zon, G., and Uznanski, B. (1985), *J*. *Chromatography,* 326:263, have reported the synthesis of certain mixtures of phosphorothioates or methyphosphonate oligonucleotides and have separated them by chromatography. However, they were only able to separate the diastereomers of certain small oligomers having a limited number of diastereomerically pure phosphorus linkages.

In a preliminary report, J.W. Stec, *oligonucleotides as antisense inhibitors of gene expression: Therapeutic implications,* meeting abstracts, June 18-21, 1989, noted that a non-sequence-specific thymidine homopolymer octamer -- i.e. a (dT)₈-mer, having "all-except-one" Rp configuration methylphosphonate linkages -- formed a thermodynamically more stable hybrid with a 15-mer deoxyadenosine homopolymer -- i.e. a d(A)₁₅-mer -- than did a similar thymidine homopolymer having "all-except-one" Sp configuration methylphosphonate linkages. The hybrid between the "all-except-one" Rp (dT)₈-mer and the d(A)₁₅-mer had a Tm of 38°C while the Tm of the "all-except-one" Sp (dT)₈-mer and the d(A)₁₅-mer was < 0°C. The hybrid between a (dT)₈-mer having natural phosphodiester linkages, i.e. octathymidylic acid, and the d(A)₁₅-mer was reported to have a Tm of 14°C. The "all-except-one" thymidine homopolymer octamers were formed from two thymidine methylphosphonate tetrameric units with high diastereomeric excess linked by a natural phosphodiester linkage.

Six or more nucleotides units are generally necessary for an oligonucleotide to be of optimal use in applications involving hybridization. It is often preferred to have even more nucleoside units for best performance, often as many as 10 to 30. Because it has not been possible to stereochemically resolve more than two or three adjacent phosphorus linkages, the effects of induced chirality in the phosphorus linkages of chemically synthesized oligonucleotides has not been well assessed heretofore.
This is because with few limited exceptions, the sequence-specific phosphorothioate, methylphosphonate, phosphotriester or phosphoramidate oligonucleotides obtained utilizing known automated synthetic techniques have been mixtures with no diastereomeric excess.

Some aspects of the use of enzymatic methods to synthesize oligonucleotides having chiral phosphorus linkages have been investigated. Burgers, P.M.J. and Eckstein, F. (1979), *J. Biological Chemistry,* 254:6889; and Gupta, A., DeBrosse, C., and Benkovic, S.J. (1982) *J*. *Bio. Chem.,* 256:7689 enzymatically synthesized diastereomerically pure polydeoxyadenylic acid having phosphorothioate linkages. Brody, R.S. and Frey, P.S. (1981), *Biochemistry,* 20:1245; Eckstein, F. and Jovin, T.M. (1983), *Biochemistry,* 2:4546; Brody, R.S., Adler, S., Modrich, P., Stec, W.J., Leznikowski, Z.J., and Frey, P.A. (1982) *Biochemistry, 21:* 2570-2572; and Romaniuk, P.J. and Eckstein, F. (1982) *J*. *Biol. Chem.* 257:7684-7688 all enzymatically synthesized poly TpA and poly ApT phosphorothioates while Burgers, P.M.J. and Eckstein, F. (1978) *Proc. Natl. Acad. Sci. USA,* 75: 4798-4800 enzymatically synthesized poly UpA phosphorothioates. Cruse, W.B.T., Salisbury, T., Brown, T., Cosstick, R. Eckstein, F., and Kennard, O. (1986), *J*. *Mol. Biol.,* 192:891, linked three diastereomeric Rp GpC phosphorothioate dimers via natural phosphodiester bonds into a hexamer. Most recently Ueda, T., Tohda, H., Chikazuni, N., Eckstein, R., and Watanabe, K. (1991) *Nucleic Acids Research,* 19:547, enzymatically synthesized RNA's having from several hundred to ten thousand nucleotides incorporating Rp linkages of high diastereomeric excess. Enzymatic synthesis, however, is disadvantageous in that it depends on suitable polymerases that may or may not be available, especially for modified nucleoside precursors.

As reviewed by W. J. Stec and A. Wiek (1994), *Angew. Chem. Int*. Ed. English 33:709, the oxathiaphospholane method has been successful for the preparation of phosphorothioates with defined stereochemistry. However, it suffers from disadvantages, such as the non-trivial preparation of diastereomerically pure oxathiaphospholane, and the difficulty in synthesizing and isolating satisfactorily pure oligomers longer than 12-mers.

It would therefore be of great advantage to provide oligonucleotides having phosphorus linkages with controlled stereochemistry.

### OBJECTS OF THE INVENTION:

It is one object of this invention to provide sequence-specific oligonucleotides having chirally pure phosphorothioate linkages with high diastereomeric excess.

Another object is to provide phosphorus-linked oligonucleotides having substantially all Rp or all Sp linkages.

A further object is to provide research and diagnostic materials for assaying bodily states in animals, especially diseased states.

It is yet another object to provide new methods for synthesizing sequence-specific oligonucleotides having chirally pure phosphorothioate linkages, and useful intermediates therefor.

### SUMMARY OF THE INVENTION:

The present invention provides stereoselective methods for preparing sequence-specific oligonucleotides having chiral phosphorus linkages. In certain preferred embodiments, these methods comprise the steps of:
- reacting a first synthon of Formula I: 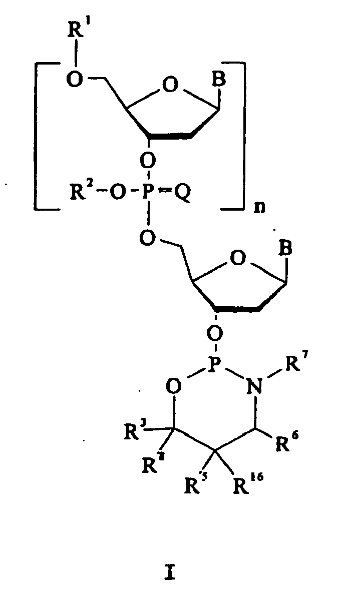 wherein:
   Q is independently O or S;
   R¹ is a hydroxyl protecting group;
   R² is a chiral auxiliary of formula -C(R⁸)R³-C(R¹⁶)R⁵-CHR⁶-NHR⁷;
   R³ is hydrogen, alkyl, cyanomethyl, monohalomethyl, dihalomethyl, trihalomethyl, -CH₂R₄, -CH₂Si (R⁴)_{3'} or -CH₂-SOₖR⁴ where k is 0, 1 or 2;
   R⁴ is independently alkyl, aryl, aralkyl or alkaryl having up to 15 carbon atoms, -N(R₇₀)-C(=O)-R₇₁, -S-C (=O)-R₇₀, or -O-C(=O)-O-N(R₇₀)(R₇₁) ;
      R₇₀ and R₇₁ are each independently alkyl, α-halo substituted alkyl, aralkyl, α-halo substituted aralkyl, or aryl substituted with up to three electronegative groups;
   R⁵ is H, -CN, -Si(R⁴)₃, SOₖR⁴ or halogen;
   or R⁸ and R¹⁶ are each H, and R³ and R⁵, together, form one of the structures: 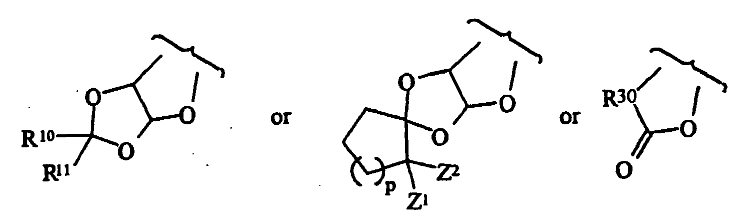 wherein:
      R¹⁰ and R¹¹ are H, alkyl having from 1 to about 10 carbons, -CH₂C(=O)OR²², -CH₂CN, -CH₂Si(CH₃)₃, or o- or *p*-C₆H₄-R²¹;
      R²¹ is hydrogen, -O-C(=O)CH₃, alkoxy having from 1 to about 10 carbons, -NO₂, or -N(R²²)₂;
      R²² is independently H or alkyl having from one to about 10 carbon atoms;
      p is 1 or 2;
      Z¹ and Z² are independently halogen, -CN, -Si(CH₃)₃, or -C(=O)OR²²;
      R³⁰ is hydrogen, -O-C(=O)CH₃, alkoxy having from 1 to about 10 carbons, or -O-Si(R₄)₃;
   R⁶ is H, alkyl or aralkyl having up to 15 carbon atoms;
   or R⁵ and R⁶, together with the atoms to which they are attached, form a 5 or 6 membered ring;
   R⁷ is alkyl or aralkyl having up to 15 carbon atoms;
   or R⁶ and R⁷, together, form one of the structures 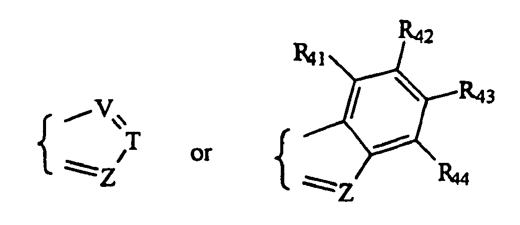
   wherein V, T, and Z are independently CH or N;
   R₄₁ , R₄₂, R₄₃*,* and R₄₄ are each independently H or an electronegative group;
   R⁸ is H or methyl;
   R¹⁶ is H, alkyl or aralkyl having up to 15 carbon atoms;
   B is a nucleobase; and
   n is an integer from 0 to 50;
   with a second synthon of Formula II: 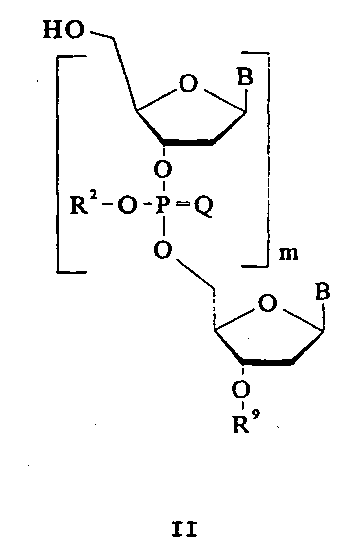 wherein:
   R⁹ is a hydroxyl protecting group or a linker connected to a solid support; and
   m is an integer from 0 to 50;
   for a time and under reaction conditions effective to form a third synthon of Formula III: 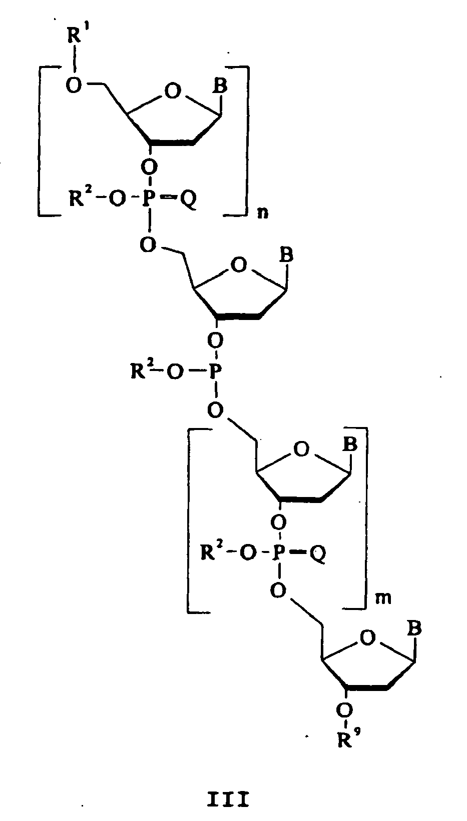 and
- contacting said third synthon with a sulfurizing agent to form an oligomer of Formula IV: 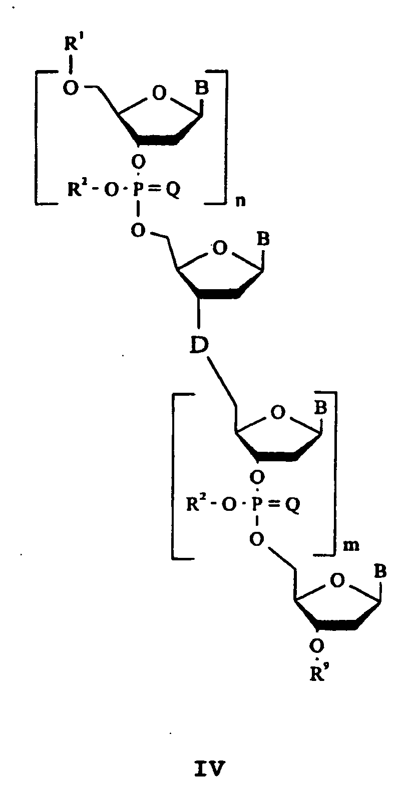
wherein D is said phosphorothioate linkage having the formula:

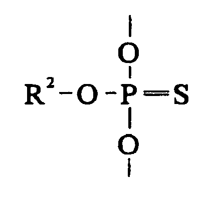

In preferred embodiments, said phosphorothioate linkage is diastereomerically enriched. In other preferred embodiments about 75% of the phosphorothioate linkage is in a single stereoisomeric form. In further preferred embodiments about 85% of the phosphorothioate linkage is in a single stereoisomeric form. In especially preferred embodiments about 95% of the phosphorothioate linkage is in a single stereoisomeric form. Most preferably, the phosphorothioate linkage is in a single stereoisomeric form, substantially free of other stereoisomeric forms. Preferably, the first synthon is in a single stereoisomeric form, substantially free of other stereoisomeric forms.

In some preferred embodiments n is 0. In further preferred embodiments, R¹ groups are subsequently removed to yield new second synthons for iterative synthesis, and chiral auxiliaries are removed after iterative synthesis is completed. In preferred embodiments of the present methods the oligomer of Formula IV contains a plurality of phosphorothioate linkages.

Preferably, first and second synthons are reacted at a temperature of from about -20°C to about 40°C, with from about -15°C to about 0°C being more preferred.

In some preferred embodiments the first synthon is formed by reacting a compound of Formula V: 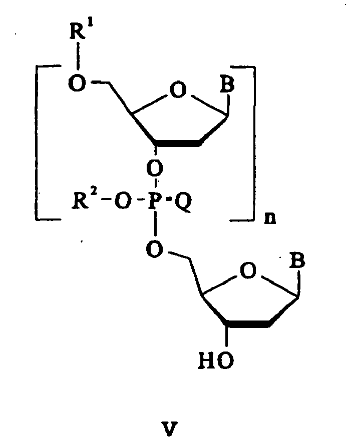 with an azaphospholane of Formula VIa: 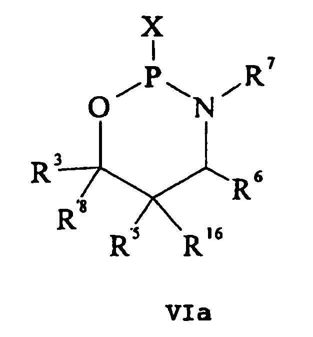 wherein R³-R⁸ are as defined above; and X is halogen, dialkylamino, imidazole, triazole or substituted phenoxy wherein said substituents are electron withdrawing, preferably halogen or nitro.

In some embodiments the azaphospholane described above is produced by reacting a reagent of formula HO-C(R⁸)R³-C(R¹⁶)R⁵-CHR⁶-NHR⁷ and a phosphorus trihalide, phosphorus tri(dialkylamide), phosphorus triphenoxide or phosphorus triimidazolide.

In more preferred embodiments the first synthon is formed by reacting a compound of Formula VII: 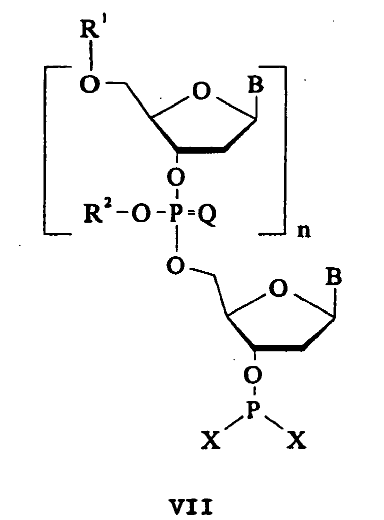 and a γ-amino alcohol of formula HO-C(R⁸)R³-C(R¹⁶)R⁵-CHR⁶-NHR⁷. Preferably, X is chlorine, dialkylamino or diphenoxy, and said reaction is stereoselective. It is especially preferred that the first synthon is in a single stereoisomeric form, substantially free of other stereoisomeric forms.

In some preferred embodiments the reaction of first and second synthons is performed in the presence of a catalyst, said catalyst preferably having one of the Formulas VIII or IX: 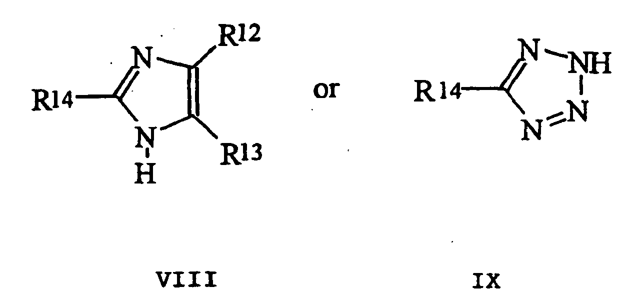 wherein:
R¹² and R¹³ are independently hydrogen, halogen, cyano, nitro, alkyl having from one to 10 carbons, substituted alkyl having from one to 10 carbons, an ester group, or R¹² and R¹³ together with the carbon atoms to which they are attached, form a substituted or unsubstituted phenyl ring where said substituents are electron withdrawing; and
R¹⁴ is hydrogen, halogen, cyano, nitro, thio, alkyl having from one to 10 carbons, substituted alkyl having from one to 10 carbons, norbornyl, substituted norbornyl, aryl, substituted aryl wherein said substituents are electron withdrawing, or has the formula: 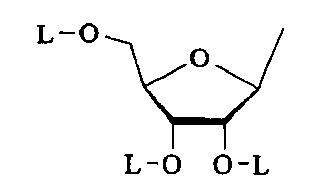
wherein L is protecting group.

In some preferred embodiments R¹⁴ is halogen or nitro, preferably bromine, and R¹² and R¹³ are each halogen or each cyano, with cyano being especially preferred.

Other preferred embodiments R¹⁴ has one of the formulas: 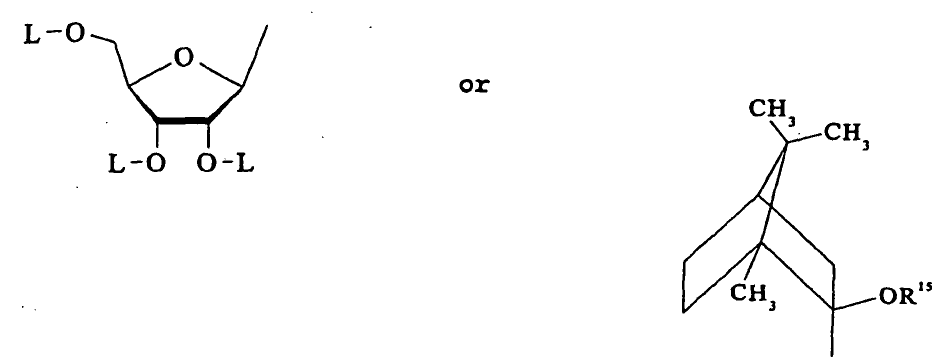 wherein R¹⁵ is H, methyl, trialkylsilyl or acetyl.

In some preferred embodiments of the method R³ is cyanomethyl or -CH₂-SOₖR⁴ where k is 0, 1 or 2, and R⁷ is lower alkyl or aralkyl.

In further preferred embodiments said first synthon has one of the Formulas Xa, XIa, XIIa, XIIIa or XXa: 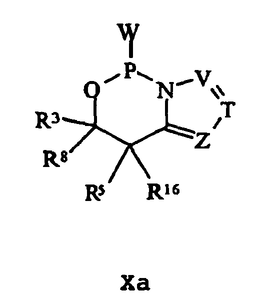 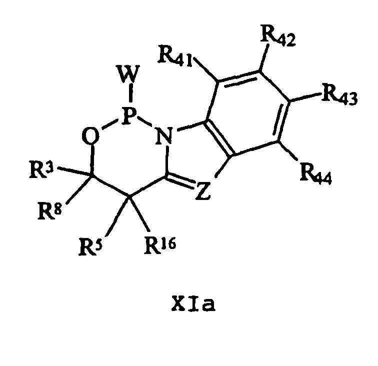 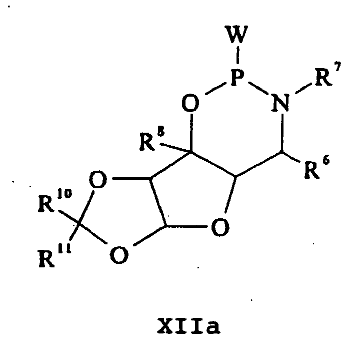 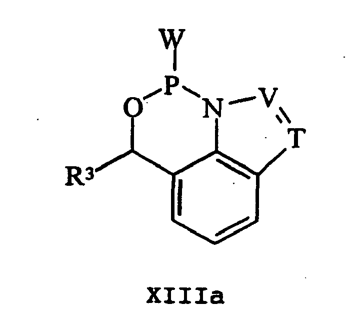 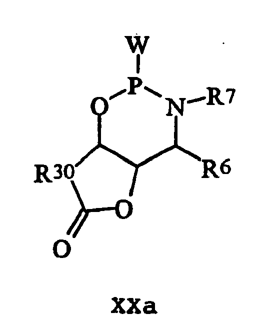 wherein W has the formula: 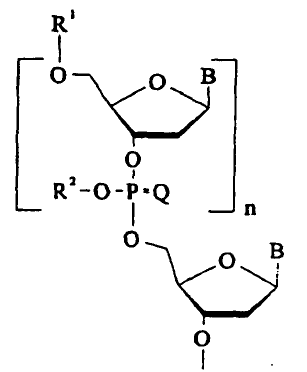 and R¹-R¹⁶, V, T and Z are as defined above.

Other preferred first synthons have the Formula Xb or Xc:

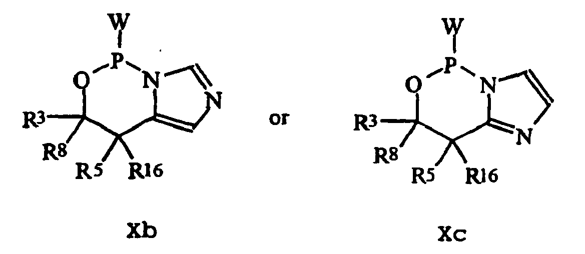

More preferred first synthons have the Formula XVIIa or XVIIIa:

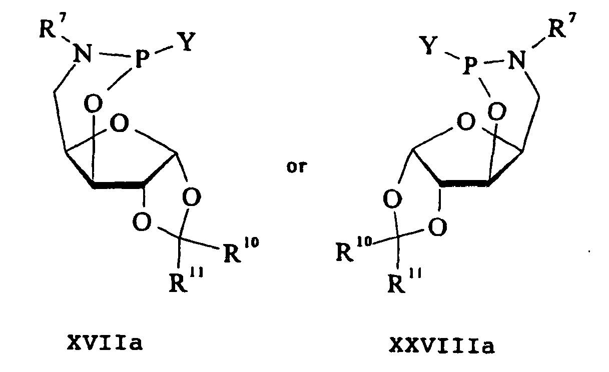

Particularly preferred first synthons have the Formula XIVa:

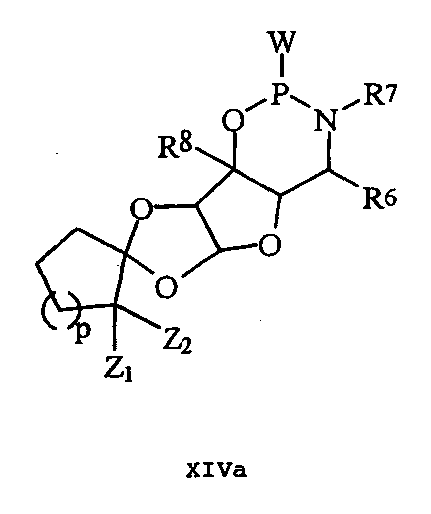

Especially preferred first synthons have the Formula XVa or XVIa:

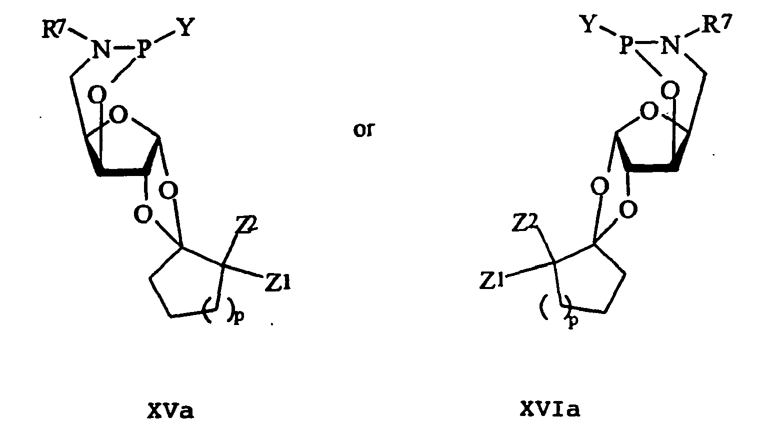

In preferred embodiments of the methods of the invention R¹ groups are removed from the oligomers, thus creating new second synthons for further iterative synthesis.

Also provided according to the invention are phosphorothioate oligomers produced by the method of claim 1, and azaphospholanes having Formula VIb:

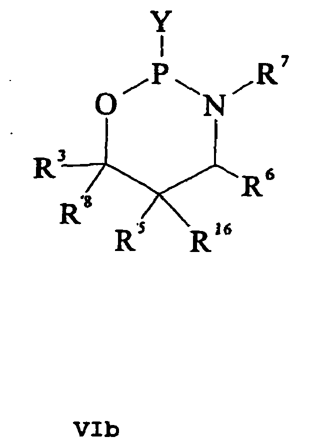

In preferred embodiments of the invention, 75% of said azaphospholanes having Formula VIb are in a single stereoisomeric form, with 85% being more preferred, and 95% being particualrly preferred. In especially preferred embodiments, the azaphospholanes having Formula VIb are in a single stereoisomeric form, substantially free of other stereoisomeric forms.

In preferred embodiments the azaphospholane has one of the Formulas Xb, XIb, XIIb, XIIIb, Xd, Xe or XXb:

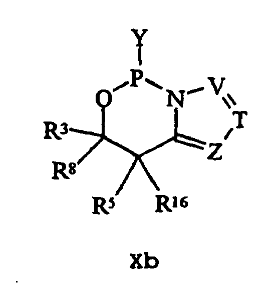

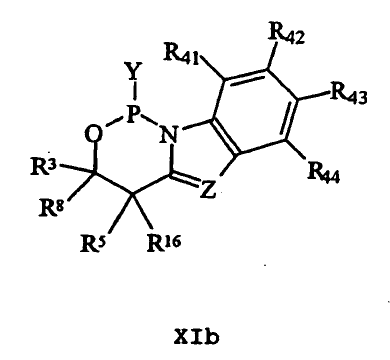

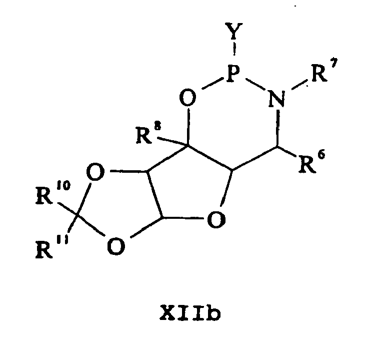

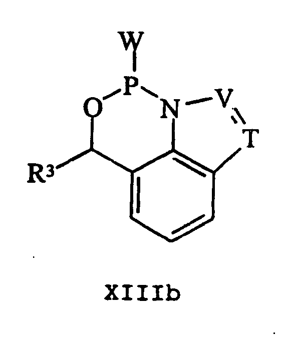

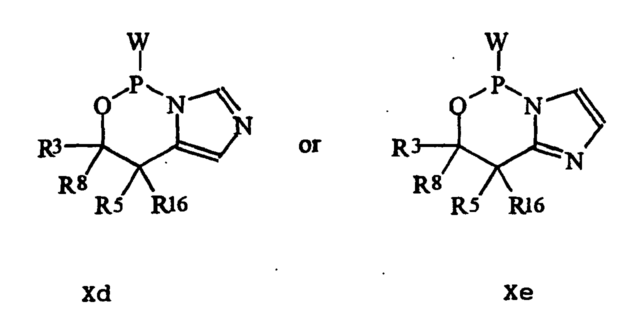

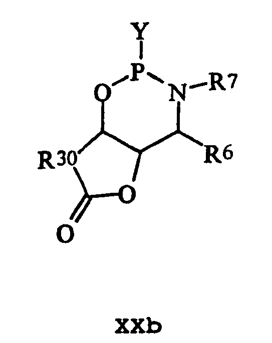

In other preferred embodiments the azaphospholane has the Formula XVIIb or XVIIIb:

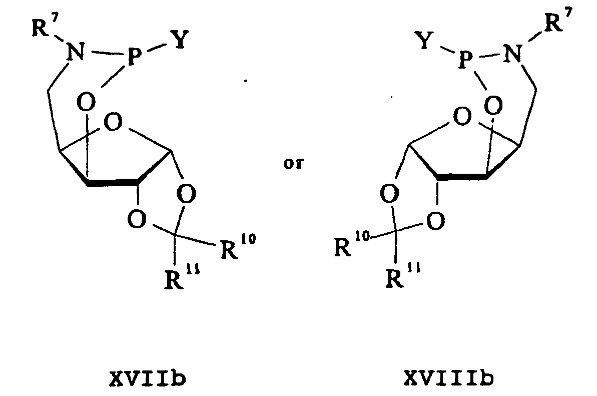

In some particularly preferred embodiments the azaphosphalane has the Formula XIVb:

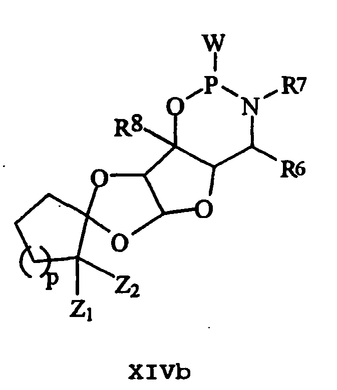

Especially preferred embodiments the azaphospholane has the Formula XVb or XVIb:

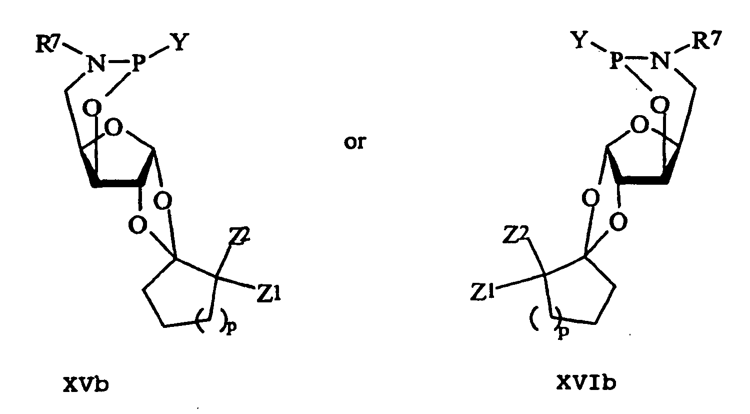

Also provided in accordance with the invention are oligomeric compounds comprising a phosphite linkage having the Formula XXX:

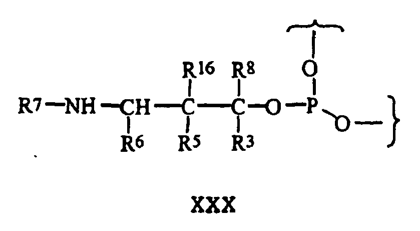

In preferred embodiments of the invention, 75% of said phosphosphite linkage is in a single stereoisomeric form, with 85% being more preferred, and 95% being particualrly preferred. In especially preferred embodiments, the phosphosphite linkage is in a single stereoisomeric form, substantially free of other stereoisomeric forms.

### DETAILED DESCRIPTION OF THE PREFERRED EMBODIMENTS

The present invention is directed to methods for the synthesis of phosphorothioate compounds having diastereomerically enriched phosphorothioate linkages, and to intermediates useful in their preparation.

In one aspect, the invention provides methods for the preparation of phosphorothioate linkages comprising the steps of reacting a first synthon of Formula I: 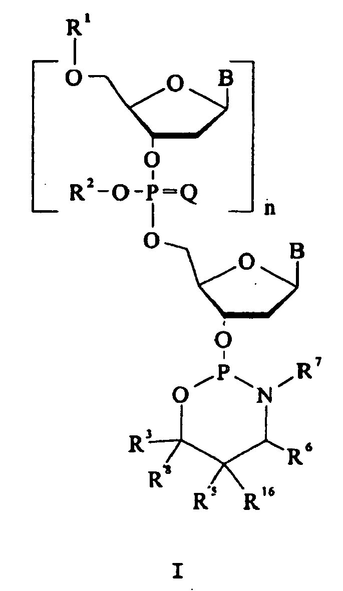 wherein:
Q is independently O or S;
R¹ is a hydroxyl protecting group;
R² is a chiral auxiliary of formula -C(R⁸)R³-C(R¹⁶)R⁵-CHR⁶-NHR⁷;
R³ is hydrogen, alkyl, cyanomethyl, monohalomethyl, dihalomethyl, trihalomethyl, -CH₂R₄, -CH₂Si(R⁴)₃, or -CH₂-SOₖR⁴ where k is 0, 1 or 2;
R⁴ is independently alkyl, aryl, aralkyl or alkaryl having up to 15 carbon atoms, -N(R₇₀)-C(=O)-R₇₁, -S-C(=O)-R₇₀, or -O-C(=O)-O-N(R₇₀)(R₇₁);
   R₇₀ and R₇₁ are each independently alkyl, α-halo substituted alkyl, aralkyl, α-halo substituted aralkyl, or aryl substituted with up to three electronegative groups;
R⁵ is H, -CN, -Si(R⁴)₃, SOₖR⁴ or halogen;
or R⁸ and R¹⁶ are each H, and R³ and R⁵, together, form one of the structures 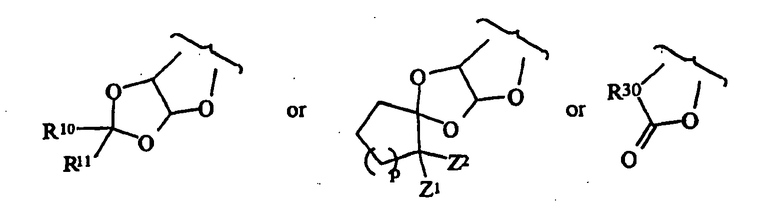 wherein:
   R¹⁰ and R¹¹ are H, alkyl having from 1 to about 10 carbons, -CH₂C(=O)OR²², -CH₂CN, -CH₂Si(CH₃)₃, or *o*- or *p*-C₆H₄-R²¹;
   R²¹ is hydrogen, -O-C(=O)CH₃, alkoxy having from 1 to about 10 carbons, -NO₂, or -N(R²²)₂;
   R²² is independently H or alkyl having from one to about 10 carbon atoms;
   p is 1 or 2;
   Z¹ and Z² are independently halogen, CN, -Si(CH₃)₃, and -C(=O)OR²²;
   R³⁰ is hydrogen, -O-C(=O)CH₃, alkoxy having from 1 to about 10 carbons, or -O-Si(R₄)₃;
R⁶ is H, alkyl or aralkyl having up to 15 carbon atoms;
or R⁵ and R⁶, together with the atoms to which they are attached, form a 5 or 6 membered ring;
R⁷ is alkyl or aralkyl having up to 15 carbon atoms;
or R⁶ and R⁷, together, form one of the structures 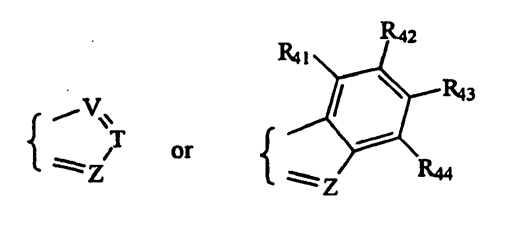
wherein V, T, and Z are independently CH or N;
R₄₁, R₄₂, R₄₃ and R₄₄ are each independently H or an electronegative group;
R⁸ is H or methyl;
R¹⁶ is H, alkyl or aralkyl having up to 15 carbon atoms;
B is a nucleobase; and
n is an integer from 0 to 50; with a second synthon of Formula II: 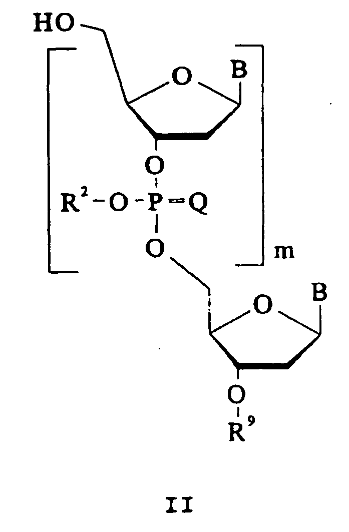 wherein:
R⁹ is a hydroxyl protecting group or a linker connected to a solid support; and
m is an integer from 0 to 50; for a time and under reaction conditions effective to form a third synthon of Formula III: 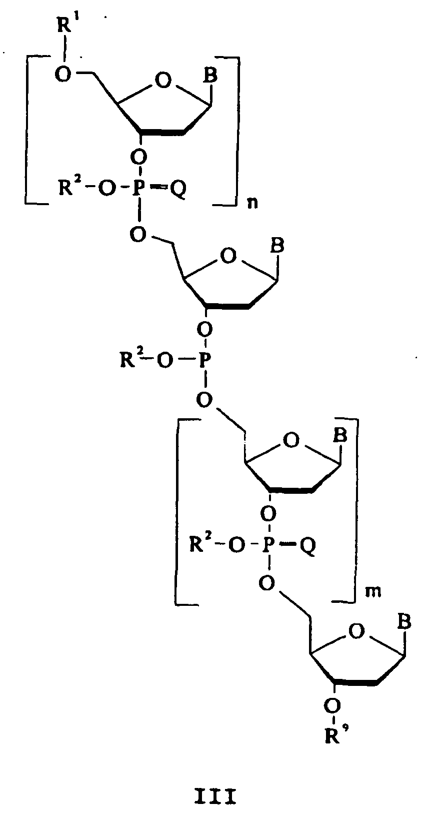
and
- contacting said third synthon with a sulfurizing agent to form an oligomer of Formula IV: 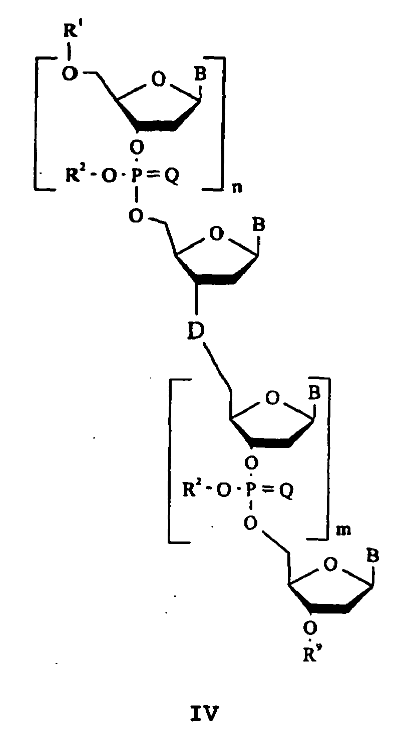
wherein D is said phosphorothioate linkage having the formula:

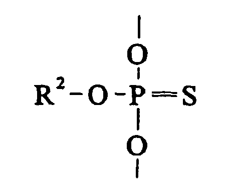

In accordance with the invention, first synthons are cyclic phosphoramidites having the general Formula Vic: 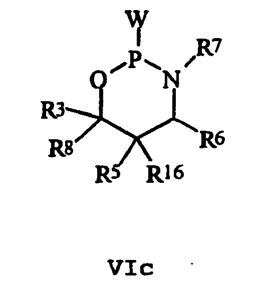 in which W, R³, R⁵-R⁸ and R¹⁶ are as defined above.

The reaction of first and second synthons is conducted in the presence of a catalyst. The structures of the first synthon and the catalyst are chosen such that the opening of the cyclic O-P-N phosphoramidite (azaphospholane) ring proceeds by the stereoselective breaking of the intracyclic P-N bond of the azaphospholane, to yield a third synthon, which is diastereomerically enriched at phosphorus. Accordingly, in preferred embodiments of the methods of the invention, first synthons are diastereomerically enriched, and more preferably in a single stereochemical form, substantially free of other stereochemical forms. It is also advantageous for the first synthon and the catalyst to bear substituent groups which are of relatively large size (i.e., bulky groups) to aid in the proper orientation of reactants to achieve the desired stereoselectivity. As used herein, the term stereoselective has its normal meaning as a process in which one stereoisomer is produced or destroyed more rapidly than another, resuting in a predominance of the favored stereoisomer.

In preferred embodiments catalysts have one of the Formulas VIII or IX: 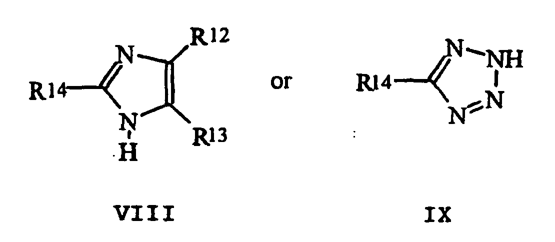 wherein R¹²-R¹⁴ are as defined above.

It has been found in accordance with the present invention that imidazole catalysts having electron-withdrawing substituents, in addition to substituents of relatively large size, are especially advantageous in production of stereochemically enriched products. While not wishing to be bound by a particular theory, it is believed that the catalyst first protonates the azaphospholane nitrogen, creating a good leaving group, which is displaced by the catalyst or its conjugate base. The imidazole or tetrazole attached to the phosphorus is then displaced either by the 3'-hydroxyl of the nucleosidic species, leading to a phosphite triester of high stereochemical purity, or by the catalyst, leading to epimerization.

It has been found in accordance with the present invention that catalysts which have appreciable acidity (i.e., which have pKa values of about 2 to 4) and which are relatively large can overcome the tendency toward epimerization at phosphorus, and result in stereoselective addition of the free 5'-hydroxyl of the nucleosidic species to be added. Thus, preferred substituents for groups R¹³, R¹⁴ and R¹⁵ are those which are electron withdrawing, (and which therefore increase acidity), and of a size sufficient to maintain stereoselectivity. It will be recognized, however, that it is not necessary that all three groups R₆, R₇ and R₈ be of great bulk, so long as the overall size of the catalyst is sufficient to afford the desired stereoselectivity. Thus preferred R¹² and R¹³ groups are independently hydrogen, halogen, cyano, nitro, alkyl having from one to 10 carbons, substituted alkyl having from one to 10 carbons, an ester group, or R¹² and R¹³, together with the carbon atoms to which they are attached, form a substituted or unsubstituted phenyl ring where said substituents are electron withdrawing. Preferred R¹⁴ groups include hydrogen, halogen, cyano, nitro, thio, alkyl having from one to 10 carbons, substituted alkyl having from one to 10 carbons, norbornyl, substituted norbornyl, aryl, substituted aryl wherein said substituents are electron withdrawing, or has the formula: 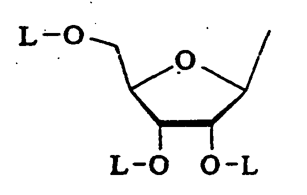 wherein L is protecting group. In prefered embodiments of the invention the catalyst is 2,4,5-tribromoimidazole, dibromocyanoimidazole, or dicyanobromoimidazole. In particularly preferred embodiments the catalyst is 4,5-dicyano-2-bromoimidazole. DE 2317453 discloses substituted imidazoles, including 2-bromo-4,5-dicyanoimidazole.

It has been found in accordance with the present invetion that the dicyanoimidazole, bromoimidazole, and tribromoimidazole catalysts described in accordance with the present invention are useful as substitutes for tetrazole catalysts in standard solid phase oligonucleotide synthetic regimes. Such synthetic procedures are well known in the art, and are extensively described in the literature. *See* for example, Caruthers U.S. Patents Nos. 4,415,732; 4,458,066; 4,500,707; 4,668,777; 4,973,679; and 5,132,418; and Koster U.S. Patents Nos. 4,725,677 and Re. 34,069, and *oligonucleotides and Analogues, A Practical Approach,* Eckstein, F., IRL Press, New York (1991). The use of the catalysts of the invention in these synthetic methologies provides significant advantages over tetrazole catalysts, including, for example, significantly lower cost.

In other preferred embodiments R¹⁴ has one of the formulas: 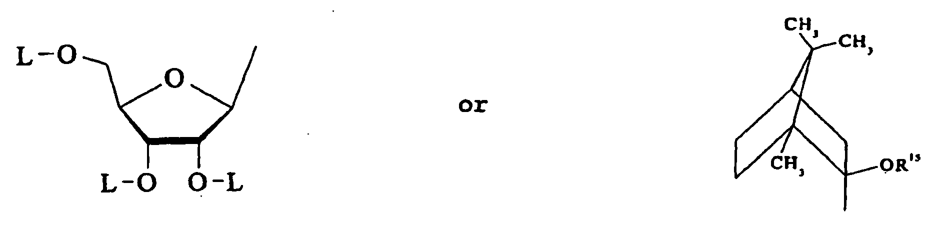 wherein R¹⁵ is H, methyl, trimethylsilyl or acetyl.

In some preferred embodiments R⁶ and R⁷, together with the atoms to which they are attached, form an heterocyclic (i.e., imidazole, triazole or tetrazole) ring, which performs the function of the catalyst. Preferred first synthons which incorporate the catalyst therein have the general Formula Xa or XIIIa: 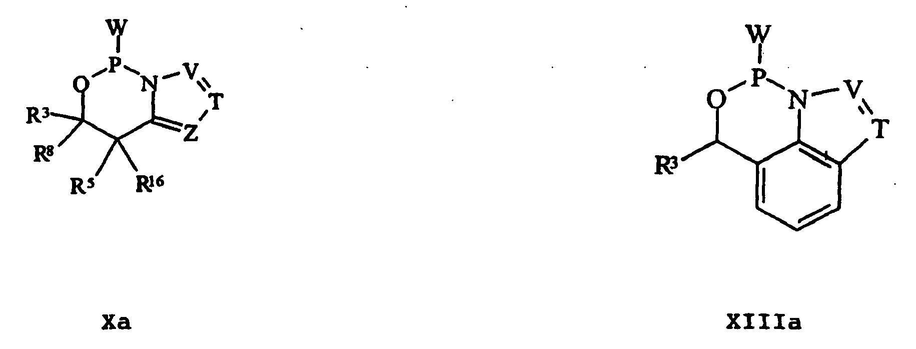 wherein V, T and Z are each independently N or CH. In especially preferred embodiments the first synthons incorporate imidazole rings, and have the Formula Xb or Xc:

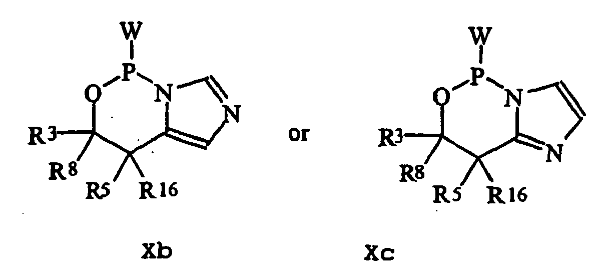

In further preferred embodiments of the invention the imidazole portions of the first synthons are further substituted, for example, by having a phenyl ring fused thereto, which preferably bears one or more electronegative groups. Thus in another preferred embodiment first synthons have the Formula XIa:

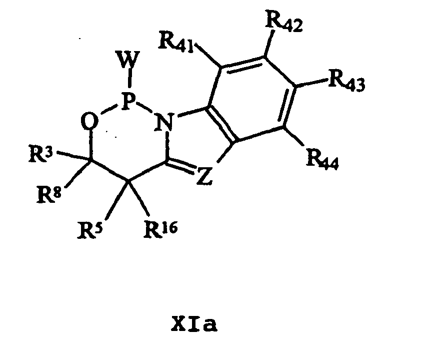

In further preferred embodiments, first synthons incorporate other relatively large substituent groups which facilitate the stereoselective opening of the azaphospholane ring. In particularly preferred embodiments first synthons have the Formula XIIa, and particularly Formula XVIIa or XVIIIa: 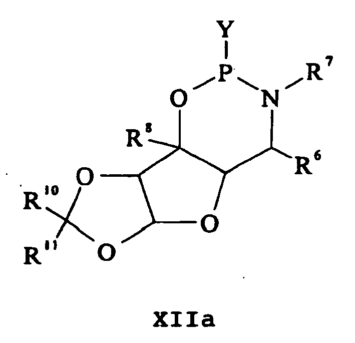 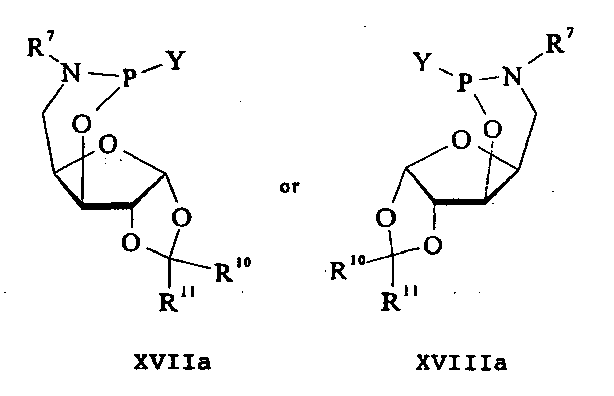 in which R¹⁰ and R¹¹ are as defined above.

In especially preferred embodiments, first synthons have the Formula XVb or XVIb:

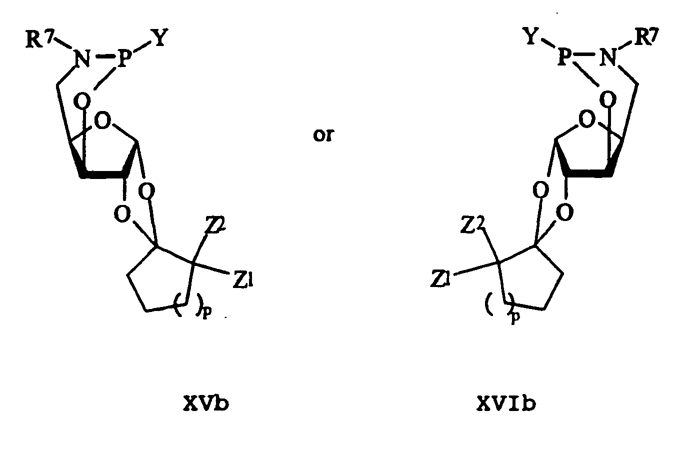

In some preferred embodiments, the first synthon is obtained by reaction of a compound of Formula V: 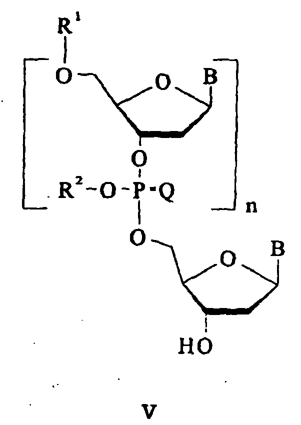 with an azaphospholane of Formula VIa: 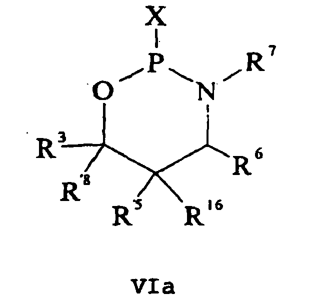 wherein R³-R⁸ are as defined above; and X is halogen, preferably chlorine, dialkylamino, imidazole or substituted phenoxy wherein said substituents are electron withdrawing, and preferably are halogen or nitro.

In more preferred embodiments the first synthon is obtained by reaction of a compound of Formula VII: 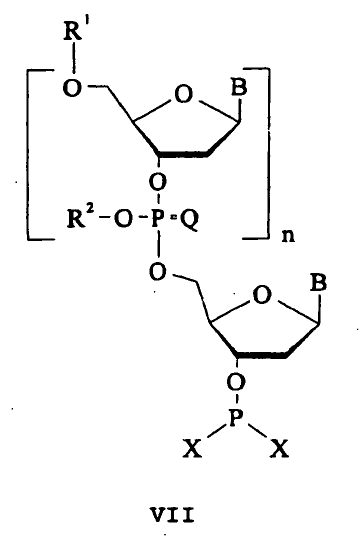 and a γ-amino alcohol of formula HO-C(R⁸)R³-C(R¹⁶)^{R}-CHR⁶-NHR⁷; wherein X and R¹-R¹⁶ are as defined above.

R₂ is a chiral auxiliary, which has the formula -C(R⁸)R³-C(R¹⁶)R⁵-CHR⁶-NHR⁷, and which is formed as a consequence of the opening of the cyclic phosphite ring.
The chiral auxiliary functions as a protecting group for the phosphorus linkage during the course of the synthesis of oligomeric phosphorothioates. Accordingly, chiral auxiliaries are allowed to remain on the growing chain, and are removed at the end of the iterative synthetic regime. Removal of chiral auxiliaries can be conveniently accomplished in a single treatment after the completion of the iterative synthesis by treatment with either acidic reagents or by base catalyzed β-elimination. Suitable acidic reagents include α-halo organic acids such as, for example, 70% trifluoroacetic acid. Suitable reagents for removing chiral auxiliaries by β-elimination include ammonia and fluoride ion. Removal of chiral auxiliaries via β-elimination should be particularly advantageous where first synthons have the Formula XXa or Formula XIa, especially where at least one of R₄₁, R₄₂, R₄₃, and R₄₄ is an electronegative group, or where R³ is -CH₂R₄, R⁴ is independently -N(R₇₀)-C(=O)-R₇₁, -S-C(=O)-R₇₀, or -O-C(=O)-ON(R₇₀)(R₇₁).

After reacting first and second synthons to form a third synthon, the third synthon is sulfurized to form a phosphorothioate linkage having the formula:

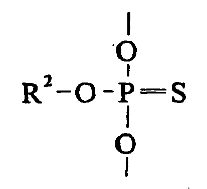

Sulfurization may be accomplished by any of the several sulfurizing agents known in the art to be suitable for conversion of phosphites into phosphorothioates. Useful sulfurizing agents include Beaucage reagent described in e.g., Iyer, R.P.; Egan, W.; Regan, J.B.; Beaucage, S.L., 3H-1,2-Benzodithiole-3-one 1,1-Dioxide as an Improved Sulfurizing Reagent in the Solid-Phase Synthesis of Oligodeoxyribonucleoside Phosphorothioates, *Journal of American Chemical Society,* **1990,** 112, 1253-1254 and Iyer, R.P.; Phillips, L.R.; Egan, W.; Regan J.B.; Beaucage, S.L., The Automated Synthesis of Sulfur-Containing Oligodeoxyribonucleotides Using 3H-,2-Benzodithiol-3-one 1,1-Dioxide as a Sulfur-Transfer Reagent, *Journal of Organic Chemistry,* **1990,** 55, 4693-4699. Tetraethyl-thiuram disulfide can also be used as described in Vu, H.; Hirschbein, B.L., Internucleotide Phosphite Sulfurization With Tetraethylthiuram Disulfide, Phosphorothioate Oligonucleotide Synthesis Via Phosphoramidite Chemistry, *Tetrahedron* Letters, **1991,** 32, 3005-3007. Further useful reagents for this step are dibenzoyl Tetrasulfide, Rao, M.V.; Reese, C.B.; Zhengyun, Z., Dibenzoyl Tetrasulphide - A Rapid Sulphur Transfer Agent in the Synthesis of Phosphorthioate Analogues of Oligonucleotides, *Tetrahedron Letters,* **1992,** 33, 4839-4842; di(phenylacetyl)disulfide, Kamer, R.C.R.; Roelen, H.C.P.F.; van den Eist, H.; van der Marel, G.A.; van Boom, J.H., An Efficient Approach Toward the Synthesis of Phosphorothioate Diesters Va the Schonberg Reaction, *Tetrahedron Letters,* **1989,** 30, 6757-6760; sulfur; and sulfur in combination with ligands like triaryl, trialkyl or triaralkyl or trialkaryl phosphines.

The methods of the present invention can also be used to produce analogs of phosphorothioates, including phosphoroselenoates and phosphoroboronates. For example, phosphoroselenoates can be prepared by the methods of the invention by utilizing potassium selenocyanate in place of the sulfurizing agents described above. Phosphoroboronates can be prepared by similar adaptation of oxidizing agents known known in the art. *See,* for example, *Antisense Research and Applications,* Crooke, S.T., and Lebleu, B., Eds. CRC Press, Boca Raton, Florida (1993).

R₉ and R₁ can each be a hydroxyl protecting group.Protecting groups are known *per se* as chemical functional groups that can be selectively appended to and removed from functionalities, such as hydroxyl groups and carboxyl groups. These groups are present in a chemical compound to render such functionality inert to chemical reaction conditions to which the compound is exposed. The tert-butyldimethylsilyl (TBDMS) group is representative of protecting groups useful for protecting the hydroxyl functionality. A preferred protecting group for R¹ is the dimethoxytrityl group. Other representative groups may be found in Greene, T.W. and Wuts, P.G.M., *"Protective Groups in Organic Synthesis"* 2d. Ed., Wiley & Sons, 1991. Typically, protecting groups are removed at the end of the iterative synthesis.

R₉ may alternatively be a linker connected to a solid support. Solid supports are substrates which are capable of serving as the solid phase in solid phase synthetic methodologies, such as those described in Caruthers U.S. Patents Nos. 4,415,732; 4,458,066; 4,500,707; 4,668,777; 4,973,679; and 5,132,418; and Koster U.S. Patents Nos. 4,725,677 and Re. 34,069. Linkers are known in the art as short molecules which serve to connect a solid support to functional groups (e.g., hydroxyl groups) of initial synthon molecules in solid phase synthetic techniques. Suitable linkers are disclosed in *Oligonucleotides And Analogues A Pratical Approach,* Ekstein, F. Ed., IRL Press, N.Y, 1991.

Alkyl groups according to the invention include straight chain, branched, and cyclic carbon and hydrogen containing groups such as methyl, isopropyl, and cyclohexyl groups. Preferred alkyl groups have 1 to about 6 carbon atoms.

Aralkyl groups according to the invention include both alkyl and aryl portions, although the point of attachment of such groups is through an alkyl portion thereof. Benzyl groups provide one example of an aralkyl group. Alkaryl groups include both alkyl and aryl portions, and are attached through their aryl portions. The term aryl is intended to denote monocyclic and polycyclic aromatic groups including, for example, phenyl, naphthyl, xylyl, pyrrole, and furyl groups. Although aryl groups (*e.g.*, imidazo groups) can include as few as 3 carbon atoms, preferred aryl groups have 6 to about 14 carbon atoms, more preferably 6 to about 10 carbon atoms. The alkyl, alkaryl, and aryl groups may be substituted (*e.g*., i.e, bear halogens and hydroxy groups) or unsubstituted moieties. In some prefered embodiments the alkyl or aralkyl groups bear electron withdrawing substituents, preferably halogen atoms, in one their α-carbons. Examples of α-halo substituted alkyl groups include mono-, di-, and trihalo methyl groups. Examples of α-halo substituted aralkyl groups include α-halo benzyl groups.

Certain substituent groups of compounds of the invention bear electron withdrawing groups. As used herein, the term "electron wihdrawing" has its normal meaning as a chemical functionality which electronically or inductively causes the withdrawal of electron density form the moiety to which the electron withdrawing groups is attached. Representative electron withdrawing groups include nitro groups, halogens, cyano, carboxyl groups and substituted carboxy groups such as ester groups and amido groups. Other electron withdrawing groups will be apparent to those of skill in the art, once armed with the present disclosure.

Substituent B is a nucleobase. The term nucleobase as used herein is intended to include naturally occurring nucleobases (i.e., heterocyclic bases found in naturally occurring nucleic acids) and their non-naturally occurring analogs. Thus, nucleobases according to the invention include naturally occurring bases adenine (A), guanine (G), thymine (T), cytosine (C) and uracil (U), both in their unprotected state and bearing protecting or masking groups. Examples of nucleobase analogs include N⁴,N⁴⁻ethanocytosine, 7-deazaxanthosine, 7-deazaguanosine, 8-oxoN⁶-methyladenine, 4-acetylcytosine, 5-(carboxyhydroxymethyl) uracil, 5-fluorouracil, 5-bromouracil, 5-carboxymethylaminomethyl-2-thiouracil, 5-carboxymethylaminomethyl uracil, inosine, N⁶⁻isopentyladenine, 1-methyladenine, 2-methylguanine, 5-methylcytosine, N⁶-methyladenine, 7-methylguanine, 5-methylaminomethyl uracil, 5-methoxyaminomethyl-2-thiouracil, 5-methoxyuracil, pseudouracil, 5-methyl-2-thiouracil, 2-thiouracil, 4-thiouracil, 5-(1-propynyl)-4-thiouracil, 5-(1-propynyl)-2-thiouracil, 5-(1-propynyl)-2-thiocytosine, 2-thiocytosine, and 2,6-diaminopurine. Other suitable base analogs, for example the pyrimidine analogs 6-azacytosine, 6-azathymidine and 5-trifluoromethyluracil, may be found in Cook, D.P., et al., International Publication No. 92/02258, which is herein incorporated by reference.

The compounds of the invention are preferably up to 50 nucleobases in length, with 10 to 30 nucleobases being more preferred, and 15 to 25 nucleobases being especially preferred.

In preferred embodiments the phosphorothioate linkage produced by the method of the invention is diastereomerically enriched. The term "diastereomerically enriched" denotes the predominance of one stereochemical form over the other. In preferred embodiments the phosphorothioate linkage is 75% in a single stereochemical form. In further preferred embodiments the phosphorothioate linkage is 85% in a single stereochemical form, with 90% being further preferred and 95% being especially preferred. In further preferred embodiments the phosphorothioate linkage is in a single stereochemical form, substantially free of other stereochemical forms.

Preferably, following sulfurization, the phosphorothioate is next converted to a new first synthon. This is first accomplished by the removal of the 5'-hydroxyl protecting group R₁, under conditions which will necessarily depend upon the chemical identity of the specific R₁ group. After removal of the protecting group, the unprotected 5'-alcohol may be employed as a new second synthon in the iterative method. Libraries of dimeric and higher synthons may be prepared and stored to facilitate the iterative synthesis of desired nucleobase sequences.

Also provided according to the invention are azaphospholanes of Formula VIb: 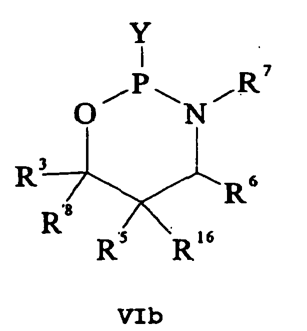 wherein Y is X or W, wherein X is halogen, dialkylamino, imidazole, or substituted phenoxy wherein said substituents are electron withdrawing, and W has the formula: 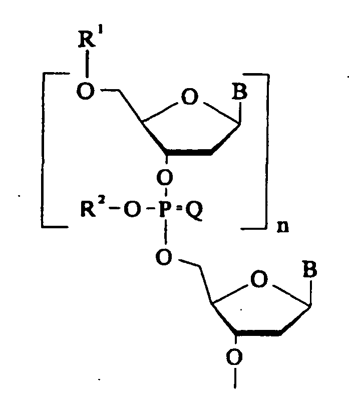 wherein constituent members are as defined above. Preferably, the azaphospholanes of Formula VIb are diastereomerically enriched. In particular, it is advantageous to have defined stereochemistry around phosphorus atom, to afford diastereomerically enriched products upon stereoselective opening of the azaphospholane ring.

In preferred embodiments, compounds of the invention have one of the Formulas Xb, XIb, XIIb, XIIIb or XXb: 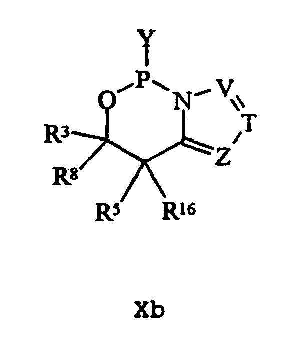 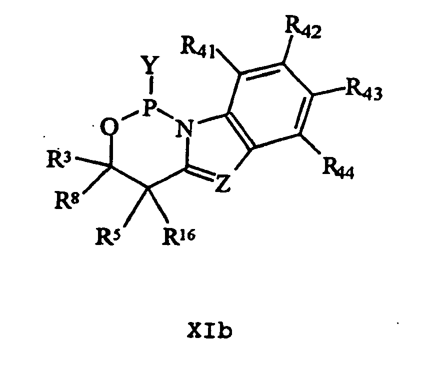 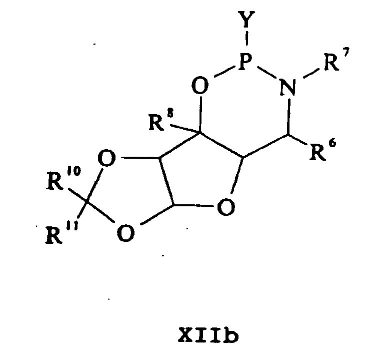 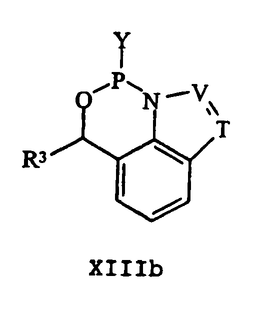 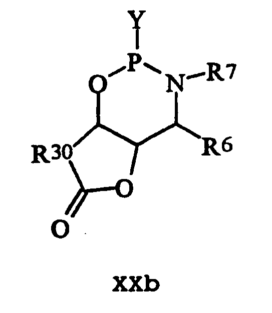 wherein R³-R¹⁶, Y, V, T, Z, Z₁, Z₂ and p are as defined above.

Paticularly preferred embodiments of the compounds of the invention have the Formula XIVb, Xd, Xe, XVIIb, XVIIIb, XVb or XVIb:

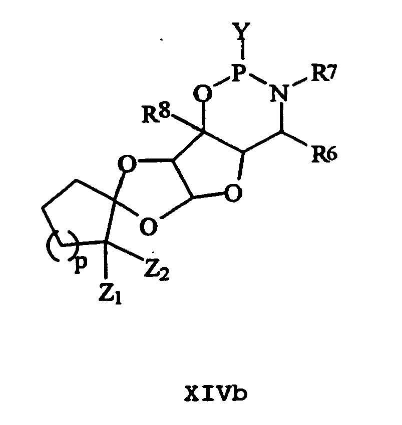

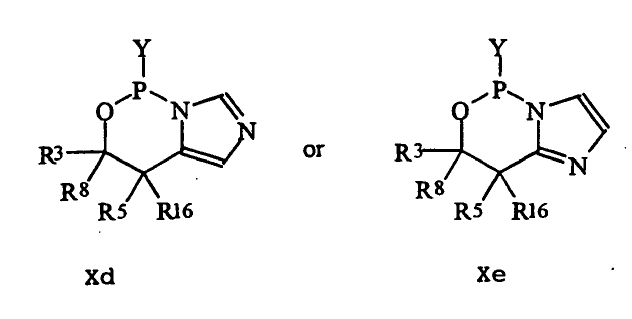

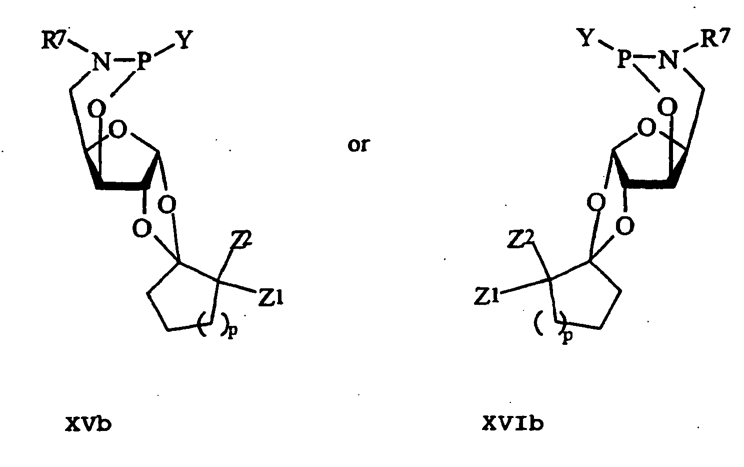

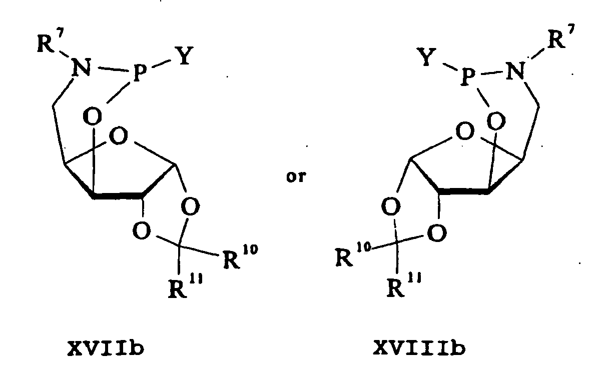

As used herein, the term "contacting" means directly or indirectly causing placement together of moieties to be contacted, such that the moieties come into physical contact with each other. Contacting thus includes physical acts such as placing the moieties together in a container. The term "reacting" as used herein means directly or indirectly causing placement together of moieties to be reacted, such that the moieties chemically combine or transform.

The method of the invention is performed in the presence of a solvent, for example chloroform or acetonitrile. Other solvents suitable for use in the present method will be readily apparent to those skilled in the art, once having been made aware of the present disclosure.

In general, it is preferred that the molar ratio of the catalyst to the first synthon starting material be from about 1 to about 50; preferably from about 2.5 to about 10.

The method of the present invention can be carried out in any suitable vessel which provides efficient contacting between the first and second synthons, and the catalyst. The reaction vessel used should be resistant to the components of the reaction mixture. Glass-lined vessels would be suitable for this purpose. Additional vessel materials will be apparent to those skilled in the art.

The reagents of the present method may be added in any order. The method is preferably carried out under an inert atmosphere, any should be carried out in a dry atmosphere. Any suitable inert gas may be employed, such as nitrogen, helium and argon.

Preferably, the method is carried out at temperatures ranging between about -20°C and about 40°C, with temperatures ranging from about -15°C to about 0°C being more preferred.

Reaction time is generally from about one minute to about two hours, with reaction times of from about one minute to about 10 minutes being preferred.

Product can be recovered by any of several methods known to those of skill in the art. Preferably, products are recovered by chromatography. Additional separation of isomers can be accomplished by techniques known in the art including high performance liquid chromatography.

When R⁹ is a solid support, purification is carried out after removal of the oligonucleotide from the solid support using methods known in the art.

The invention is further illustrated by way of the following examples. These examples are illustrative only and are not intended to limit the scope of the appended claims.

### Examples:

### General methods.

Melting points (m.p.) were determined using an Electrothermal MP apparatus and are uncorrected. Optical rotation measurements were carried out in the indicated solvents employing a Jasco® DIP-140 digital polarimeter. Mass spectra (CI or EI) were obtained on an HP 5980A quadrupole mass spectrometer in the direct-inlet mode.

NMR spectra were recorded on JEOL 270, Varian XL200, XL300, or Unity 500 spectrometers. Chemical shifts are given in the δ scale in parts per million. The assignments of proton spectra are based on COSY experiments. The residual proton signals of deuteriochloroform (δ 7.24 ppm), methanol (δ 3.30ppm) and acetonitrile (δ 1.93ppm) were used as reference in these solvents. The multiplicities are recorded using the following abbreviations: s, singlet; d, doublet; t, triplet; q, quartet; m, multiplet. ³¹P NMR spectra were obtained on either a Varian XL200, XL300 or Unity 500 instrument, and chemical shifts are given with respect of aqueous phosphoric acid. Peak assignments of ¹³C-NMR spectra were, in some cases, made with the aid of APT, HMQC or HETCOR® experiments. ³¹P-NMR spectra were recorded on Jeol CFP 270 and Varian UNITY® 500 at 68.7 MHz, 125.7 MHz using a 85% H₃PO₄ as external standard.

Fast atom bombardment (FAB-MS) were obtained on an KRATOS MS 25RFA spectrometer in the direct-inlet mode. High resolution FAB mass spectra of key compounds were obtained on a ZAB 2F HS spectrometer in the direct inlet mode (Biomedical Spectrometry Unit).

Tetrahydrofuran (THF) was distilled from sodium benzophenone ketyl. Dichloromethane was distilled from P₂O₅. Triethylamine and acetonitrile were distilled from CaH₂. N,N-Dimethyl formamide was dried by shaking with KOH, followed by distillation. Thin-layer chromatography (TLC) was performed using Kieselgel 60 F₂₅₄ aluminium-backed plates (0.2 mm thickness) and visualized by UV and./ or dipping in a solution of ammonium molybdate (2.5 g) and ceric sulfate (1g) in 10% v/v aqueous sulphuric acid (100 ml), followed by heating. Kieselgel 60 (Merck 230-400 mesh) silica gel was employed for column chromatography.

### Preparatory Example 1

### 3R-hydroxy-N-iso-propylbutanoamide (10)

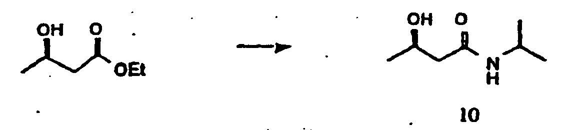

A 2 M solution of trimethylaluminium in hexane (50 ml, 100 mmol) was slowly added to a solution of 8.5 ml (100 mmol) isopropylamine in 100 ml dichloromethane under nitrogen at room temperature. The mixture was stirred for 30 min and then cooled to 0°C before 6.6 g (50 mmol) of ethyl-3R-hydroxybutanoate was added. The reaction mixture was stirred at room temperature for 2 hours for completion, carefully quenched with dilute HCl and extracted with chloroform. The organic extract was dried over MgSO₄ and concentrated to affored 7 g N-isopropyl 3R-hydroxy butanoamide. After recrystallization, 5.5 g pure amide 10 was obtained (yield 76%) .: m.p. 62°C; ¹H NMR (200 MHz, CDCl₃) δ 6.41 (m, 1H, NH), 4.35 (b, 1H, OH), 3.78-4.15 (m, 2H, MeCH, Me₂CH), 2.08-2.32 (m, 2H, CH₂), 1.12 (d, J=6.3 Hz, 3H, Me), 1.07 (d, J=6.6 Hz, 6H, NCHMe₂) ; ¹³C NMR (50 MHz, CDCl₃) δ 171.5 (C=O), 64.7 (CHOH), 43.8 (NHCH), 41.0 (CH₂), 22.7 (Me), 22.4 (NCHMe₂); MS(EI) m/e 145 ([M⁺], 25%), 130 (27), 112 (4), 101 (6), 86 (34), 69 (8), 58 (22), 44 (100); HRMS(EI) m/e calc'd for C₇H₁₅O₂N [M⁺]: 145.1103, found 145.1109.

### Preparatory Example 2

### 2R-Hydroxy-4-(N-isopropyl)aminobutane (11)

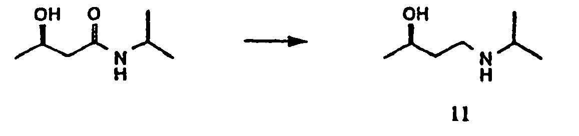

To a solution of 32 ml 1 M borane (32 mmol) in THF was added 2.32 g (16 mmol) 3R-hydroxy-N-*iso*-propyl-butyl butanoamide 10 in 20 ml THF at 0°C under nitrogen. The solution was then brought to reflux and maintaned there for one hour. The reaction mixture was cooled to room temperature and 1 N HCl was added slowly to quench the reaction. THF was removed *in vacuo,* and the aqueous solution was saturated with solid NaOH and then was extracted three times with a total 300 ml chloroform. The combined chloroform phase was dried, filtered and distilled to afford 1.4 g 3-(N-isopropylamino) butan-2-ol 11 as a clear, colorless liquid (yield 67%).: ¹H NMR (200 MHz, CDCl₃) δ 3.80-3.96 (m, 1H, CHOH), 2.8.6-2.98 (m, 1H, MeCH), 2.56-2.75 (m, 2H, NCH₂), 1.24-1.60 (m, 2H; CH₂), 1.08 (d, J=6.1 Hz, 3H, Me), 0.98 (d, J=6.2 Hz, 6H, Me₂); ¹³C NMR (200 MHz, CDCl₃) δ 69.5 (OCH), 48.6 (NHCH₂), 46.0 (NHCH), 37.2 (CH₂), 23.5, 22.9, 22.5; MS(EI) m/e 131 ([M⁺], 10%), 116 (81), 98 (35), 72 (100), 58 (30), 56 (45), 44 (37); HRMS(EI) m/e calc'd for C₇H₁₇ON [M⁺]: 131.1310, found 131.1311; [a]_{D}²⁰=32.5° (c=0.21, chloroform).

### Preparatory Example 3

### 2R-Hydroxy-4-(N-tert-butyl)aminobutane (31)

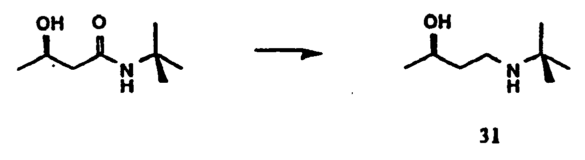

To a solution of 26.4 ml 1 M (26.4 mmol) borane in THF was added 2.1 g (13.2 mmol) 3R-hydroxy-N-*tert-*butylbutanoamide in 20 ml THF at 0°C under nitrogen. The solution was then brought to reflux and maintained there for one hour. The reaction mixture was cooled down to room temperature and 1 N HCl was added slowly to quench the reaction. THF was removed *in vacuo,* and the aqueous solution was saturated with solid NaOH and then was extracted three times with a total 250 ml diethyl ether. The combined organic phase was dried, filtered and distilled to afford 398 mg 2R-hydroxy-4-(N-*tert*-butyl)aminobutane 31 as a clear, colorless liquid (yield 20.1%).: ¹H NMR (200 MHz, CDCl₃) δ 3.80-3.92 (m, 1H, CHOH), 3.40-3.80 (b, 2H, OH, NH), 2.50-2.90 (m, 2H, NCH₂), 1.28-1.61 (m, 2H, CH₂), 1.07 (d, 3H, Me), 1.03 (s, 9H, Me₃)

### Preparatory Example 4

### 5'-O-(tert-butyldimethylsilyl) thymidine (1)

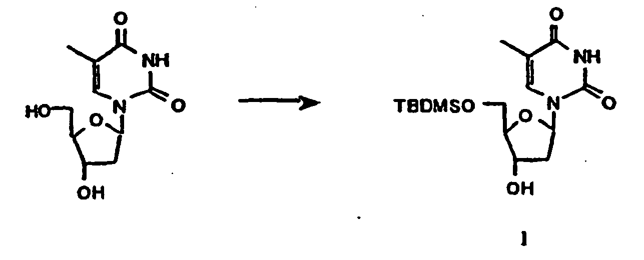

To a solution of 2.42 g (10 mmol) thymidine in 15 ml DMF was added 1.7 g (25 mmol) imidazole and 1.6 g (10.6 mmol) *tert*-butyldimethyl silyl chloride. The solution was stirred at room temperature for 3 hours. DMF was then removed in *vacuo* and the residue was dissolved in 150 ml of ethyl acetate. The solution was washed with water and the organic layer was dried over MgSO₄. After removing the solvent, the solid was recrystallized with ethyl acetate/pentane to obtain 2.5 g pure 5'-O-(tert-butyldimethylsilyl) thymidine 1 (70% yield).: m.p. 193-194°C; ¹H NMR (500 MHz, CDCl₃) δ 9.0 (s, 1H, NH), 7.50 (s, 1H, H-6), 6.36 (dd, J=5.8, 8.1 Hz, 1H, H-1'), 4.44 (m, 1H, H-3'), 4.03 (m, 1H, H-4'), 3.85 (m, 2H, H-5'), 2.66 (d, J=3.8 Hz, 1H, OH), 2.35 (m, 1H, H=2'), 2.07 (m, 1H, H-2'), 1.89 (s, 3H, C=CMe), 0.89 (s, 9H, CMe₃), 0.09 (s, 6H, SiMe₂); ¹³C NMR (125 MHz, CDCl₃) δ 163.8 (C-4), 150.4 (C-2), 135.4 (C-6), 110.9 (C-5), 87.2 (C-4'), 85.0 (C-1'), 72.6 (C-3'), 63.6 (C-5'), 41.1 (C-2'), 25.9 (SiCMe₃), 18. 3 (SiCMe₃), 12.5. (C=CMe), -5.4 (SiMe₂), -5.5 (SiMe₂).

### Preparatory Example 5

### 5'-O-(4,4'-dimethoxytrityl) thymidine (16)

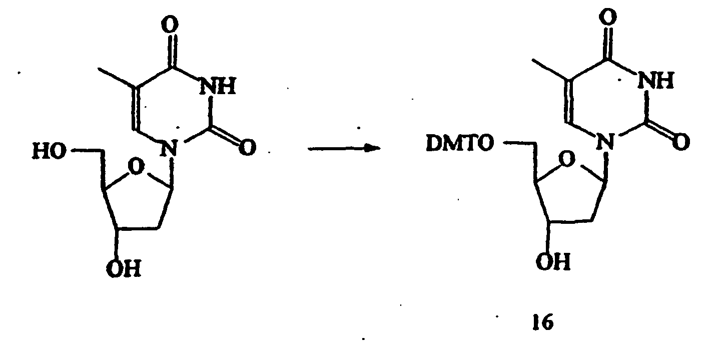

Triethylamine (10 ml) in 200 ml THF was injected into a solid mixture of 6.8 g (28.0 mmol) thymidine and 10.2 g (28.6 mmol) 4,4'-dimethoxytrityl chloride under nitrogen with stirring. The solution was stirred at room temperature for 2 hours. After completion of the reaction, 10 ml methanol was added to consume the excess DMTrCl. The mixture was stirred for 5 minutes and the solvent removed by rotary evaporation. The residue was dissolved in 250 ml of ethyl acetate and the solution was washed with saturated NaHCO₃ and dried over MgSO₄. The solid was recrystallized from ethyl acetate/hexane to obtain 13.0 g 5'-protected thymidine 16 (85.6%).: m.p. 124-126°C; ¹H NMR (200 MHz, CDCl₃) δ 8.97 (s, 1H, NH), 7.60 (m, 1H, H-6), 6.72-7.42 (m, 13H, Ph), 6.42 (m, 1H, H-1'), 4.56 (m, 1H, H-3'), 4.05 (m, 1H, H-4'), 3.78 (s, 6H, OMe₂), 3.41 (m, 2H, H-5'), 2.62 (m, 1H, OH), 2.46 (m, 2H, H-2'), 1.46 (s, 3H, Me); ¹³C NMR (50 MHz, CDCl₃) δ 163.0 (C-4), 157.6, 149.8 (C-2), 143.5, 135.0 (C-6), 134.7, 134.6, 129.4, 127.4, 127.3, 126.5, 112.8, 110.9 (C-5), 86.7 (C-4'), 86.2, 84.7 (C-1'), 72.5 (C-3'), 63.8 (C-5'), 55.5 (OCH₃), 41.3 (C-2'), 12.5 (CH₃).

### Preparatory Example 6

### 5'-O-(4,4'-dimethoxytrityl)-3'-O-(tert-butyldimethlsilyl) thymidine (17)

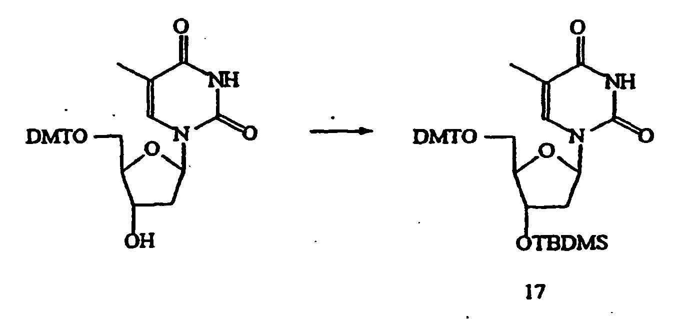

To a solution of 13.0 g (23.9 mmol) 5'-O-(4,4'-dimethoxytrityl)thymidine 16 in 50 ml DMF was added 3.0 g (44 mmol) imidazole and 3.6 g (23.9 mmol) *tert*-butyl-dimethylsilyl chloride. The solution was stirred at room temperature for 3 hours. DMF was then removed *in vacuo* and the residue was dissolved in 300 ml of ethyl acetate. The solution was washed with water and the organic layer was dried over MgSO₄. After concentration of the solution and recrystallization from ethyl acetate/hexane, the solid product 5'-O-(4,4'-dimethoxy)-3'-(tert-butyldimethylsilyl)thymidine 17 was used directely for the next reaction.

### Preparatory Example 7

### 3'-O-(tert-butyldimethylsilyl)thymidine (18)

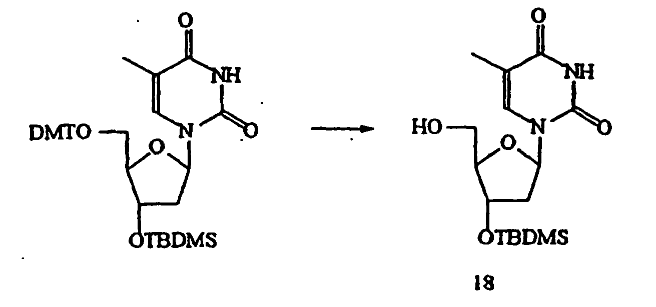

A solution of 5 g (7.6 mmol) 5'-O-(4,4'-dimethoxytrityl)-3'-O-(tert-butyldimethylsilyl) thymidine 17 in 100 ml 80% aq. acetic acid was stirred until the removal of dimethoxytrityl group was completed. Saturated Na₂CO₃ was then added to adjust the pH of the solution to 6-7. The solution was then extracted with ethyl acetate. The extract was dried and the mixture was chromatographed on a silica gel column (CH₂Cl₂: MeOH=20:1) to give 2.5 g of 3'-O-(*tert-*butyldimethylsilyl) thymidine 18 (92.6%).: m.p.93-95°C (lit. 83-84°C); H NMR (200 MHz, CDCl₃) δ 9.18 (s, 1H, NH), 7.36 (b, 1H, H-6), 6.12 (t, J=6.8 Hz, 1H, H-1'), 4.44-4.48 (m, 1H, H-3'), 3.69-3.91 (m, 3H, H-4', H-5'), 2.87 (m, 1H, OH), 2.15-2.35 (m, 2H, H-2'), 1.87 (s, 3H, C=CMe), 0.86 (s, 9H, CMe₃), 0.05 (s, 6H, SiMe₂); ¹³C NMR (125 MHz, CDCl₃) δ 163.9 (C-4), 150.4 (C-2), 137.1 (C-6), 110.9 (C-5), 87.6 (C-4'), 86.8 (C-1'), 71.5 (C-3'), 61.9 (C-5'), 40.4 (C-2'), 25.7 (SiCMe₃), 17.9 (SiCMe), 12.5 (C=CMe), -4.7 (SiMe₂), -4.9 (SiMe₂).

### Example 8

### 2-Chloro-3-iso-propyl-6R-methyl-1-oxa-3-aza-2-phosphacyclohexane (12)

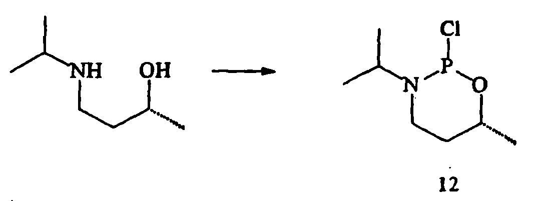

To a solution of 2.2 ml (3.45 g, 25 mmol) phosphorus trichloride in 30 ml dichloromethane was added a solution of 2.89 g (22 mmol) 2R-hydroxy-4-(N-*iso*-propyl)aminobutane 11 and 5.0 g (6.9 ml, 50 mmol) triethylamine in 20 ml dichloromethane with vigorous stirring under nitrogen at 0°C. Stirring was continued at room temperature for 0.5 hour. The solvent was removed by evaporation under reduced pressure and the residue was extracted with diethyl ether (3x50 ml). Distillation gave 2.9 g of product 12 (74.4% yield).: ¹H NMR (200 MHz, CDCl₃) 6 4.48-4.68 (m, 1H, OCH), 3.40-3.61 (m, 1H, NCH), 3.15-3.38 (m, 1H, NCH₂), 2.80-2.96 (m, 1H, NCH₂), 1.71-1.85 (m, 2H, CH₂), 1.25 (d, J=6.4 Hz, 3H, Me), 1.13 (dd, 6H, Me₂); ¹³C NMR (50 MHz, CDCl₃) δ 69.5 (d, J=4.3 Hz, OCMe), 49.6 (d, J=34 Hz, NCMe)₂, 37.4 (d, J=5.5 Hz, NCH₂), 33.4 (CH₂), 22.2 (d, J=4.3 Hz, Me), 21.1 (d, J=13.3 Hz, Me₂), 19.2 (d, J=5.0 Hz, Me₂) ; ³¹P NMR (81 MHz,CDCl₃) δ 160.6.

### Example 9

### Phosphoramidite (13a)

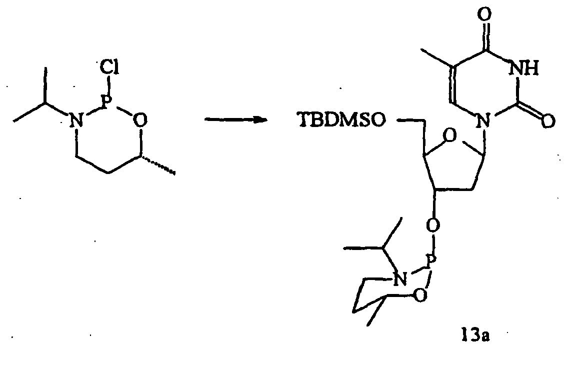

To a solution of 215 mg (1.1 mmol) of 2-chloro-3-isopropyl-6R-methyl-1-oxa-3-aza-2-phosphacyclohexane 12 in 40 ml CH₂Cl₂ was added a solution of 356 mg (1.0 mmol) of 5-O-(*tert*-butyldimethylsilyl)thymidine and 0.154 ml triethylamine (111 mg, 1.1 mmol) in 20 ml CH₂Cl₂ with vigorous stirring under nitrogen at 0°C. The mixture was stirred at room temperature until TLC indicated that the reaction had gone to completion. The reaction mixture was transferred to 150 ml ethyl acetate, which was previously washed with a saturated NaHCO₃ solution. Saturated NaHCO₃ was then added to wash the solution. The separated organic phase was dried over MgSO₄. A white foam (quantitative yield) was formed after the solvent was removed by evaporation under reduced pressure. Two components were found from ³¹P NMR spectra in a ratio of 1:3 (135.0ppm:133.6ppm). The reaction mixture in CDCl₃ was then refluxed for about 4 hours to get a ratio of up to 1:12 (135.Oppm:133.6ppm). After chromatography on a silica gel column (hexane:ethyl acetate:triethylamine=5:3:2), the fast eluting component (133.6ppm) was separated as a pure diastereomer.: ¹H NMR (500 MHz, CDCl₃) δ 7.50 (m, 1H, H-6), 6.36 (dd, J=8.5, 5.8 Hz, 1H, H-1'), 4.56 (m, 1H, H-3'), 4.30 (m, 1H, HCMe), 4.04 (m, 1H, H-4'), 3.75-3.90 (m, 2H, H-5'), 3.39 (hept, J=6.3 Hz, 1H, NCH), 3.24 (m, 1H, NCH₂), 2.72 (m, 1H, NCH₂), 2.35 (m, 1H, H-2'), 2.06 (m, 1H, H-2'), 1.89 (d, J=0.9 Hz, 3H, CH₃), 1.64 (m, 2H, CH₂), 1.16 (d, J=6.4 Hz, 3H, OCHMe), 1.08 (dd, J=2.0 Hz, 6.3Hz, 6H, NCMe₂), 0.89 (s, 9H, SiCMe₃), 0.09 (d, J=1.5 Hz, 6H, SiMe₂); ¹³C NMR (125 MHz, CDCl₃) δ 163.9 (C-4), 150.5 (C-2), 135.3 (C-6), 110.9 (C-5), 86.5 (d, J=2.8 Hz, C-4'), 84.8 (C-1'), 73.3 (d, J=19.2 Hz, C-3'), 66.0 (d, J=2.7 Hz, OCH), 63.2 (C-5'), 49.2 (d, J=34.8 Hz, NCH), 40.4 (d, J=4.6 Hz, C-2'), 36.6 (d, J=5.5 Hz, NCH₂), 34.6 (CH₂), 25.9 (SiCMe₃), 22.9 (d, J=4.6 Hz, OCMe), 21.8 (d, J=10 Hz, NCMe₂), 21.0 (d, J=4.6 Hz, NCMe₂), 18.3 (SiCMe₃), 12.5 (C=CMe), -5.5 (SiMe₂), -5.4 (SiMe₂); ³¹P NMR (81 MHz, CDCl₃) δ 133.6; MS (CI, NH₃)) m/e 516 ([M+H⁺], 37%), 390 (21), 339 (92), 178 (100), 160 (55); HRMS (CI, NH₃) m/e calc'd for C₂₃H₄₃N₃O₆PSi [M+H⁺] : 516.2659, found 516.2664

### Example 10

### Phosphoramidite (13b)

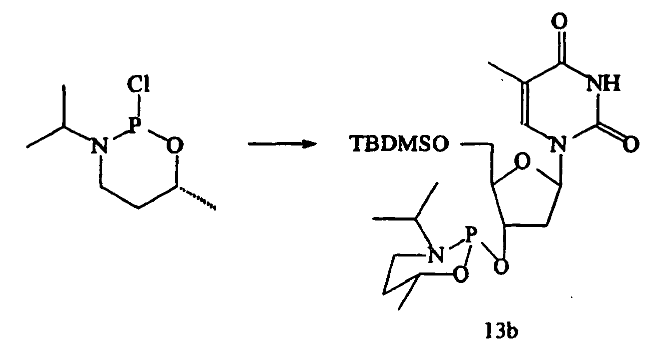

To a solution of 215 mg (1.1 mmol) 2-chloro-3-isopropyl-6R-methyl-1-oxa-3-aza-2-phosph ocyclohexane 12 in 40 ml CH₂Cl₂ was slowly added a solution of 356 mg (1.0 mmol) 5'-O-(tert-butyldimethylsilyl)thymidine 1 and 0.154 ml triethylamine (111 mg, 1.1 mmol) in 20 ml CH₂Cl₂ with vigorous stirring under nitrogen at -78°C. The mixture was stirred at -78°C until TLC indicated that the reaction had gone to completion. The reaction mixture was quickly transferred to 150 ml ethyl acetate, which was previously washed with a cold saturated NaHCO₃ solution. Cold saturated NaHCO₃ was then added to wash the solution. The separated organic phase was dried over MgSO₄. A white foam (quantitative yield) was formed after the solvent was removed by evaporation under reduced pressure at 0°C. Two components were found from ³¹P NMR spectra in a ratio of 5:1 (135.1ppm:133.6ppm). The reaction mixture in CDCl₃ was immediately chromatographed on a silica gel column (hexane:ethylacetate:triethylamine=5:3:2) and the slow eluting component (135.1ppm) was separated as an enriched diastereomer (92% pure).: ¹H NMR (500 MHz, CDCl₃) δ 7.48 (m, 1H, H-6), 6.32-6.35 (dd, J=5.4, 7.8 Hz, 1H, H-1'), 4.55-4.60 (m, 1H, H-3'), 4.02-4.10 (m, 1H, OCH), 4.00 (m, 1H, H-4'), 3.74-3.89 (m, 2H, H-5'), 3.36-3.45 (m, 1H, NCH), 3.09-3.14 (m, 1H, NCH₂), 2.85-2.93 (m, 1H, NCH₂), 2.40-2.44 (m, 1H, H-2'), 2.12-2.21 (m, 1H, CH₂), 2.00-2.06 (m, 1H, H-2'), 1.88 (s, 3H, C=CCH₃), 1.81-1.86 (m, 1H, CH₂), 1.28 (d, J=6.3 Hz, 3H, OCMe), 1.11 (dd,J= 6.3, 11.7 Hz, 6H, NCMe₂), 0.89 (s, 9H, Me₃), 0.08 (d, J=2.0 Hz, 6H, SiMe₂); ¹³C NMR (125 MHz, CDCl₃) δ 163.7 (C-4), 150.2 (C-2), 135.5 (C-6), 110.8 (C-5), 87.0 (d, J=6.4 Hz, C-4'), 84.8 (d, J=5.5 Hz, C-1'), 72.8 (d, J=19.2 Hz, OCHMe), 70.3 (d, 7.3, C-3'), 63.1 (C-5'), 49.5 (d, J=39.4 Hz, NCHMe₂), 40.1 (d, J=1.8 Hz, C-2'), 36.9 (d, J=5.5 Hz, NCH₂), 31.5 (d, J=7.3 Hz, CH₂), 25.9 (SiCMe₃), 23.3 (OCMe), 21.7 (d, J=11.0 Hz, NCHMe₂), 21.1 (d, J=5.5 Hz, NCHMe₂), 18.4 (SiCMe₃), 12.5 (C=CMe), -5.4 (SiMe₂), -5.5 (SiMe₂); ³¹P NMR (81 MHz, CDCl₃) δ 135.1

### Example 11

### Phosphoramidite (14)

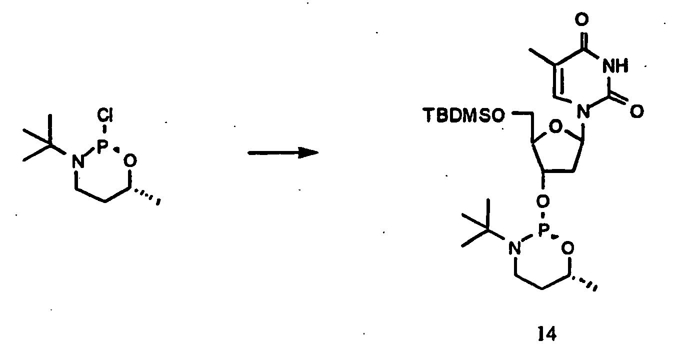

To a solution of 0.2 ml (315 mg, 2.3 mmol) phosphorus trichloride in 10 ml dichloromethane was added a solution of 320 mg (2.2 mmol) butanol 31 and 0.7 ml triethylamine in 5 ml dichloromethane with vigorous stirring under nitrogen at 0°C and then at room temperature for 0.5 hour. The solvent was removed by evaporation under reduced pressure and the residue was extracted with diethyl ether (2x40 ml). After removing the ether, 512 mg residual oil was obtained. Then 209 mg (1.0 mmol) of this residual oil was dissolved in 10 ml dichloromethane, a solution of 200 mg (0.56 mmol) 5'-O-(tert-butyldimethylsilyl)thymidine 1 and 0.16 ml triethylamine (1.1 mmol) in 10 ml CH₂Cl₂ was added with vigorous stirring under nitrogen at room temperature. The mixture was stirred at room temperature until TLC indicated that the reaction had gone to completion. The reaction mixture was transferred to 100 ml ethyl acetate, which was previously washed with saturated NaHCO₃ solution. Saturated NaHCO₃ and then water was added to wash the solution. The separated organic phase was dried over MgSO₄. A white foam (quantitative yield) was formed after the solvent was removed by evaporation under reduced pressure. Two components were found from ³¹P NMR spectra in a ratio of 1:5 (132.4ppm:130.8ppm). The reaction mixture in C₆D₆ was then refluxed for about 4 hours to get a ratio of up to 1:9 (d 137.8ppm:136.1ppm in benzene).

### Example 12

### Phosphoramidite (15)

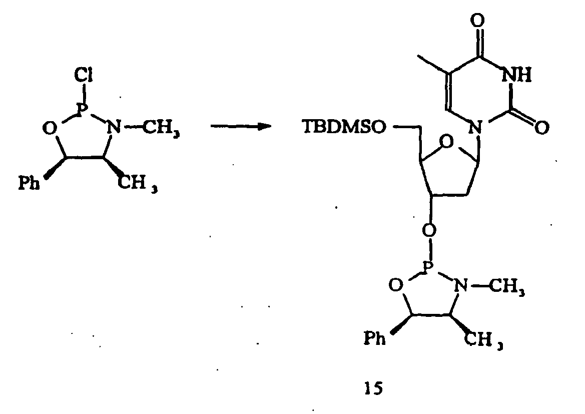

To a solution of 6.30 g (4.0 ml, 46 mmol) phosphorus trichloride in 100 ml dichloromethane was added a solution of 6.61 g (40 mmol) (1R,2S) ephedrine and 10.1 g (14 ml, 100 mmol) triethylamine in 50 ml dichloromethane with vigorous stirring under nitrogen at 0°C and then at room temperature for 0.5 hour. The solvent was removed by evaporation under reduced pressure and the residue was extracted with diethyl ether (3x100 ml). After removing the ether, 9.3 g residual oil was obtained. Then 345 mg (1.5 mmol) of this residual oil was dissolved in 40 ml dichloromethane, a solution of 356 mg (1.0 mmol) 5'-O-(*tert*-butyldimethylsilyl)thymidine 1 and 0.154 ml triethylamine (111 mg, 1.1 mmol) in 20 ml CH₂Cl₂ was added with vigorous stirring under nitrogen at -78°C. The mixture was stirrred at -78°C until TLC indicated that the reaction had gone to completion. The reaction mixture was quickly transferred to 150 ml ethyl acetate, which was previously washed with cold saturated NaHCO₃ solution. Cold saturated NaHCO₃ was added to wash the solution. The separated organic phase was dried over MgSO₄. A white foam (quantitative yield) was formed after the solvent was removed by evaporation under reduced pressure at 0°C. Two components were found from the ³¹p NMR spectrum in a ratio of 1:2 (147.3ppm:141.6ppm). The reaction mixture in CDCl₃ was then refluxed for about 4 hours to get a ratio of up to 1:5 (151.5ppm:143.5ppm). After chromatography on silica gel column (hexane:ethyl acetate:triethylamine=5:3:2), the slow eluting component (143.5ppm) was separated as a pure diastereomer.: ¹H NMR (500 MHz, CDCl₃) δ 7.23-7.50 (m, 5H, Ph), 6.35 (m, 1H, H-1'), 5.60 (d, J=6.8 Hz, 1H, PhCH), 4.71-4.75 (m, 1H, H-5'), 4.01-4.02 (m, 1H, H-4'), 3.78-3.87 (m, 2H, H-5'), 3.52-3.58 (m, 1H, MeCH), 2.64 (d, J=12.2 Hz, 3H, NMe), 2.31-2.36 (m, 1H, H-2'), 2.05-2.11 (m, 1H, H-2'), 1.90 (s, 3H, C=CMe), 0.90 (s, 9H, CMe₃), 0.60 (d, J=6.4 Hz, 3H, CHMe), 0.09 (s, 6H, SiMe₂); ¹³C NMR (125 MHz, CDCl₃) δ 163.9 (C-4), 150.5 (C-2), 137.9 (Ph), 135.3 (C-6), 128.1 (Ph), 127.7 (Ph), 126.7 (Ph), 111.0 (C-5), 86.7 (d, J=1.8 Hz, C-4'), 84.7 (OCPh), 84.6 (d, J=9.2 Hz, C-1'), 73.1 (d, J=18.3 Hz, C-3'), 63.0 (C-5'), 57.5 (d, J=5.6 Hz, NCMe), 40.6 (d, J=5.5 Hz, C-2'), 28.8 (d, J=17.4, NCH₃), 25.9 (SiCMe₃), 18.3 (SiCMe₃) 14.6 (d, J=3.7 Hz, NCHMe), 12.5 (C=CMe), -5.4 (SiMe₂), -5.5 (SiMe2); ³¹P NMR. (81 MHz, CD₃CN) δ 143.5

### Example 13

### Phopshite triester (20a) from phosphoramidite 13a with MeOH

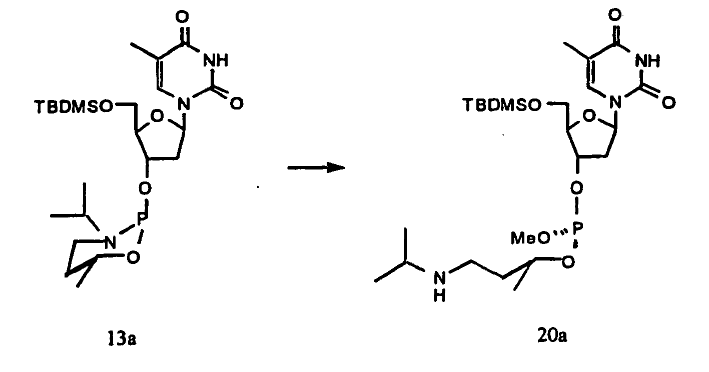

Diastereomeric pure phosphoramidite 13a (10 mg, fast eluting component) in 0.5 ml CDCl₃ was put in 5 mm NMR tube, and 50 µl of dry MeOH (a large excesses) was then added by syringe, followed by 1 mg of dicyanoimidazole. The reaction was monitored by ³¹P NMR until the reaction went to completion. The phosphite triesters formed in a ratio of 10:1 (139.4ppm:138.8ppm) and were purified on a silica gel column.: ¹H NMR (500 MHz, CDCl₃) δ 7.48 (s, 1H, H-6), 6.35 (dd, J=5.4, 8.8 Hz, 1H, H-1'), 4.78-4.82 (m, 1H, H-3'), 4.24-4.33 (m, 1H, OCH), 4.10 (d, J=2.4 Hz, 1H, H-4'), 3.76-3.89 (m, 2H, H-5'), 3.50 (d, J=10.7 Hz, 3H, CH₃), 2.78 (m, 1H, NCH), 2.65 (m, 2H, NCH₂), 2.40 (m, 1H, H-2'), 2.04 (m, 1H, H-2'), 1.89 (d, J=1.0 Hz, 3H, C=C-CH₃), 1.64-1.78 (m, 2H, OCHCH₂), 1.25 (d, J=6.4 Hz, 3H, OCHCH₃), 1.03 (d, J=6.3 Hz, 6H, NCHMe₂), 0.91 (s, 9H, SiCMe₃), 0.10 (d, J=2.0 Hz, 6H, SiMe₂); ¹³C NMR (125 MHz, CDCl₃) δ 163.5 (C-4), 150.1 (C-2), 135.3 (C-6), 110.9 (C-5), 86.5 (d, J=2.7 Hz, C-4'), 84.8 (C-1'), 72.1 (d, J=8.2 Hz, C-3'), 69.5 (OCH), 63.0 (C-5'), 49.2 (d, J=9.2 Hz, OCH₃), 48.8 (Me₂CNH), 40.3 (d, J=3.7 Hz, C-2'), 38.7 (d, J=4.6 Hz, CH₂), 25.9 (SiCMe₃), 22.9 (d, J=3.7 Hz, NCHMe),₂18.3 (SiCMe), ₃12.5 (C=CMe), -5.4 (SiMe₂); ³¹P NMR (121 MHz, CDCl₃) δ 139.4

### Example 14

### Phophite triester (20b) from Phosphoramidite 13b with Methanol

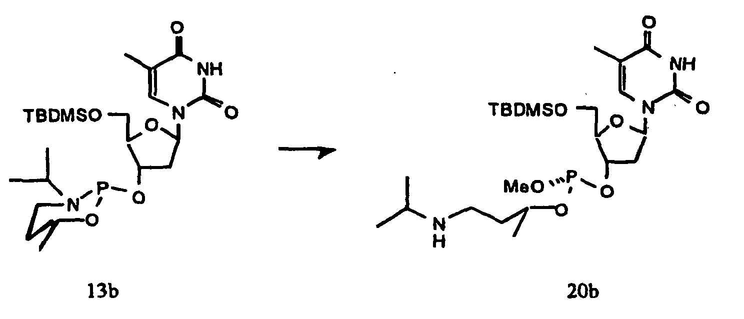

Diastereomeric enriched (75%) phosphoramidite 13b (10 mg, slow eluting component) in 0.5 ml CDCl₃ was put in 5 mm NMR tube, and 50 µl dry MeOH (a large excess) was then added by syringe, followed by 1 mg of dichloroimidazole. The reaction was monitored by ³¹P NMR until the reaction went to completion. The phosphite triesters formed in a ratio of 1:2 (139.4ppm:138.8ppm) were purified on a silica gel column. ¹H NMR (500 MHz, CDCl₃) 6 7.48 (s, 1H, H-6), 6.34 (m, 1H, H-1'), 4.78-4.82 (m, 1H, H-3'), 4.25-4.33 (m, 1H, OCH), 4.11 (d, J=2.4 Hz, 1H, H-4'), 3.77-3.89 (m, 2H, H-5'), 3.49 (d, J=10.3 Hz, 3H, CH₃), 2.76 (m, 1H, NCH), 2.64 (m, 2H, NCH₂), 2.39 (m, 1H, H-2'), 2.05 (m, 1H, H-2'), 1.90 (s, 3H, C=C-CH₃), 1.65-1.78 (m, 2H, OCCH₂), 1.26 (d, J=6.4 Hz, 3H, OCHCH₃), 1.04 (d, J=6.4 Hz, 6H, NCHMe₂), 0.91 (s, 9H, sicMe₃), 0.10 (d, J=2.0 Hz, 6H, SiMe₂) ³¹P NMR (121 MHz, CDCl₃) δ 138.8

### Example 15

### Phophite triester (32) from Phosphoramidite 13a with n-Butanol

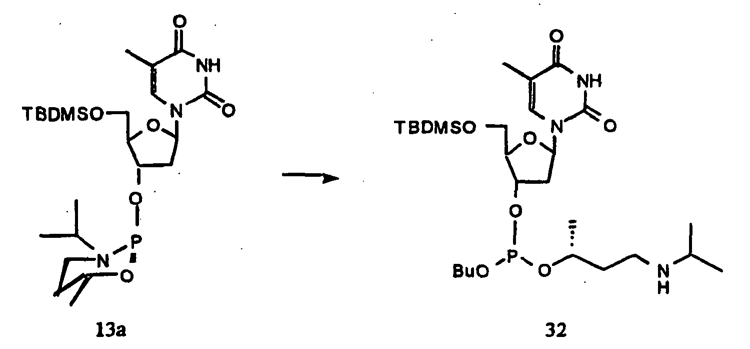

Diastereomerically pure phosphoramidite 13a (10 mg, fast eluting component) in 0.5 ml CDCl₃ was placed in 5 mm NMR tube, and 50 µl dry n-butanol (a large excess) was then added by syringe, followed by 1 mg of dicyanoimidazole. The reaction was monitored by ³¹P NMR until the reaction went to completion. The phosphite triesters formed in a ratio of 7:1 (138.9ppm:138.5ppm) were purified on a silica gel column. ¹H NMR (500 MHz, CDCl₃) δ 7.49 (d, J=1.5 Hz, 1H, H-6), 6.35 (dd, J=5.5, 8.8 Hz, 1H, H-1'), 4.83 (m, 1H, H-3'), 4.24-4.31 (m, 1H, OCH), 4.11 (d, J=2.0 Hz, 1H, H-4'), 3.71-3.89 (m, 4H, 2H-5', OCH₂), 2.78 (m, 1H, NCH), 2.66 (m, 2H, NCH₂), 2.39 (m, 1H, H-2'), 2.02 (m, 1H, H-2'), 1.89 (s, 3H, C=C-CH₃), 1.63-1.77 (m, 2H, OCMeCH₂), 1.57 (m, 2H, CH₂), 1.36 (m, 2H, CH₂), 1.24 (d, J=6.3 Hz, 3H, OCHCH₃), 1.04 (d, J=6.3 Hz, 6H, NCHMe₂), 0.91 (s, 9H, SiCMe₃), 0.10 (d, J=2.0 Hz, 6H, SiMe₂); ³¹P NMR (121 MHz, CDCl₃) δ 138.9.

### Example 16.

### Phosphite triester (33a) from phosphoramidite 7a with Methanol

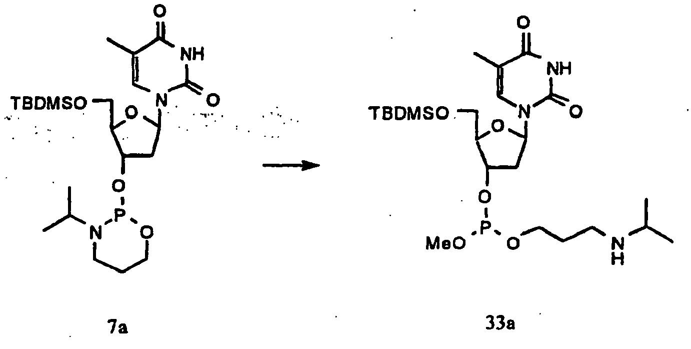

Diastereomerically pure phosphoramidite 7a (10 mg, fast eluting component) in 0.5 ml CDCl₃ was put in 5 mm NMR tube, and 50 µl dry methanol (a large excess) was then added by syringe, followed by 1 mg of 4,5-dicyano-2-bromoimidazole. The reaction was monitored by ³¹P NMR until the reation went to completion. The phosphite triester consisted of only one diastereomer (139.1ppm) and was purified on a silica gel column. ¹H NMR (500 MHz, CDCl₃) δ 7.48 (s, 1H, H-6), 6.34 (dd, J=5.4, 8.8 Hz, 1H, H-1'), 4.78 (m, 1H, H-3'), 4.09 (m, 1H, H-4'), 3.76-3.89 (m, 4H, 2H-5', OCH₂), 3.50 (d, J=10.2 Hz, 3H, OCH₃), 2.80 (m, 1H, NCHMe₂), 2.67 (t, J=6.8 Hz, 2H, NHCH₂), 2.39 (m, 1H, H-2'), 2.05 (m, 1H, H-2'), 1.90 (s, 3H, C=C-CH₃), 1.78 (m, 2H, OCHCH₂), 1.03 (d, J=6.3 Hz, 6H, NCHMe₂), 0.91 (s, 9H, SiCMe₃), 0.10 (s, 6H, SiMe₂); ³¹P NMR (121 MHz, CDCl₃) δ 139.1; MS(CI) m/e: 534 ([M+H⁺], 100%), 502 (14.4), 376 (13.6), 339 (74.6), 281 (20.4), 164 (59.8)

### Example 17

### Phophite triester (33b) from phosphoramidite 7b with Methanol

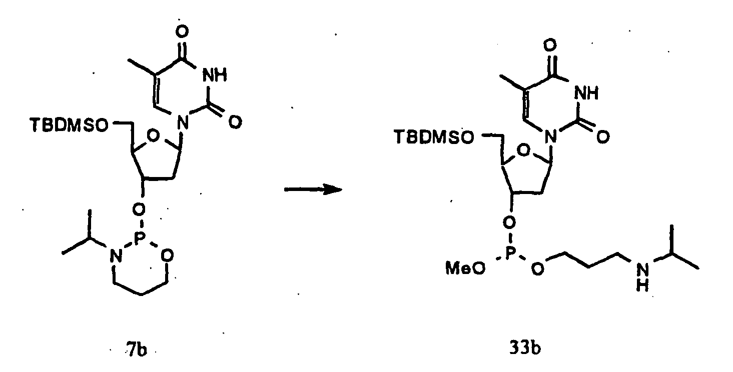

Diastereomerically enriched (92%) phosphoramidite 7b (10 mg, slow eluting component) in 0.5 ml CDCl₃ was placed in 5 mm NMR tube, and 50 µl dry methanol (a large excess) was then added by syringe, followed by 1 mg of dicyanobromoimidazole. The reaction was monitored by ³¹P NMR until the reaction went to completion. Diastereomerically enriched phosphite triester 33b (92%) in a ratio of 1:11 (139.2ppm:138.8ppm) was purified on a silica gel column. ³¹P NMR (121 MHz, CDCl₃) δ138.8

### Example 18

### Thiophosphonate (44)

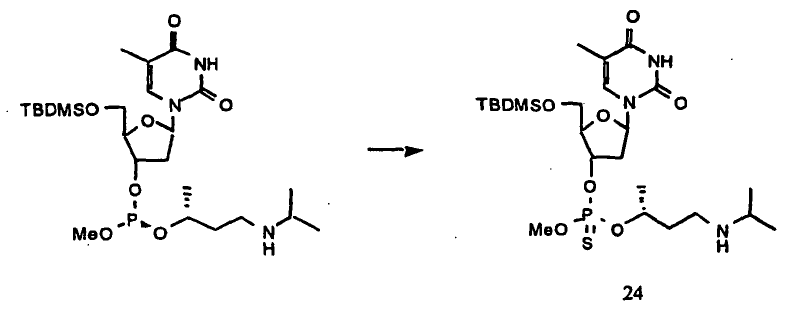

Diastereomerically pure 13a (100 mg, fast eluting component) was dissolved in 5 ml CDCl₃, and 0.5 ml MeOH was then added by syringe, followed by 5 mg of 2-bromo-4,5-dicyanoimidazole. The reaction was monitored by ³¹P NMR until the reaction went to completion with almost only one diastereomer of phosphite triester 20a (139.4 ppm) being found, and then 10 mg sulfur was added. Within 5 minutes, the sulfurization went to completion. After chromatography (ethyl acetate:triethylamine=1:1), sulfurized product 24 was obtained in an oily form. ¹H NMR (500 MHz, CDCl₃) δ 7.50 (d, J=1.4 Hz, 1H, H-6), 6.36 (dd, J=5.4, 9.3 Hz, 1H, H-1'), 5.08 (m, 1H, H-3'), 4.63-4.72 (m, 1H, OCH), 4.25 (m, 1H, H-4'), 3.88 (m, 2H, H-5'), 3.73 (d, J=13.7 Hz, 3H, OCH₃), 2.77 (m, 1H, NCHMe)₂, 2.67 (t, J=6.4 Hz, 2H, NCH₂), 2.48 (m, 1H, H-2'), 2.09 (m, 1H, H-2'), 1.90 (d, J=1.5 Hz, 3H, C=C-CH₃), 1.70-1.86 (m, 2H, OCCH₂), 1.32 (d, J=5.9 Hz, 3H, OCHCH₃), 1.04 (dd, J=2.9, 5.9 Hz, 6H, NCHMe₂), 0.92 (s, 9H, SiCMe₃), 0.12 (s, 6H, SiMe₂); ³¹P NMR (121 MHz, CDCl₃) δ 67.5

### Example 19

### 2-Bromo-4,5-dicyanoimidazole (21)

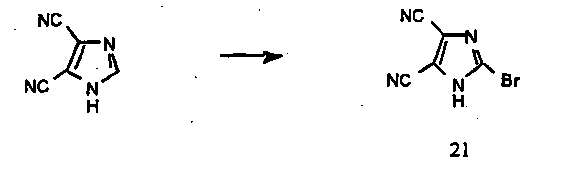

To 1.18 g (10 mmol) 4,5-dicyanoimidazole and 25 ml 0.1 M NaOH was added 1.8 ml Br₂ (35 mmol). The mixture was stirred overnight at room temperature and then acidified with dilute HCl. The solid was filtered, rinsed with water and recrystallized from water to give 1.5 g of dicyanobromoimidazole 21 (yield 76.4%).: m.p. 147-149°C (lit. 141-143°C); Rf=0.65 (ethyl acetate:methanol=4:1); MS(EI): 198 ([M+2], 96%), 196 ([M⁺], 100%),171 (28.5), 169 (29.2), 117 (27.4), 91 (19.0), 64 (20.6), 53 (22.4), 38 (18.8).

### Example 20

### Synthesis of (S)-methyl-3-(5-imidazolyl)-2-hydroxypropionate (3) hydrochloride

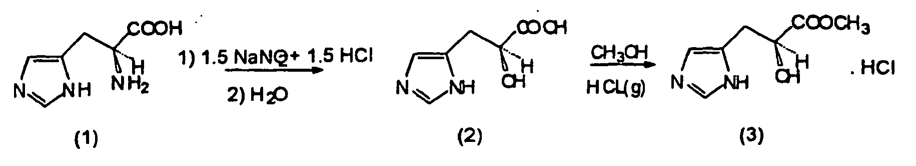

(L)-Histidine (3.103 g, 20 mmol) was first dissolved in 30 ml of 1 N hydrochloric acid solution. This solution was cooled down to 0°C, then a solution of sodium nitrite (2.070 g, 30 mmol) in 10 ml distilled water was added dropwise over a period of 1 hour. The solution was stirred overnight at 0°C, then evaporated to dryness in vacuo with heating. 20 ml of distilled water was added to the solid residue, the mixture was evaporated once more with toluene in order to azeotropically remove the water residue as much as possible. After drying the compound in high vacuum overnight and without isolation of the intermediate acid, the mixture was dissolved into 50 ml of dry methanol and stirred under Ar. This solution was cooled down to 0°C and a stream of gaseous hydrogen chloride was bubbled through the mixture. After 1.5 hours, TLC indicated disappearance of the acid and the reaction was stopped. The mixture was evaporated in vacuo with heating to yield a sticky yellow solid that could be crystallized from a mixture of ethanol and ether to yield 3.10 g of (2). HCl, m.p. 139-142°C, [α]_{D} -21° (c 1.9, methanol, 25°C) (litt. -22°) ¹HNMR (200MHz, CD₃OD) δ 8.90 (s, 1H, NCHN); 7.30 (s, 1H, NCHC); 4.40 (dd, ABX, ³J_{Ha-Hx}=5.40Hz ³J_{Hb-Hx}=5.00Hz, CHOH); 3.72 (s, 3H, OCH₃); 2.85-3.10 (ABX, 2H, ²J_{Ha-Hb}=13.75 Hz, ³J_{Hb-Hx}=5.0Hz, ³J_{Ha-Hx} =5.4Hz, CH₂); ¹³C NMR (non decoupled) (125 MHz, D₂O) δ 175.3 (s,CO); 134.2 (d, NCHN); 129.7 (s, CH₂CN); 117.6 (d, CCHN); 69.9 (d, CHOH); 53.6 (q, CH₃); 29.5 (t,CH₂); M.S. (M+1)⁺ 171

### Example 21

### Synthesis of imidazo-oxazaphospholidine (4)

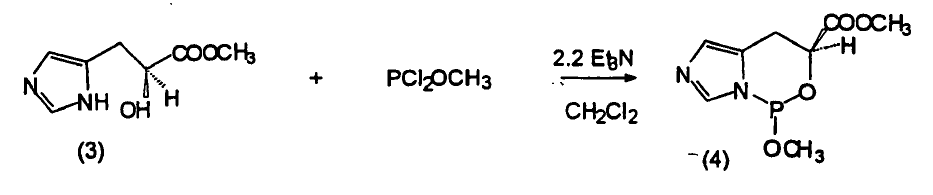

In scrupulously dry glassware, compound (3) (0.236 g, 1.39 mmol) was added and dried in vacuo overnight, then put under an atmosphere of Ar. This compound was suspended into 5 ml of dry ether, then triethylamine (0.20 ml, 3.15 mmol) was added. The suspension was cooled down to 0°C and stirred under Ar. Then, methyl dichlorophosphite (0.15 ml, 1.58 mmol) was syringed into the mixture quickly. As soon as the phosphite was introduced, a thick white precipitate was observed, corresponding to the formation of triethylammonium chloride. After 15 min, ³¹P NMR showed several signals between 176 and 120 ppm, as well as after 2 to 4 hours. After overnight stirring at room temperature, ³¹P NMR showed a single signal at 143.5 ppm. Compound decomposed upon trying to handle it (dilution in dry ether, filtration in an Ar atmosphere, concentration by evaporation of the ether) as indicated by ³¹P NMR by several peaks at 5-20 ppm, corresponding most likely to H-phosphonates derivatives. Changing the reaction conditions did not bring any improvement.
Therefore, compound (4) could not be further purified and analyzed.

### Example 22

### Synthesis of 1-tritylimidazole (5)

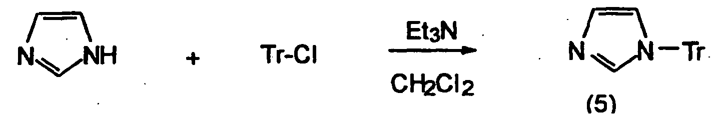

To a solution of trityl chloride (5.58 g, 20.0 mmol.) in dry methylene chloride (100 ml) cooled down to 0°C and stirred under Ar, was added dropwise over 1.5 hours a solution of imidazole (1.36 g, 20.0 mmol.) and triethylamine (2.7 mml, 20 mmol.) in 50 ml dry methylene chloride. At the end of the addition, the reaction mixture was allowed to warm up to room temperature and stirred under Ar at that temperature overnight. The reaction mixture was then washed with 20ml of a 10% solution of ammonium chloride, then with 20 ml of distilled water. The organic phase was dried over magnesium sulfate and evaporated *in vacuo* to yield quantitatively a white solid. Recrystallization from methylene chloride/hexanes yielded 5.60 g of (5) (yield = 90% after recrystallization).
m.p. 214°C; ¹H NMR (200 MHz, CDCl₃) δ 7.43 (m, 1H, NCHN), 7.3-7.4 (m, 9H, 3xC₆H₅), 7.1-7.2 (m, 6H, 3xC₆H₅), 7.0 (m, 1H, Ph₃CNCH=CH), 6.81 (m, 1H, Ph₃CNCH=CH); ¹³C NMR (50 MHz, CDCl₃) δ 142.3, 139.0, 129.6, 128.2, 128.3, 121.6.

### Example 23

### synthesis of (S)-1-(2-(1-triphenylmethyl)-imidazolyl)-propan-2-ol (S)-(6)

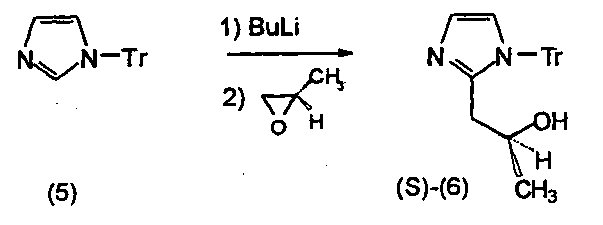

To a solution of N-tritylimidazole (1.55 g, 5 mmol) in freshly distilled THF (50 ml) cooled down to -78°C and stirred under dry Ar, was added a 2.5 M solution of n-butyllithium in pentane (2.4. ml, 6 mmol). The addition lasted for 30 min, and the deep red solution obtained was allowed to warm up to 0°C, stirred at room temperature for 1 hour, then cooled down again to -78°C. At that temperature, (S)-propylene oxide (0.35g, 6 mmol) was added dropwise. After 30 min, the solution was allowed to warm up to 0°C and was stirred at that temperature for 12 hours until TLC indicated that the reaction did not further proceed. The solution was poured into 50 ml of saturated NH₄Cl solution, and the resulting mixture was extracted with CH₂Cl₂. After flash chromatography (hexane:acetone:triethylamine 78:21:1), 1.44g of the pure product was collected in 78% yield: m.p. 201°C; ¹H NMR (200 MHz, CDCl₃) δ 7.20-7.40 (m, 9H, 3xC₆H₅), 7.10-7.18 (m, 6H, 3xC₆H₅), 6.90 (d, 1H, ³J_{H-H}=1.2; CHNCPh₃), 6.71(d, 1H, ³J_{H-H}=1.2; CH=CHNCPh₃), 5.83 (b, 1H, OH), 3.40-3.60 (m, 1H, CHCH₃), 1.78-2.05 (ABX, 2H, ³J_{Hb-Hx}=3.2Hz, ³J_{Ha-Hx}= 8.5Hz, ²J_{Ha-Hb}=16.2Hz, CH₂), 0.81 (d, ³J_{H-H}=6.0Hz, 3H, CH₃); ¹³C NMR (50 Mhz, CDCl₃) δ _149.2, 142.1, 129.6, 127.9, 127.7, 124.7, 121.0 (NCCN), 74.6, 65.0 (CHOH), 38.1 (CH₂), 22.3 (CH₃).

### Example 24

### Synthesis of (S)-1-(2-imidazolyl)-propan-2-ol (S)-(7)

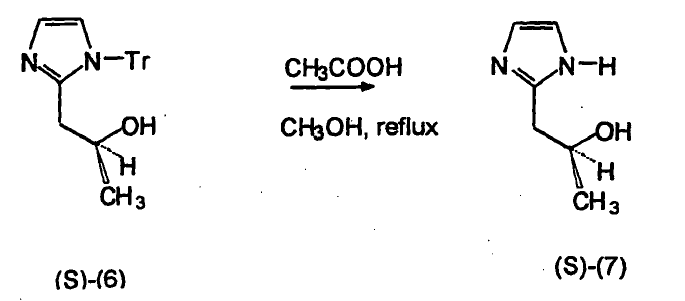

A solution of N-tritylimidazolylpropanol (S)-(6) (2.39 g, 6.51 mmol) in 80 ml methanol containing 4.3 ml glacial acetic acid (5%) was refluxed for about 12 hours. After that time, TLC indicated disappearance of the starting materials. The mixture was concentrated *in vacuo* and a white precipitate appeared upon addition of 50 ml of cold distilled water. The mixture was chilled, then filtered, and the white precipitate was washed with cold distilled water (10ml). The filtrate was then evaporated twice, and the residual yellow oil redissolved in 50 ml dry methanol and passed through the weakly basic anion exchange resin (hydroxide form) IRA-68. The solution was then evaporated to yield a solid residue that could be recrystallized from a mixture of methanol and ethyl acetate. yield: 0.80g, 98% of pure (S)-(7). m.p. 119-121°C; ¹H NMR (200 Mhz, CD₃OD) δ 6.96 (s, 2H, NCHCHN); 3.96 (m, 1H, CHCH₃); 2.4-2.65 (ABX, ³J_{Ha-Hx}=6.3Hz, ³J_{Hb-Hx}=6.7Hz, ³J_{Ha-Hb}=14.5Hz, CH₂); 0.87 (d, 3H, ³J_{H-H}=6.3Hz, CH₃); ¹³C NMR (50MHz, CD OD) δ 145.8 (s, CH2CNH); 121.2 (d, NCH=CHNH); 67.1 (d, CHOH); 38.4 (t, CH2); 23.1 (q, CH3); MS (CI): [M+1]⁺. 127

### Example 25

### Synthesis of 1-mothoxy-3-methyl-imidazo-[2,1-e]-(3,4-dihydro)-oxazaphosphorine (8)

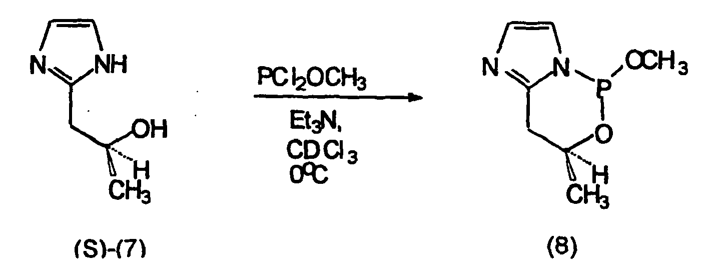

In an NMR tube previously dried in vacuo and under Ar was introduced 23.0 mg (0.20 mmol) of (S)-(2-imidazolyl)-propan-2-ol (S)-(7), then the tube was sealed with a septum and flushed with Ar. CDCl₃ (0.7 ml) was then introduced, followed by 127 µl (1.0 mmol) of triethylamine. The alcohol did not dissolve, and this suspension was cooled down to 0°C while shaking the tube. At that temperature, 18.9 µl (0.20 mmol) of methyl dichlorophosphite was syringed inside the tube. Upon shaking, the alcohol dissolved instantaneously, and an exothermic reaction was noticed. After about 1 hour, ³¹P NMR revealed the presence of several peaks around 120-140 ppm. After about 3 hours, a single product (8) was observed by ³¹P NMR, as evidenced by its chemical shift at 118.8 ppm.. There was no further characterization of this compound, as all efforts to isolate it have been unsuccessful and have led to the hydrolysis of this extremely water sensitive bicyclic structure, most likely to the corresponding H-phosphonate.

### Example 26

### Synthesis of 1-ethoxy-3-methyl-imidazo-[2,1-e]-(3,4-dihydro)-oxazaphosphorine (9)

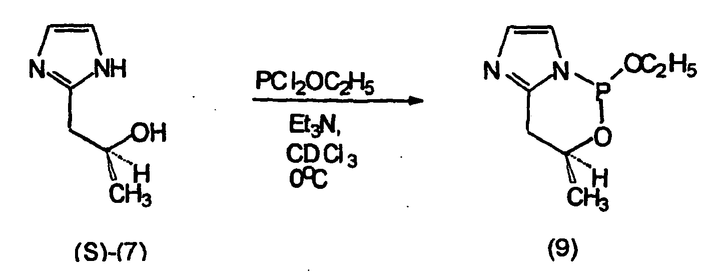

To an NMR tube previously dried in vacuo and under Ar, was introduced 18.9 mg (0.15 mmol) of (S)-(2-imidazolyl)-propan-2-ol. (S)-(7). The tube was then sealed with a septum and flushed with Ar. Then, 0.7 ml CDCl₃ was introduced, followed by 105 µl (0.75 mmol) of triethylamine. The alcohol did not dissolve, and this suspension was cooled down to 0°C while shaking the tube. At that temperature, 17.2 µl (0.15 mmol) of ethyl dichlorophosphite was syringed into the NMR tube. Upon shaking, the alcohol dissolved instantaneously, and an exothermic reaction was noticed. After about 2 hours, a single product was observed by ³¹P NMR as evidenced by its chemical shift at 118.2 ppm. After about 1 hour, the presence of other products around 120-140 ppm was noticed, one of which (121.1 ppm) is probably the other diastereomer. There was no further characterization of this compound.

### Example 27

### Synthesis of thiophosphate ethyl (S)-1-(2-imidazolyl)-prop-2-yl isopropyl ester (22)

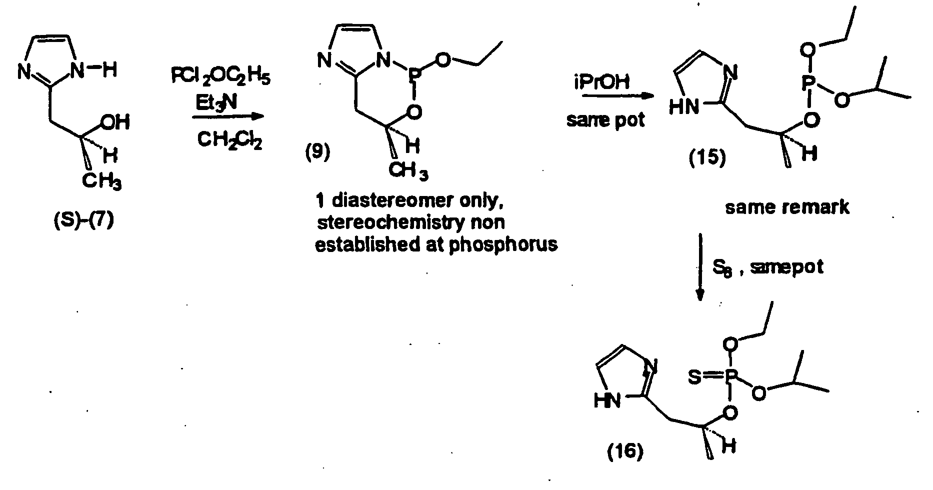

In an NMR tube, to a suspension of 18.9 mg (0.15 mmol) (S)-1-(2-imidazolyl)-propan-2-ol (S)-(7) in 0.7 ml dry deuterated chloroform and 0.21 ml triethylamine (1.5 mmol), shaken at room temperature under Ar was introduced with a syringe 17.2 µl (0.15 mmol) of ethyl dichlorophosphite. The reaction mixture was shaken at room temperature and the chiral imidazolylpropanol dissolved immediately in an exothermic process. At that point, ³¹P NMR indicated the formation of several products, among which was one having a signal at 118.3 ppm and one having a signal at 120.5 ppm. (suspected diastereomers). After one hour and regular shaking of the NMR tube, ³¹P NMR indicated only one peak at 118.3 ppm. At that stage, 20 µl (0.45 mmol) of isopropanol was introduced, and the tube was shaken again. ³¹P NMR indicated after 20 min the presence of a single peak at 140.6 ppm, indicating that the displacement of the imidazole moiety had given rise to a single diastereomer (19). 32 mg sulfur (1 mmol) was then introduced and the ³¹P-NMR was again recorded. The spectrum indicated a single peak at 64.8 ppm. The product (22) was then concentrated *in vacuo* and purified by flash chromatography (ethyl acetate / hexanes / triethylamine 79/20/1). ¹H NMR (200 MHz, CDCl₃) 6.96 (s, 2H, NCH=CH-N); 4.83-4.97 (m, 1H, P-O-CH-CH₃); 4.58-4.75 (dh, 1H, ³J_{H-H}= 6.2 Hz, ³J_{H-P}= 9.6 Hz , OCH (CH₃)₂); 3.96-4.12 (m, 2H, P-O-CH₂CH₃); 2.98-3.20 (2xABX, 2H, ²J_{Ha-H} = 15.5Hz, ³J_{Ha-Hx}= 6.23Hz, ³J_{Hb-Hx}= 4.6 Hz, ⁴J_{H-P} = 1.5Hz, CH₂CHCH₃); 1.21-1.34 (m, 12H, CH(CH₃) + CH(CH₃)₂ + CH₂CH₃) ¹³C NMR (50 MHz , CDCl₃) 144.00 (s, N-C=N); 121.70 (s, N-C=C-N); 74.90 (d, ²J_{C-P}= 6.1Hz, P-O-CH-(CH₃)CH₂); 73.80 (d, ²J_{C-P}=5.7 Hz, P-O-CH₂-CH₃); 6 4 . 2 5 (d, ²J_{C-} p=5.8 Hz, P-O-CH(CH₃)₂); 35.95 (s, P-O-CH(CH₃)CH₂-); 23.40 (s, P-O-CH(CH₃)₂); 21.06 (s, P-O-CH(CH₃)CH₂-); 15.83 (s, P-O-CH₂₋CH₃) 31p NMR (81 MHz, CDCl₃) δ 64.8 ppm M.S. (CI), [M+1]⁺ 293.

### Example 28

### Synthesis of thiophosphate ethyl (S)-1-(2-imidazolyl)-prop-2-yl 5' terbutyldimethylsilylthymidinyl ester (19)

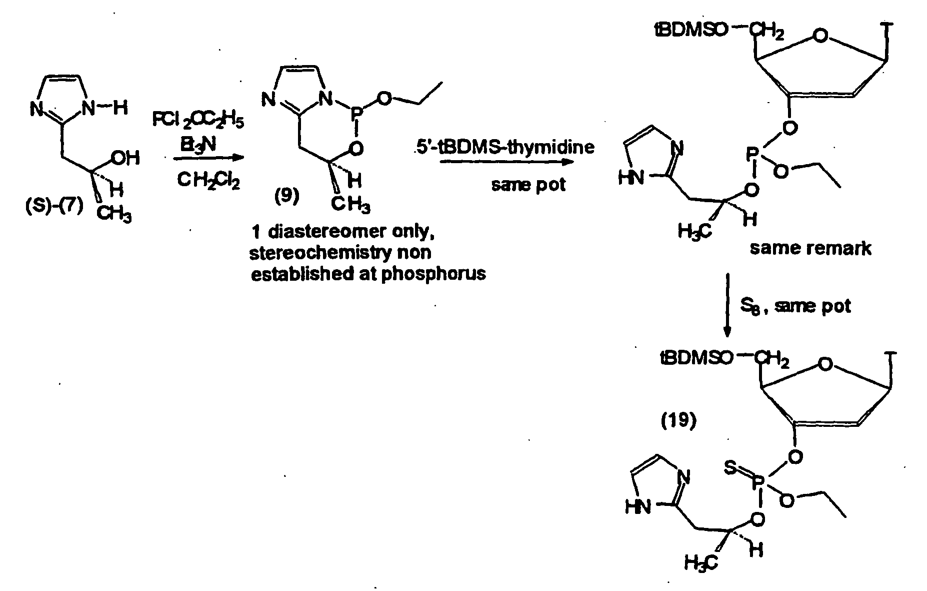

To a suspension of (S)-imidazolylpropanol (S)-(7) (0.30 mmol, 37.8 mg) in 2 ml dry dichloromethane and triethylamine (1.5 mmol, 0.21 ml) cooled down to 0°C and stirred under Ar, was slowly added ethyl dichlorophosphite (0.30 mmol, 35 *µ*l). The reaction mixture was then allowed to warm up to room temperature, the solid starting materials dissolved, and after about 2 hours, ³¹P NMR indicated the presence of a single peak at 118.3 ppm. At that time, the mixture was cooled down to 0°C again, and at that temperature was added a mixture of 5'-tBDMS-thymidine (1) (0.30 mmol, 106 mg) in 1.5 ml of dry methylene chloride. The reaction mixture was allowed to warm up to room temperature again. ³¹P NMR indicated, after 30 min, a full conversion of the peak at 118.3 ppm to a single peak at 141.2 ppm, assigned to (23). Elemental sulfur S₈ (0.9 mmol, 29 mg) was then added after about 30 minutes. ³¹P NMR indicated conversion of the previously formed product to a single product (24) with a peak at 66.3 ppm. Evaporation of the reaction mixture followed by flash chromatography (ethyl acetate/triethylamine 80/20) afforded thioate (24) as a sticky solid (127 mg, 72%). ¹H NMR (300 MHz, CD₂Cl₂) δ 7.48 (d, 1H, ⁴J_{H.H}=1.3Hz, C=CH); 6.94 (s, 2H, NCH=CHN); 6.25 (dd, 1H, ³J_{H-H}=5.1Hz, ³J_{H-H}=9.2Hz, NCHO); 4.84-5.05 (m, 2H, POCH(CH₃)CH₂ + POCHCH₂O); 4.15-4.24 (m, 1H, SiOCH₂CHO); 3.96-4.14 (dq, 2H, ³J_{H-P}=9.4Hz, ³J_{H-H}=7.0Hz, POCH₂CH₃); 3.76-3.92 (ABX, 2H, ²J_{Ha-Bb}=11.5Hz, ³J_{Hb-Hx}=2.5Hz, ³J_{Ha-Hx}=2.4Hz, SiOCH₂CHO); 2.98-3.08 (dd, 2H, ³J_{H-H}=5.8Hz, ⁴J_{H-P}=1.1Hz, POCH(CH₃)CH₂-Im); 1.98-2.08 (m, 2H, POCHCH₂CHN); 1.88 (s, 3H, CH=CCH)₃; 1.39 (d, 3H, ³J_{H-H}=6.2Hz, POCH(CH₃)); 1.28 (dt, 3H, ³J_{H-H}=7.0Hz, ⁴J_{H-P}=0.9Hz, POCH₂CH₃); 0.91 (s, 9H, SiC(CH₃)₃); 0.12 (s, 6H, Si(CH₃)₂).
¹³C NMR (121 MHz, CD₂Cl₂, decoupled from ¹H) δ 164.1 (s, NCOC(CH₃)); 151.1 (s, NCON); 144.2 (s, NC=N); 135.5 (s, NC=C(CH₃)C=O); 132.4 (s, NC=CN); 186.1 (s, 11.5 (C(CH₃)CO); 86.1 (s, SiOCH₂CHO); 86.0 (s, NCHO); 79.9 (d, ²J_{C-P} =4.4Hz, POCH(CH₃)); 76.2 (d, ²J_{C-P}=5.7Hz, POCH₂CH₃); 65.0 (d, ²J_{C-P} =5.6Hz, POCHCHO); 63.8 (s, SiOCH₂); 39.4 (s, POCHCH₂CHN); 36.6 (POCH(CH₃)CH₂); 26.1 (SiC(CH₃) ₃) ; 21.3 (POCH(CH₃)); 18.6 (SiC(CH₃)₃); 16.0 (s, POCH₂CH₃); 12.7 (s, C=CCH₃); -5.4 (d, Si(CH₃)₂); MS (CI) (M+1)⁺ 589.

### Example 29

### 1,2-O-3,5-O-dicyclopentylidene-D-xylofuranose (25)

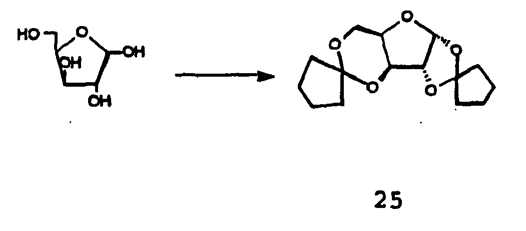

To a solution of trimethyl orthoformate (5mmol, 547µl) and p-toluene sulfonic acid (0.2mmol, 38mg) in dioxane (10ml) under a nitrogen atmosphere at 0°C, was added dropwise cyclopentanone (40mmol, 3.5ml). This solution was stirred at room temperature for 2 hours and D-xylose (2mmol, 300mg) was added with stirring continued overnight. Then the reaction mixture was neutralized with triethylamine. Evaporation of the solvent furnished a yellow syrupy residue. A solution of the syrupy residue in chloroform (20ml) was washed with water (20ml). The aqueous layer was extracted with chloroform (3x15ml). The combined chloroform layers were dried (MgSO₄). After removing the solvent, the mixture was chromatographed on a silica gel column (Hexane:Ethyl acetate=5:1) to give 340mg white solid (60%). ¹HNMR(200 MHz, CDCl3) δ 5.99 (δ, J=3.80 Hz, 1H, H-1), 4.45 (d, J=3.81Hz, 1H, H-2), 4.24 (d, J=2.15Hz, 1H, H-3), 4.14 - 3.46 (m, 3H, H-4, 2xH-5), 1.98 - 1.55 (m, 16H, cyclopentylidene protons); ¹³C NMR (125 MHz, CDCl₃) δ 121.23 (C-1OCOC-2), 109.24 (C-30COC-5), 105.08 (C-1), 84.49 (C-2), 74.25 (C-4), 71.83 (C-3), 61.50 (C-5), 39.52, 36.82, 36.19, 29.74, 24.09, 23.49, 22.80, 22.37 (cyclopentylidene carbons); MS (CI) m/e: 283(M+H⁺).

### Example 30

### 1,2-O-cyclopentylidene-D-xylofuranose (26)

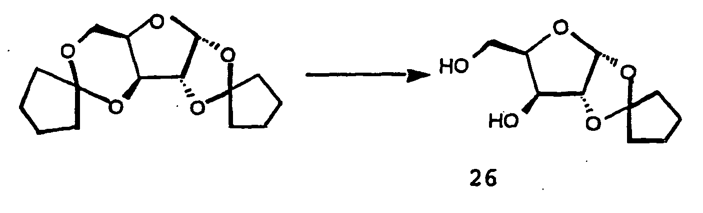

The 1,2-O-3,5-O-dicyclopentylidene-D-xylofuranose (25) (1mmol, 282mg) was dissolved in acetic acid-water (2:1) (14ml) at room temperature. The reaction mixture was stirred for 3 hours, followed by TLC. Solvent was evaporated with high vacum, and coevaporated with methanol three times and dried in vacuo overnight to give 196 mg white solid (91%). ¹H NMR (50OMHz, CDCl₃) δ 5.94 (d, J=3.91Hz, 1H, H-1), 4.44 (d, J=3.91Hz, 1H, H-2), 4.32 (d, J=2.44Hz, 1H, H-3), 4.18 (m, 1H, H-4), 4.10 - 4.02 (m, 2H, 2xH-5),1.96 - 1.61 (m, 8H, cyclopentylidene protons); ¹³C NMR (125MHz, CDCl₃) δ 121.41 (OCO), 104.53 (C-1), 85.59 (C-2), 78.76 (C-3), 76.83 (C-4), 61.07 (C-5), 36.84, 36.19 (CH₂CCH₂), 23.47, 22.80 (CH₂CH₂CCH₂CH₂); MS (CI) : m/e 217 (M+H⁺).

### Example 31

### 1,2-O-cyclopentylidene-5'-O-tosyl-D-xylofuranose (27)

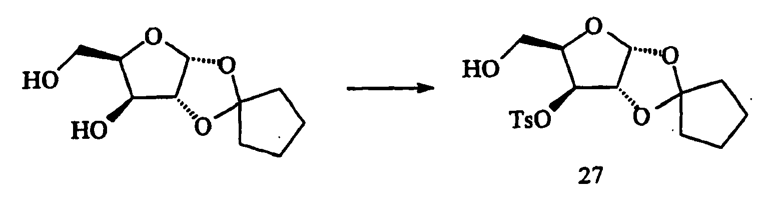

To a solution of 1,2-O-cyclopentylidene-D-xylofuranose (26) (0.81mmol, 176mg) in dry pyridine (6ml) under nitrogen atmosphere at 0°C, p-toluenesulfonyl chloride (1.12eq., 173mg) was added. The reaction mixture was stirred at 0°C for 3 hours. Then 5 ml water was added to quench the reaction, and the solvent was evaporated in high vacuum, and coevaporated with toluene twice. The mixture was dissolved in chloroform, washed with water three times and dried over MgSO₄. Removal of the solvent furnished 259 mg (27) as a white solid (86%). ¹H _{NMR} (200MHz, CDCl₃) δ 7.80 - 7.32 (AA'BB', 4H, Ph), 5.84 (d, J=3.63Hz, 1H, H-1), 4.44 (d, J=3.7Hz, 1H, H-2), 4.42 - 4.29 (m, 3H, H-3, 2xH-5), 4.27-4.09 (m, 1H, H-4), 2.44 (s, 3H, CH₃), 1.89-1.61 (m, 8H, cyclopentylidene protons); ¹³C NMR (270MHz, CDCl₃) 145.34 , 132.30, 130.06, 128.08 (aromatic), 121.86 (OCO), 104.80 (C-1), 85.15 (C-2), 77.73 (C-3), 74.41 (C-4), 66.21 (C-5), 37.02, 36.44(CH₂CCH₂), 23.54, 22.98 (CH₂CH₂CCH₂CH₂), 21.73 (CH₃); MS (CI) : m/e 371 (M+H⁺).

### Example 32

### 1,2-O-dicyclopentylidene-5'-isopropylamine-D-xylofuranose (28)

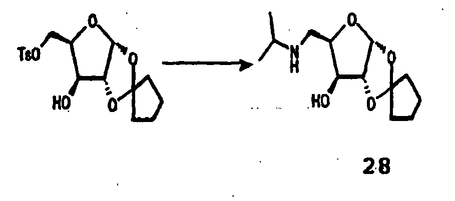

A solution of 1,2-O-cyclopentylidene-5'-tosyl-D-xylofuranose (27) (7.1mmol, 2.64g) in isopropylamine (15ml) was stirred at 55°C over night in a pressure bottle. The solvent was removed by rotary evaporator and the remaining yellow syrup was taken up with chloroform and washed with a saturated solution of sodium bicarbonate, and then with brine. The organic phase was dried over MgSO₄, the solvent was evaporated and the residue was flash chromatographed on silica gel (ethyl acetate-3% triethylamine) to furnish
1.33g (28) as a white solid (73%), m.p.44-45°C; [α]_{D}²⁰ = 31.06 (C=2,CHCl₃); ¹H NMR (500MHz, CDCl₃) δ 8.0(bs, NH) 5.90 (d, J=3.91Hz, 1H, H-1), 4.38 (d, J=3.91Hz, 1H, H-2), 4.27 (d, J=2.44Hz, 1H, H-3), 4.20 (d, J=2.93Hz, 1H, H-4), 3.36 - 2.92 (ABX, 2H, 2xH-5), 2.74-2.70 (heptet, 1H, NCH), 1.95 -1.63 (m, 8H, cyclopentylidene protons), 1.04-1.03 (dd, 6H, Me₂CH); ¹³C NMR (500MHz, CDCl₃) δ 121.06 (OCO), 104.82 (C-1) 86.14 (C-2), 78.30 (C-3), 77.07(C-4), 48.64(NCH), 45.90(C-5), 36.85, 36. 32 (CH₂CCH₂), 23.51, 22.84 (CH₂CH₂CCH₂CH₂) 22.64 (CH₃CHN), 22.34(CH₃CHN); MS (CI) : m/e 258([M+H⁺], 100%); HRMS(EI) m/e calc'd for C₁₃H₂₃NO₄) [M⁺]: 257.16270, found 257.1630.

### Example 33

### Chlorophosphoramidite (29)

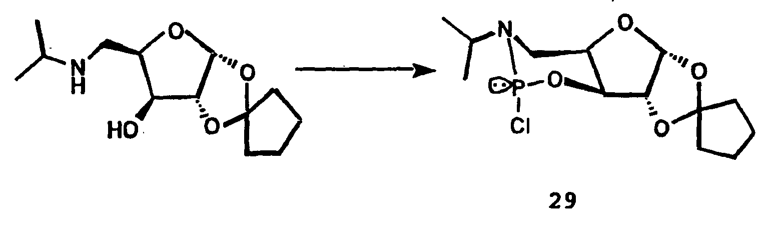

In a scrupulously dry NMR tube, 9.6 µl (0.11mmol) phosphorus trichloride was placed via syringe, followed by 0.25ml CDCl₃. The NMR tube was cooled at 0°C, and a solution of the 1,2-O-dicyclopentylidene-5'-isopropylamino-D-xylofuranose 28 (25.7mg, 0.1mmol) and triethylamine (27.8ul, 0.22mmol) in CDCl₃ (0.35ml) were added under a nitrogen atmosphere with shaking of the NMR tube. An exothermic reaction was noticed. The NMR tube was then cooled to -78°C, pumped and sealed. The sealed NMR tube was heated to 40°C and the reaction was followed by ³¹P NMR until a single peak was found in the ³¹PNMR spectrum. The product was not isolated, and was used directly in the following step. ¹H NMR (500MHz, CDCl₃) δ 5.75 (d, J=3.91Hz, 1H, H-1), 4.51 (t, J=2.44Hz, J=2.93Hz, H-3), 4.37(d, J=3.91Hz, 1H, H-2), 4.19-4.17(m, 1H, H-4), 3.42-3.34(heptet, 1H, NCH), 3.33-3.00 (ABX, 2H, 2xH-5), 1.81-1.51(m, 8H, cyclopentylidene protons), 1.05(d, J=6.84Hz, 6H, Me₂CH); ¹³C NMR (125MHz, CDCl₃) δ 121.48 (OCO), 104.26 (C-1), 83.86, 83.83(d, J=3.66Hz, C-2), 73.04, 72.99(d, J=6.41Hz, C-3), 72.35(C-4), 50.42, 50.13(d, J=35.72Hz, C-5), 36.95, 36.91(d, J=5.50Hz, NCH), 36.62, 35.91(CH₂CCH₂), 23.22, 22.50(CH₂CH₂CCH₂CH₂), 20.89, 20.79(d, J=12.82Hz, CH₃CHN), 19.64, 19.60(d, J=5.50Hz, CH₃CHN); ³¹P NMR (202MHz, CDCl₃) δ 148.42.

### Example 34

### 5'-O-(tert-butyl dimethyl silyl)-thymidine-3'-O-Phosphoramidite (30)

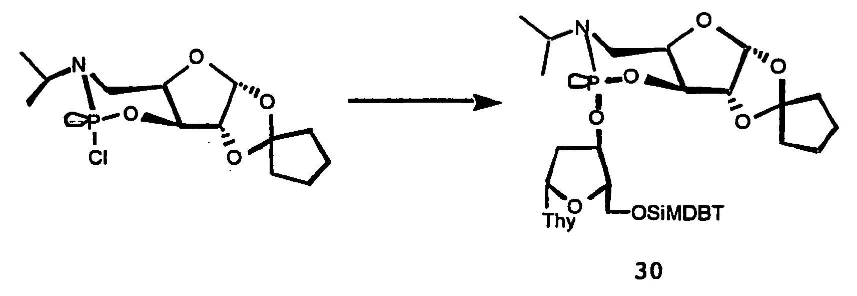

To the same NMR tube from the example 29, a solution of 5'-O-(tert-butyldimethylsilyl)-thymidine (35.6mg, 0.1mmol) in 0.45ml CDCl₃ and triethylamine (14µl, 0.11mmol) was added slowly at 0°C under nitrogen atmosphere. Then the NMR tube was cooled to -78°C, pumped and sealed. The sealed NMR tube was heated to 50°C and the reaction was followed by ³¹P NMR until a single peak corresponding to a new product was found in the ³¹PNMR spectrum. The solution was poured into flask and taken up with ethyl acetate (prewashed with a saturated solution of sodium bicarbonate) and washed with saturated solution of sodium bicarbonate. The organic layer was dried over MgSO₄, the solvent was removed to furnish a white foam in a quantatitive yield. The crude pruduct was flash chromatographed on a silica gel column (hexane-ethyl acetate-triethylamine = 5:3:2); m.p.68-70°C; [α]_{D}²⁰ = 62.9°(c=0.5, CHCl₃) ¹H NMR (500MHz, CDCl₃) δ 7.98 (bs, 1H, NH), 7.46(d, J=1.00Hz, 1H, H-6), 6.34-6.31.(dd, J=5.86Hz, J=8.30Hz, 1H, H-1'), 5.88(d, J=3.91Hz, 1H, H-1"), 4.56-4.53(m, 1H, H-3'), 4.41(d, J=3.91Hz, 1H, H-2"), 4.35(m, 1H, H-3"), 4.17 (d, J=1.95Hz, 1H, H-4"), 4.05 (d, J=1.95Hz, 1H, H-4'), 3.89-3.76 (ABX, 2H, 2xH-5'), 3.45-3.39(m, 2H, H-5", NCH), 3.03-2.99 (m, 1H, H-5"), 2.37-2.34 (m, 1H, H-2'), 2.09-2.05(m, 1H, H-2'), 1.88(d, J=1.47Hz, 3H, MeC=C), 1.95-1.61(m, 8H, cyclopentylidene protons), 1.11-1.00 (m, 6H, Me₂CH), 0.92(s, 9H, t-BuSi), 0.09(d, J=1.47Hz, 6H, Me₂Si); ¹³C NMR (125MHz, CDCl₃) δ 163.77 (C-4), 150.35(C-2), 135.17(C-6), 121.42(OCO), 110.96(C-5), 104.59(C-1"), 86.38, 86.36(d, J=2.75Hz, C-4'), 84.76(C-1'), 84.74(C-2"), 73.78, 73.63(d, J=19.23Hz, C-3'), 73.07, 73.05(d, J=1.83, C-4"), 71.83, 71.80(d, J=3.66Hz, C-3"), 63.17(C-5'), 50.09, 49.80(d, J= 36.63Hz, NCH), 40.27, 40.23 (d, J=4.58Hz, C-2'), 36.89, 36.20(CH₂CCH₂), 36.11, 36.08(d, J=3.21H_{z}, C-5"), 25.91(SiCMe3), 23.44, 22.76(CH₂CH₂CCH₂CH₂), 22.02, 21.95(d, J=9.16Hz, CH₃CHN), 21.69, 21.64(d, J=6.41Hz, CH₃CHN), 18.31(SiCMe3), 12.51(CH3C=C), -5.41, -5.48 (d, J=9.16Hz, Me₂Si) ; ³¹P NMR (81MHz, CDCl₃) δ 130.14; MS (CI) : m/e 642([M+H⁺], 78%); EI m/e [M⁺] 641, HRMS(EI) m/e calc'd for C₂₈H₄₅N₃O₉PSi [M⁺-CH₃] 626.26625, found 626.26670, and calc'd C₂₅H₃₉N₃O₉PSi [M⁺-C₄H₉]: 584.21930, found 584.2190. HRMS FAB (glycerol) m/e calcd. for C₂₉H₄₉N₃O₉PSi [MH⁺] 642, 2975; found 642.2973.

### Example 35

### Protected phosphorothioate dinucleotide (34)

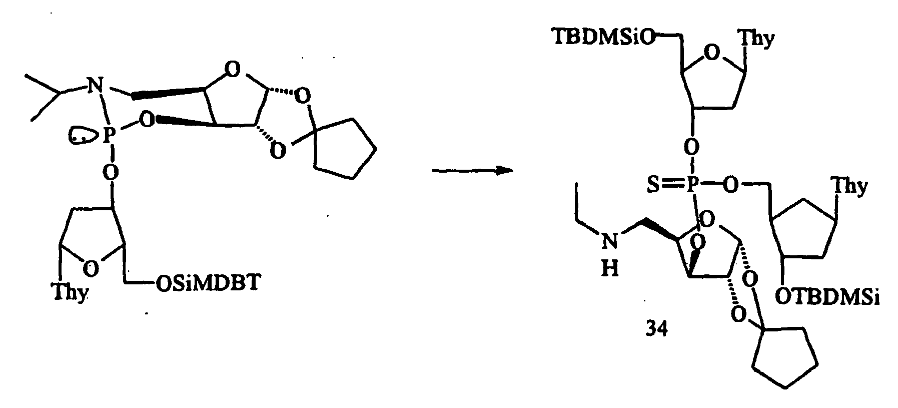

In a dried NMR tube, phosphoramidite 30 (15mg, 0.0234mmol), 3'-O-(tert-butyldimethylsilyl) thymidine (10mg, 1.2eq.) and 4,5-dicyano-2-bromo-imidazole 21 (9.17mg, 2.0eq.) were added. Then the NMR tube was dried in vacuum overnight. Dry acetonitile (0.6ml) was injected into the NMR tube at 0°C under nitrogen atmosphere. The solid dissolved instantly.' The reaction was moved to room temperature and was followed by ^{**31**}_{**P**} NMR. Within 5 minutes, the peak corresponding to the phosphoramidite disappeared and two new peaks (143.76ppm, 142.55ppm=6:1) were formed. The clean ³¹P NMR spectrum suggested use of this product directly in the following sulphurization without isolation. To this solution, Beaucage's, reagent (5.6mg, 1.2eq.) in 140µl acetonitrile (0.2M) was added. Instantaneously the ³¹P NMR showed another two peaks. (68.43ppm, 68.23ppm= 1 : 6), while the peaks corresponding to the starting material disappeared. The solution in NMR tube was transfered to a flask. Then the mixture was redisolved in ethyl acetate, washed with saturated sodium bicarbonate and water, and dried over MgSO₄. Removal of the solvent gave 22mg of white solid 34 (91%). The product was then purified by chromatography on a silica gel column (ethyl acetate : methanol=95:5). ¹H NMR (500MHz, DMSO-d6) δ 7.45(s, 1H, ³H-6), 7.42(s, 1H, ⁵H-6), 6.19-6.15(m, 2H, ⁵H-1', ³H-1'), 5.86, 5.85(d, J=3.91Hz, 1H, H-1"), 5.05-5.04(m, 1H, ⁵H-3'), 4.78-4.77(m, 1H, H-3"), 4.60, 4.59(d, J=3.42Hz, 1H, H-2"), 4.38-4.36((m, 1H, H-4"), 4.26-4.13 (m, 4H, ³H-3', 2x³H-5', ⁵H-4'), 3.93, 3.91(m, 1H, ³H-4'), 3.81-3.73(m, 2H, 2x⁵H-5'), 2.66-2.52(m, 3H, NCH, 2×H-5"), 2.43-2.40(m, 1H, ⁵H-2'), 2.31-2.22(m, 2H, 2x³H-2'), 2.09-2.07(m, 1H, ⁵H-2'), 1.78(s,3H, ³CH₃C=C), 1. 76 (s, 3H, ⁵CH₃C=C), 1.62-1.56(m, 8H, cyclopentylidene protons), 0.94-0.89(m, 6H, Me₂CHN), 0.86(s, 9H, ³t-BuSi), 0.85(s, 9H, ⁵t-BuSi), 0.069(s, 6H, Me₂Si), 0.065(s, 6H, Me₂Si); ¹³CNMR (125MHz, CDCl₃) δ 163.97, 163.66(⁵C-4, ³C-4), 150.39, 150.04 (⁵C-2, ³C-2), 135.58, 134.49-(⁵C-6, ³C-6), 121.77(OCO), 111.26, 111-04(⁵C-5, ³C-5), 104.00(C-1"), 85.59, 85.56(d, J=6.41Hz, ⁵C-4'), 85.48(³C-1'), 84.69 84.61, 84.39 (⁵C-1', ³C-4'), 83.47(C-2"), 80.85, 80.64(d, C-3"), 80.69, 80.66(d, ⁵C-3'), 79.03, 78.97(d, ³C-3'), 71 .49(C-4"), 67.66, 67.52(d, J=4.58Hz, ³C-5'), 63.47(⁵C-5'), 48.93(NCH), 45.23(C-5"), 40.37(³C-2'), 39.14, 39.09 (d, J=6.41Hz, ⁵C-2'), 37.06, 36.16(CH₂CCH₂), 25.88, 25.60(⁵SiCMe₃, ³SiCMe₃), 23.52, 22.85 (CH₂CH₂CCH₂CH₂), 22.65 22.39(NCHMe₂), 18.28, 17.81(⁵SiCMe₃, 3SiCMe₃), 12.51, 12.46 (⁵C=CCH₃, ³C=CCH₃), -4.66, -4.83, -5.40, -5.46(⁵SiMe2, ³SiMe₂) ; ³¹P NMR (202MHz, CDCl₃) δ 68.43, 68.23 (1 :6) MS (FAB) : m/e 1030(M+ H⁺).
(II). 4,5-dicyano-2-bromo-imidazole 21 (7.6mg, 2.5eq.), phosphoramidite 30 (10mg, 0.0156mmol) and 3'-0-(tert-butyldimethylsilyl) thymidine (6.7mg, 1.2eq.) were added to a dried NMR tube. The the NMR tube was dried under vacuum overnight, and dry CDCl₃ (0.5ml) was injected into the NMR tube at 0°C under an argon atmosphere. The reaction was followed by ³¹P NMR until the reaction went to completion. The ³¹P NMR spectrum showed that the peak corresponding to the phosphoramidite at 130ppm had disappeared, and that two new peaks appeared at 142.634, 141.880 ppm in a ratio of 40:1. MS(FAB) [M+ H+]: calc' d for C₄₅H₇₆N₅PO₁₄Si₂ phosphite triester 998, found 998.4.

Beaucage's reagent (3.8mg, 1.2eq.) was added directly to the solution. The ³¹PNMR(202MHz, CDCl₃, 0°C) instantaeously showed another two peaks at 67.831 and 67.514ppm in the same ratio, while the peaks corresponding to the phosphite triester disappeared. The solution in the NMR tube was transfered to a flask, the solvent was evaporated, and the product was then purified by chromatography on a silica gel column (ethyl acetate : hexane: triethylamine = 60:35:5) to give only one isomer. ³¹PNMR (202MHz, CDCl₃, room temperature) δ 68.291.
(III) Virtually identical results were obtained when the reaction was carried out at -15°C for 7 hours in CDCl₃, except that the ratio of isomers was ~68:1. MS FAB (nitrobenzyl alcohol): m/e [MH⁺] 1030 HRMS FAB CsI m/e calcd. for C₄₅H₇₇N₅O₁₄PSSi₂ [MH⁺], 1030.4464; found 1030.4460.

### Example 36

### Phosphorothioate dinucleotide (35)

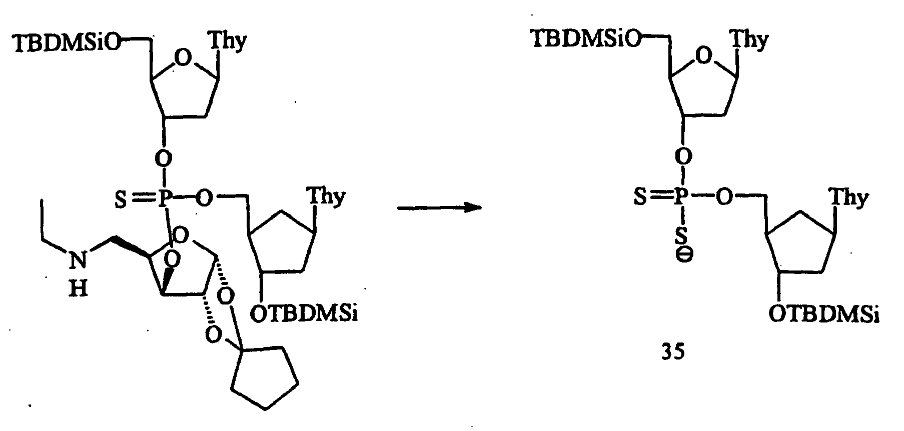

Protected phosphorothioate dinucleotide 34 (14mg, 0.0136mmol) from Example 35 was dissolved in 1ml 70% TFA-H₂O at 0°C with stirring, and then the reaction was allowed to proceed at room temperature. The reaction was followed by TLC until the spot corresponding to the starting material disappeared. Evaporation of the solvent and coevaporation with methanol three times furnished a white solid. The crude product was purified with preparative TLC plate (0.5mm) (CH₂Cl₂ : MeOH=5:1) to give 7.2mg of a white solid (35) (94%); ¹H NMR (500MHz, CD₃OD) δ 7.87 (s, 1H, ³H-6), 7.85(s, 1H, ⁵H-6), 6.36-6.33(dd, J=6.35Hz, J=7.81Hz, 1H, ⁵H-1'), 6.30-6.27(dd, J=6.35Hz, J=7.33Hz ³H-1'), 5.06-5.03(m, 1H, ⁵H-3') 4.53-5.52(m, 1H, ³H-3'),4.21-4.06(m, 4H, ⁵H-4', 2x³H-5', ³H-4'), 3.84-3.80(m, 2H, 2x⁵H-5'), 2.50-2.46(m, 1H, ⁵H-2'), 2.31-2.24(m, 2H, 2x³H-2'), 2.21-2.17(m, 1H, ⁵H-2'), 1.96(s, 3H, ³CH₃C=C), 1.87 (s, 3H, ⁵CH₃C=C); ³¹P NMR (202MHz, CD₃OD) δ 58.64 : 58.57 (6:1) MS (FAB) : m/e 563 (M+ H⁺).

### Example 37

### 1,2-O-3,5-O-dicyclopentylidene-L-xylofuranose (36)

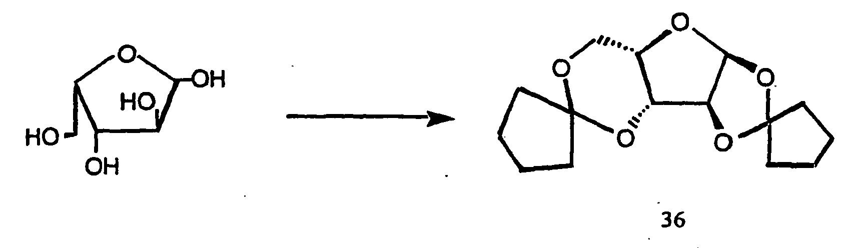

To a solution of trimethyl orthoformate (68mmol, 7.5ml) and p-toluene sulfonic acid (2mmol, 380mg) in dioxane (45ml) under a nitrogen atmosphere at 0°C, was added dropwise cyclopentanone (500mmol, 45ml). This solution was stirred at room temperature for 2 hours and L-xylose (20mmol, 3.0g) was added with stirring continued overnight. Then the reaction mixture was neutralized with triethylamine. Evaporation of the solvent furnished a yellow syrupy residue. A solution of the syrupy residue in chloroform (50ml) was washed with water (50ml). The aqueous layer was extracted with chloroform (3x20ml), and the combined chloroform layers were dried over (MgSO₄). After removing the solvent, the mixture was chromatographed on a silica gel column (hexane:ethyl acetate=5:1) to give 3.5g white solid 36 (62%). ¹HNMR(500 MHz, CDCl₃) δ 5.96 (d, J=3.91 Hz, 1H, H-1), 4.42 (d, J=3.91Hz, 1H, H-2), 4.21 (d, J=1.95Hz, 1H, H-3), 4.09 - 3.98 (m, 3H, H-4, 2xH-5), 1.97 - 1.57 (m, 16H, cyclopentylidene protons); ¹³C NMR (125 MHz, CDCl₃) δ 121.20 (C-1OCOC-2), 109.20 (C-3OCOC-5), 105.05 (C-1), 84.46 (C-2), 74.22 (C-4), 71.80 (C-3), 61.47 (C-5), 39.49, 36.80, 36.16, 29.71, 24.07, 23.47, 22.77, 22.34 (cyclopentylidene carbons); MS (CI) m/e: 283(M+H⁺).

### Example 38

### 1,2-O-cyclopentylidene-L-xylofuranose (37)

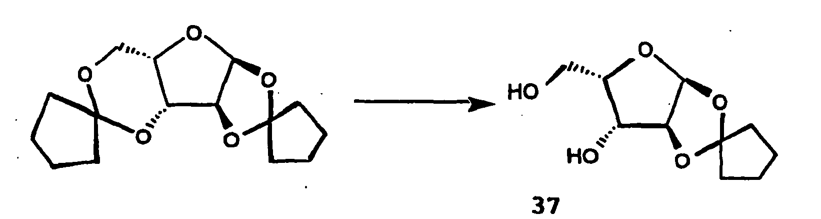

1,2-O-3,5-O-dicyclopentylidene-L-xylofuranose (36) (10mmol, 2.82g) was dissolved in acetic acid -water (2:1) (60ml) at room temperature. The reaction mixture was stirred for 7 hours. The reaction was followed by TLC. The solvent was evaporated with high vacum, coevaporated with methanol three times, and dried in vacuo overnight to give 2.16g white solid 37 (100%). ¹H NMR (500MHz, CDCl₃) S 5.92 (d, J=3.91Hz, 1H, H-1), 4.42(d, J=3.91Hz, 1H, H-2), 4.30 (d, J=2.93Hz, 1H, H-3), 4.17-4.14 (m, 1H, H-4), 4.07- 3.97 (m, 2H, 2xH-5), 1.96 - 1.60 (m, 8H, cyclopentylidene protons); ¹³C NMR (125MHz, CDCl₃) δ 121.42 (OCO), 104.52 (C-1), 85.56 (C-2), 78.81 (C-3), 76.77 (C-4), 61.01 (C-5), 36.84, 36.19 (CH₂CCH₂), 23.47, 22.79 (CH₂CH₂CCH₂CH₂); MS (CI) : m/e 217(M+H⁺).

### Example 39.

### 1,2-O-cyclopentylidene=5'-O-tosyl-L-xylofuranose (38)

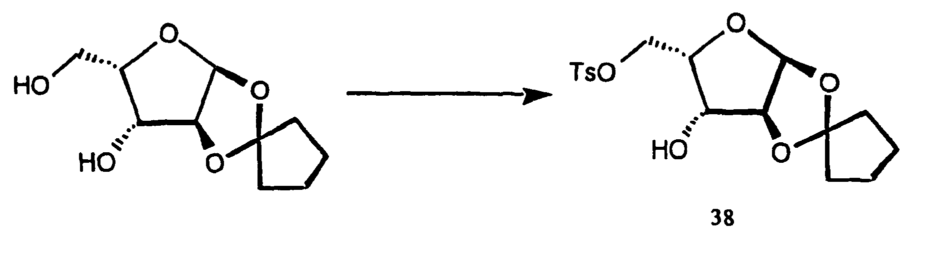

To a solution of 1,2-O-cyclopentylidene-L-xylofuranose (37) (9.4mmol, 2.03g) in dry pyridine (25ml) under nitrogen atmosphere at 0°C, p-toluenesulfonyl chloride (1.2eq., 2.15g) was added. The reaction mixture was stirred at 0°C for 12 hours. Then 10ml water was added to quench the reaction, and the solvent evaporated in high vacum, and coevaporated with toluene twice. The mixture was dissolved in chloroform, washed with water three times and dried over MgSO₄. Removal of the solvent furnished 2.96g of 38 as a white solid (85%). ¹H NMR (500MHz, CDCl₃) δ 7.78 - 7.32 (AA'BB', 4H, Ph), 5.83 (d, J=3.42Hz, 1H, H-1), 4.42 (d, J=3.91Hz, 1H, H-2), 4.42 - 4.24 (m, 3H, H-3, 2xH-5), 4.16-4.08 (m, 1H, H-4), 2.42 (s, 3H, CH₃), 1.90-1.63 (m, 8H, cyclopentylidene protons); ¹³C NMR (270MHz, CDCl₃) δ 145.25 , 130.26, 130.09, 130.01, 129.96 (aromatic), 121.71 (OCO), 104.69 (C-1), 85.04 (C-2), 77.67 (C-4), 74.25 (C-3), 66.39 (C-5), 36.89, 36.30 (CH₂CCH₂), 23.42, 22.85 (CH₂CH₂CCH₂CH₂), 21.63 (CH₃); MS (CI) : m/e 371 (M+H⁺).

### Example 40

### 1,2-O-dicyclopentylidene-5'-isopropylamine-L-xylofuranose (39)

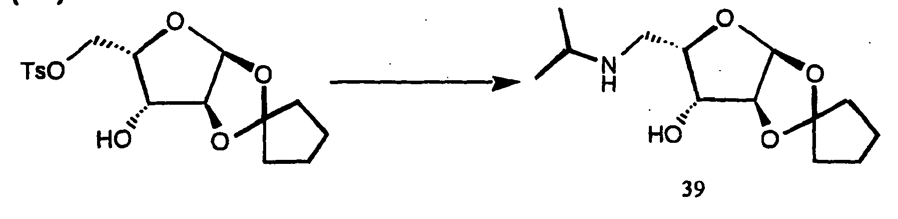

A solution of 1,2-O-cyclopentylidene-5'-tosyl-L-xylofuranose (38) (7.0mmol, 2.6g) in isopropylamine (15ml) was stirred at 55°C overnight in a pressure bottle. The solvent was removed by rotary evaporator and the remaining yellow syrup was taken up with chloroform and washed with a saturated solution of sodium bicarbonate and with brine. The organic phase was dried over MgSO₄, the solvent was evaporated and the residue was flash chromatographed on silica gel (ethyl acetate-3% triethylamine) to furnish 1.30g white solid **39** (72%). m.p. 39-41°C; [α]_{D}²⁰ = - 31.37(c=2,CHCl₃); ¹H NMR (500MHz, CDCl₃) δ 8.0(bs, NH), 5.88 (d, J=3.91Hz, 1H, H-1), 4.37 (d, J=3.91Hz, 1H, H-2), 4.25 (d, J=2.93Hz, 1H, H-3), 4.19 (m, 1H, H-4), 3.34 - 2.91 (ABX, 2H, 2×H-5), 2.73-2.71 (heptet, 1H, NCH), 1.92-1.61 (m, 8H, cyclopentylidene protons), 1.03-1.01(dd, J=2.44Hz, J=6.35Hz, 6H, Me₂CH); ¹³C NMR (50OMz, CDCl₃) δ 121.00 (OCO), 104.75 (C-1), 86.06 (C-2), 78.20 (C-3), 77.00(C-4), 48.62(NCH), 45.82(C-5), 36.78, 36.25(CH₂CCH₂), 23.46, 22-78(CH₂CH₂CCH₂CH₂), 22.57(CH₃CHN), 22.27(CH₃CHN); MS (CI) m/e 258([M+H⁺]; 100%); HRMS(EI) m/e calc'd for C₁₃H₂₃NO₄ [M⁺] : 257.16270, found 257.16250.

### Example 41

### Chlorophosphoramidite (40)

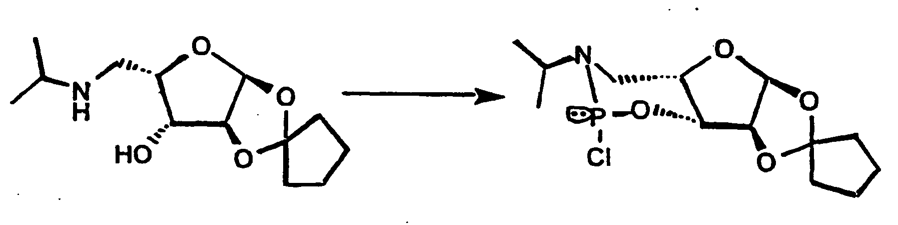

In a scrupulously dry NMR tube, 9.6µl (0.11mmol) phorsphorus trichloride was added via syringe, then 0.25ml CDCl₃ was simlarly added. This NMR tube was cooled to 0°C, and a solution of the 1,2-0-dicyclopentylidene-5'-isopropylamine-L-xylofuranose (17) (25.7mg, 0.1mmol) and triethylamine (27.8µl, 0.22mmol) in CDCl₃ (0-35ml) was added under a nitrogen atmosphere with shaking of the NMR tube. An exothermic reaction was noticed. The NMR tube was then cooled to -78°C, pumped and sealed. The sealed NMR tube was heated to 40°C and the reaction was followed by ³¹PNMR until a single peak was found in the ³¹PNMR spectrum. The product was not isolated, and was directly used in the following step. ³¹P NMR (300MHz, CDCl3) δ 148.75

### Example 42

### 5'-O-(tert-butyl dimethyl silyl)-thymidine-3'-O-Phosphoramidite (41)

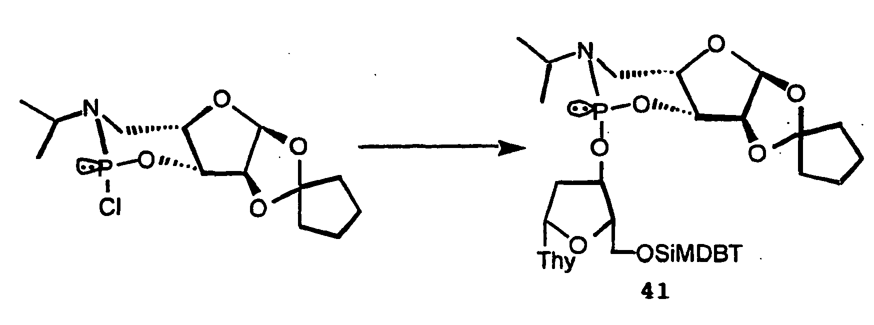

To the same NMR tube from previous Example 41, a solution of 5'-O-(tert-butyl dimethyl silyl)-thymidine (35.6mg, 0.1mmol) in 0.45ml CDCl₃ and triethylamine (14µl, 0.11mmol) was added slowly at 0°C under a nitrogen atmosphere. Then the NMR tube was cooled to -78°C, pumped and sealed. The sealed NMR tube was heated to 50°C and the reaction was followed by ³¹PNMR until a single peak corresponding to a new product was found in the ³¹PNMR spectrum. The solution was poured into a flask, and taken up with ethyl acetate (prewashed with a saturated solution of sodium bicarbonate) and washed with saturated solution of sodium bicarbonate. The organic layer was dried over MgSO₄, and the solvent was removed to furnish a white foam in a quantatitive yield. The crude pruduct was flash chromatographyed on a silica gel column (hexane-ethyl acetate-triethylamine = 5:3:2) to furnish white crystals (41); m.p. 99-101 °C; [α]_{D}²⁰ -72.0° (c=0.5,CHCl₃) ¹H NMR (500MHz, CDCl₃) δ 8.77 (bs, 1H, NH), 7.46(s, 1H, H-6), 6.33-6.30(dd, J=5.86Hz, J=7.81Hz, 1H, H-1'), 5.88(d, J=3.91Hz, 1H, H-1"), 4.56-4.53(m, 1H, H-3'), 4.43(d, J=3.42Hz, 1H, H-2"), 4.35(m, 1H, H-3"), 4.18 (d, J=1.95Hz, 1H, H-4"), 4.05 (m, 1H, H-4'), 3.90-3.76 (ABX, 2H, 2xH-5'), 3.45-3.42(m, 2H, H-5", NCH), 3.03-2.99 (m, 1H, H-5"), 2.38-2.35 (m, 1H, H-2'), 2.12-2.06(m, 1H, H-2'), 1.89(s, 3H, MeC=C), 1.96-1.62(m, 8H, cyclopentylidene protons), 1.11-1.08 (m, 6H, Me₂CH), 0.90(s, 9H, t-BuSi), 0.09, (d, J=1.95Hz, 6H, Me₂Si); ¹³C NMR (125MHz, CDCl₃) δ 163.74 (C-4), 150.31(C-2), 135.18(C-6), 121.44(OCO), 110.93(C-5), 104.59(C-1"), 86.61, 86.57(d, J=5.49Hz, C-4'), 84.75, 84.73(d, J=2.75Hz, C-1'), 84.70(C-2"), 73.07, 73.05(d, J=1.83Hz, C-3'), 73.03, 72.89(d, J=17.4, C-4"), 71.82, 71.78(d, J=4.58Hz, C-3"), 62.98(C-5'), 50.05, 49.76(d, J= 36.63Hz, NCH), 39.94, 39.92(d, J=2.75Hz, C-2'), 36.86, 36.19(CH₂CCH₂), 36.11, 36.08(d, J=3.06H_{z}, C-5"), 25.92(SiCMe3), 23.45, 22.76(CH₂CH₂CCH₂CH₂), 21.99, 21.91(d, J=9.16Hz, CH₃CHN), 21.69, 21.64(d, J=6.41Hz, CH₃CHN), 18.35(SiCMe3), 12.52(CH3C=C), -5.39, -5.45 (d, J=9.16Hz, Me₂Si); ³¹P NMR (81MHz, CDCl₃) δ 129.34; MS (CI) : m/e 642(M+H⁺). EI: m/e 641[M+]. MS FAB (nitrobenzyl alchol): m/e [MH⁺] 642, HRMS FAB (glcerol) m/e calcd. for C₂₉H₄₉N₃O₉ PSi [MH⁺] 642.2975; found 642.2973.

### Example 43

### Protected phosphorothioate dinucleotide (42)

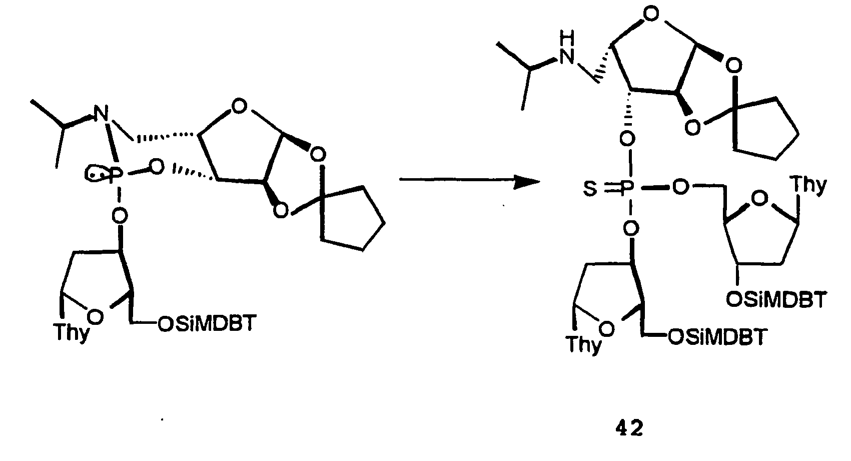

I: In a dried NMR tube, phosophoramidite 41 (15mg, 0.0234mmol), 3'-O-(tert-butyldimethylsilyl) thymidine (10mg, 1.2eq.) and 4,5-dicyano-2-bromo-imidazole 21 (9.17mg, 2.0eq.) were added. Then the NMR tube was dried in vacuum overnight. Dry acetonitile (0.6ml) was injected into the NMR tube at room temperature under a nitrogen atmosphere. The solid dissolved instantly. The reaction was followed by ³¹PNMR. Within 5 minutes, the peak corresponding to the phosphoramidite disappeared. To this solution, Beaucage's reagent (5.6mg, 1.2eq.) in 140µl acetonitrile (0.2M) was added. The peaks corresponding to the starting material disappeared. The solution in NMR tube was transfered to a flask, and the solvent evaporated. Then the mixture was redisolved in ethyl acetate, washed with saturated sodium bicarbonate and water, and dried over MgSO₄. After removal of the solvent, the product was purified by chromatography on a silica gel column (ethyl acetate : methanol=95:5) to furnish 42 in a diastereomeric ratio of 7:1(69.12, 68.91ppm). ¹H NMR (500MHz, CDCl₃) δ 7.46(s, 1H, ³H-6), 7.27(s, 1H, ⁵H-6), 6.34-6.10(m, 2H, ⁵H-1', ³H-1'), 5.86, 5.87(d, J=3.42Hz, 1H, H-1"), 5.17-5.14(m, 1H, ⁵H-3'), 4.83-4.81(d, J=10.25Hz,1H, H-3"), 4.56, 4.55(d, J=3.91Hz, 1H, H-2"), 4.38(m, 2H, ³H-3', H-4"), 4.25-4.21(m, 3H, 2x³H-5', ⁵H-4'), 3..98(m, 1H, ³H-4'), 3.91-3.85(m, 2H, 2x⁵H-5'), 2.85, 2.84(d, J=6.35Hz, 1H, 2xH-5"), 2.81(m, 1H, NCH), 2.52-2.47(dd, 2H, ⁵H-2'), 2.25-2.23(m, 2H, 2x³H-2'), 2.08-2.02(m, 1H, ⁵H-2'), 1.91 (s, 3H, ⁵CH₃C=C), 1.89 (s, 3H, ³CH₃C=C), 1.92-1.65 (m, 8H, cyclopentylidene protons), 1.05, 1.04(d, J=5.86Hz, 6H, Me₂CHN), 0.90(s, 9H, ³t-BuSi), 0.86(s, 9H, ⁵t-BuSi), 0.11(s, 6H, Me₂Si), 0.05(s, 6H, Me₂Si); ¹³CNMR (125MHz, CDCl₃) δ 163.80, 163.59 (⁵C-4, ³C-4), 150.33, 150.15(⁵C-2, ³C-2), 136.13, 134.64(⁵C-6, ³C-6), 122.03(OCO), 111.43, 111.11(⁵C-5, ³C-5), 104.28(C-1"), 86.29(³C-1'), 85.95, 85.90(d, J=6.41Hz, ⁵C-4'), 84.80 84.72, 84.69 (⁵C-1', ³C-4'), 83.59(C-2''), 80.85, 80.81(d, J=4.58Hz, C-3 "), 80.69, 80.66(d, J=4.58Hz, ⁵C-3'), 79.03, 78.97(d, J=8.24Hz, ³C-3'), 71.30(C-4"), 67.38, 67.34(d, J=5.50Hz, ³C-5'), 63.37(⁵C-5'), 48.91(NCH), 45.18(C-5"), 40.35(³C-2'), 39.12, 39.09(d, J=3.66Hz, ⁵C-2'), 37.14, 36.23(CH₂CCH₂), 25.88, 25.65(⁵SiCMe₃, ³SiCMe₃), 23.55, 22.89(CH₂CH₂CCH₂CH₂), 22.80, 22.55(NCHMe₂), 18.28, 17.86(⁵SiCMe₃, 3SiCMe₃), 12.52, 12.49 (⁵C=CCH₃, ³C=CCH₃), -4.66, -4.83, -5.40, -5.46 (⁵SiMe2, ³SiMe₂) ; ³¹P NMR (121MHz, CDCl₃) δ 69.12, 68.91 (7:1); MS (FAB) : m/e (M+ H⁺).

### Example 44

### Phosphorothioate dinucleotide (43)

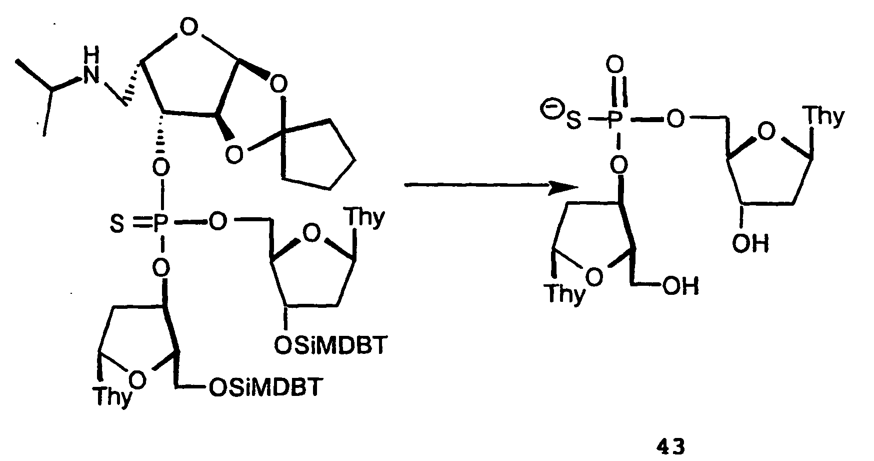

The protected phosphorothioate dinucleotide 42 obtained from example 43-I (15mg, 0.014mmol) was dissolved in 1ml 70% TFA-H₂O at 0°C with stirring and, the reaction was allowed to proceed at room temperature. The reaction was followed by TLC until the spot corresponding to the starting material disappeared. Evaporation of the solvent and coevaporation with methanol three times furnished a white solid. The crude product was purified with preparative TLC plate (0.5mm) (CH₂Cl₂ : MeOH=5:1) to give 43 as a white solid: ¹H NMR (500MHz, CD₃OD) 6 7.91 (s, 1H, ³H-6), 7.86(s, 1H, ⁵H-6), 6.36-6.33(dd, J=6.35Hz, J=7.81Hz, 1H, ⁵H-1'), 6.29-6.26(dd, J=5.86Hz, J=7.81Hz ³H-1'), 5.08-5.05(m, 1H, ⁵H-3') 4.52-5.51(m, 1H, ³H-3'),4.21(m, 1H, ⁵H-4'), 4.14-4.11(m, 2H, 2x³H-5'), 4.04 (m, 1H, ³H-4'), 3.86-3.79(m, 2H, 2x⁵H-5'), 2.48-2.44(m, 1H, ³H-2'), 2.31-2.24(m, 2H, 2x⁵H-2'), 2.23-2.16(m,1H, ³H-2'),1.97(s, 3H, ³CH₃C=C), 1.87(s, 3H, ⁵CH₃C=C); ³¹P NMR (121MHz, CDCl₃) δ 59.14 : 59.08(1:7); MS (FAB) : m/e (M+ H⁺)

### EXAMPLE 45

### Synthesis of γ-aminoalcohol 44 (Scheme 1)

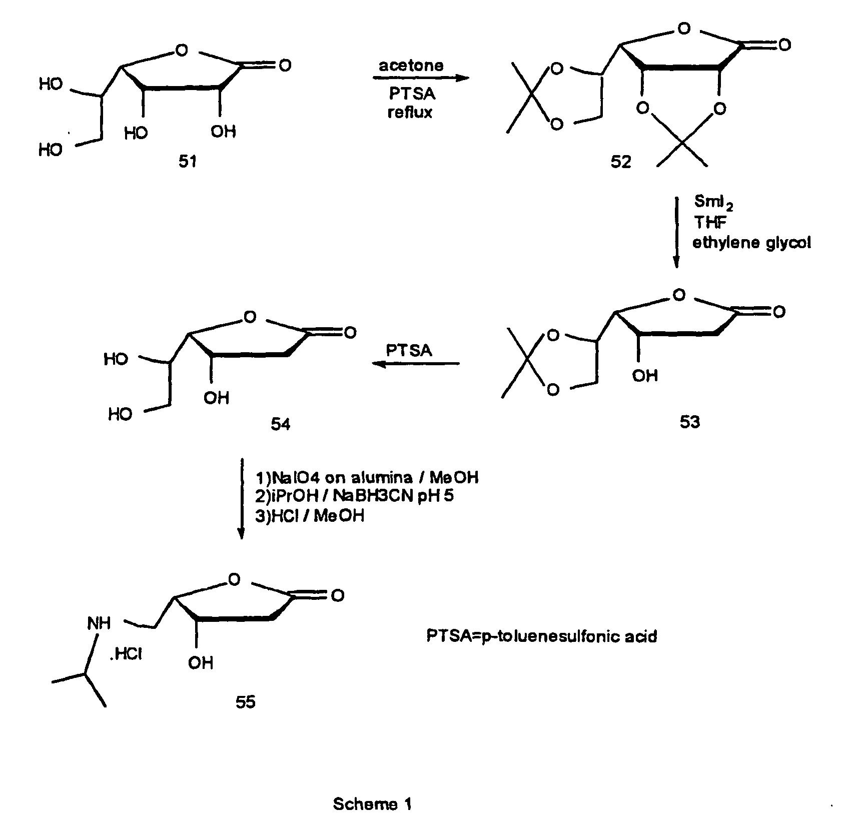

The synthesis of amino-alcohol 44 is shown in Scheme 1 below:

Starting from L-mannonic-γ-lactone 51, both diols are protected by conversion to their acetonides according to standard procedures, using acetone with para-toluenesulfonic acid as a catalyst, to yield bis-acetonide lactone 52.

The bis-acetonide is then reacted in presence of samarium iodide in a mixture of THF and ethylene glycol at room temperature according to the procedure described by Christian Girard, Ph.D. Thesis, Universite de Montreal, 1995. This reaction proceeds with high yield and high regioselectivity and affects the acetonide located on the α-position to the ester, yielding β-hydroxyester 53.

The next step is a classical acid-catalyzed deprotection of the acetonide to give triol product 54. The diol function of the triol is then cleaved by sodium periodate on alumina in methanol. The intermediate aldehyde undergoes a reductive amination in presence of isopropylamine and sodium cyanoborohydride to yield γ-aminoalcohol 55, which is isolated and stabilized as the hydrochloride salt. *See* Robert Hambalek, Ph.D. Thesis, McGill University, 1992.

γ-aminoalcohol **55** is then employed as a chiral precursor to the stereocontrolled synthesis of a P-chiral phosphorothioate dimer that can be deprotected by a base-catalyzed β-elimination. An example of this type of elimination is found in Takahata et al., *J. Org. Chem.* 1995, 60, 5628-5633.

### Preparation of Indole Containing Chiral Auxiliaries

### Example 46

### Preparation of (S)-1-(indol-2-yl)-propan-2-ol

### (S)-1-(indol-2-yl)-propan-2-ol was prepared according to the following Scheme:

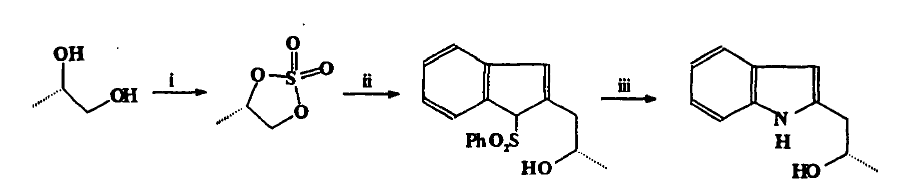

i) a. SOCl₂, CCl₄, 60°C. b. NaIO₄, RuCl₃·3H₂O, CH₃CN/H₂O, 25°C, 98%.
ii) 1-Phenylsulfonyl-indole,n-BuLi, -78°C-25°C, overnight, then add 20% H₂SO₄ and stir for 3 hours, 87%.
iii) KOH, CH₃OH/H₂O (3:1), reflux, 100%.

### Example 46A

### Preparation of 1-Phenylsulfonyl-indole (60)

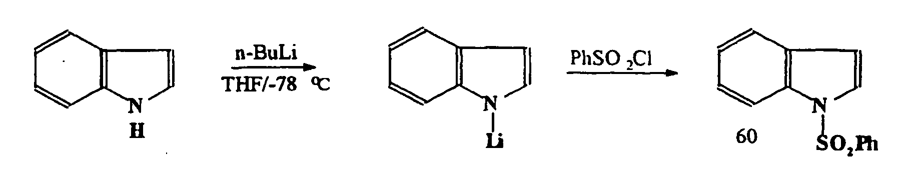

To a solution of indole (2.4 g, 20 mol) in dry THF (20 ml) under argon at -78°C was added dropwise via syringe over 10 min n-butyllithium (1.6M in hexanes; 14 ml). The cooling bath was removed and the solution was stirred for 1h while warming to 0°C. The resulting indole anion precipitated as a very fine white solid in a cloudy colorless solution. After the suspension was recooled to -78°C, benzenesulfonyl chloride (2.8 ml, 22mmol) was added via syringe over 20 min, keeping the temperature below -60°C. The resulting colorless mixture was allowed to warm slowly to room temperature overnight, poured into 2% aqueous sodium bicarbonate (30 ml), and extracted with ethyl acetate (2x25 ml). The combined extracts were washed with 2% aqueous sodium bicarbonate(30ml), water (2x25 ml), dried over anhydrous sodium sulphate, and evaporated to give a light amber oil which crystallized when triturated with 2:1 hexane/ether (15 ml). After standing in the cold (-20°C) for several hours, the product was collected by filtration, washed with hexane, and dried in vancum to provide pure 1-phenylsulfonyl-indole (60) as white crystals (4.8 g, 90.6%).
¹H NMR (270 MHz, CDCl₃) : δ 7.16-7.98 (m, 9H, C₆H₅, C₆H₄), 7.63(d, ³J=3.7, 1H, NCH), 6.71(d, ³J=3.7, 1H, NCHCH). ¹³C NMR (67.9 MHz, CDCl₃): δ 138.3, 134.9, 133.8, 130.8, 129.3, 126.8, 126.3, 124.7, 123.4, 121.5, 113.6, 109.3. m.p. 73.0-73.5°C.

### Example 46B

### (S)-1,2-propandiol cyclic sulfate (61)

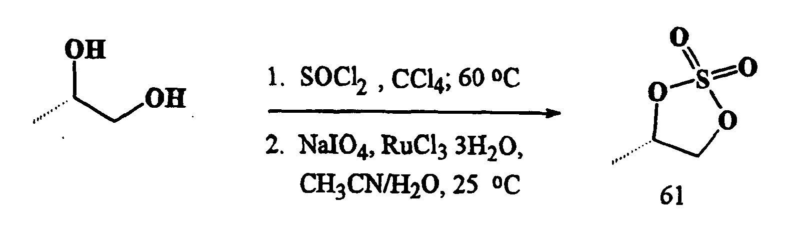

A 100-ml, two-neck round-bottom flask equipped with a reflux condenser and topped with a CaCl₂ drying tube connected to an HCl trap, and a rubber septum was charged with (S)-1,2-propanediol (2.3 g, 40 mmol) and CCl₄ (20 ml). Thionyl chloride (4 ml, 54.8 mmol) was added via a syringe to the flask, and the resulting solution was refluxed for 30 min. The solution was then cooled with an ice-water bath and diluted with CH₃CN (20 ml). RuCl₃·3H₂O (7.8 mg, 0.03 mmol) and NalO₄ (12 g, 56 mmol) were added followed by water (40 ml). The resulting mixture was stirred at room temperature for 60 min. The mixture was then diluted with ethyl acetate (150 ml), and the two phases were separated. The organic layer was washed with water (30 ml), saturated sodium bicarbonate solution (2 × 20 ml), and brine (20 ml). After drying over anhydrous sodium sulfate, the solution was filtered through a small pad of silica gel to remove the brown color. The filtrate was then concentrated to afford (S)-1,2-propanediol cyclic sulfate (61) as a colorless liquid (4.0 g, 98%).
¹H NMR (270 MHz, CDCl₃) : δ 5.10 (ddq, ³J_{CH2-H} = 8.2 Hz, 6.0 Hz, ³J_{CH3-H} = 6.2 Hz, 1H, CH), 4.72 (dd, ²J = 8.7 Hz, ³J = 6.0 Hz, 1H, CHH'), 4.28 (dd, ²J = 8.7 Hz, ³J = 8.2 Hz, 1H, CHH'), 1.55 (d, ³J = 6.2 Hz, 3H, CH3). ¹³C NMR (67.9 MHz, CDCl₃): δ 80.0, 74.3, 17.7.

### Example 46C

### (S)-2-Indolylisopropanol (62)

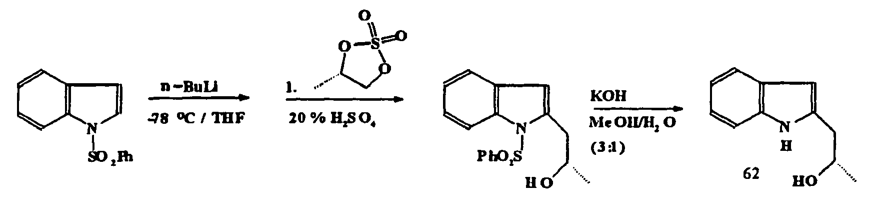

To a solution of 1-phenylsulfonyl-indole (2.57 g, 10 mmol) in dry THF (30 ml) under argon at -78°C was added dropwise via syringe over 10 min a solution of 1.6 M butyllithium (6.25 ml, 10 mmol). The mixture was stirred for 1.5 h below -70°C and then allowed to warm slowly to 5°C over 1h. The solution was cooled to -78°C and then treated via syringe with a solution of (S)-1, 2-propanediol cyclic sulfate (1.5 g, 10.8 mmol) in dry THF (10 ml). The mixture was allowed to warm slowly to room temperature overnight, poured into 20% sulfuric acid (100 ml) and stirred for 3 hrs. The solution was extracted with ethyl acetate (3 × 50 ml). The combined extracts were washed with H₂O (2×100 ml), saturated sodium bicarbonate solution (2×100 ml) and brine (2×100 ml), dried over anhydrous sodium sulphate, and rotary evaporated to afford a light amber oil. This oil was crystallized in ether:hexane (1:1) to provide (S)-1-phenylsulfonyl-2-indolylisopropanol as white crystals (2.85 g, 90%).
¹H NMR (270 MHz, CDCl₃) : δ 7.17-8.16 (m, 9H, C₆H₅, C₆H₄), 6.51 (d, ⁴J = 0.76, 1H, NCCH), 4.26 (m, 1H, CHO), 3.25, 3.01 (m, 2H, CHH'), 1. 91 (s, br, 1H, OH), 1.30 (d, ³J = 6.2Hz, 3H, CH₃). ¹³C NMR (67.9 MHz, CDCl₃): δ 138.8, 138.5, 137.4, 133.8, 129.7, 129.3, 126.3, 124.4, 123.9, 120.5, 115.1, 111.6, 67.2, 39.1, 23.1. m.p.: 88-89°C .

Cleavage of the phenylsulfonyl protecting group was achieved with potassium hydroxide. 2.85 g (S)-1-phenylsulfonyl-2-indolylisopropanol was dissolved in 50ml methanol/water (3:1) containing 1.5 g KOH. The solution was refluxed for 5 hours and extracted with ethyl acetate (2x50 ml). The combined extracts were washed with H₂O (2x100 ml) and brine (2x100 ml), dried over anhydrous sodium sulphate, and evaporated to afford pure (S)-2-indolylisopropanol (1.65 g, 95%) as a light amber oil.
¹H NMR (270 MHz, CDCl₃): δ 8.51 (s, 1H, NH), 7.58-7.05 (m, 4H, C₆H₄), 6.28 (m, 1H, NCCH), 4.10 (m, 1H, CHO), 2.93, 2.76 (m, 2H, CHH'), 2.06 (s, br, 1H, OH), 1.25 (d, ³J = 6.2 Hz, 3H, CH₃) ¹³C NMR (67.9 MHz, CDCl₃) : δ 136.6, 136.2, 128.9, 121.3, 119.9, 119.7, 110.7, 100.9, 68.0, 37.5, 23.3.

### Example 47

### Preparation of Indole Oxazaphosphorine

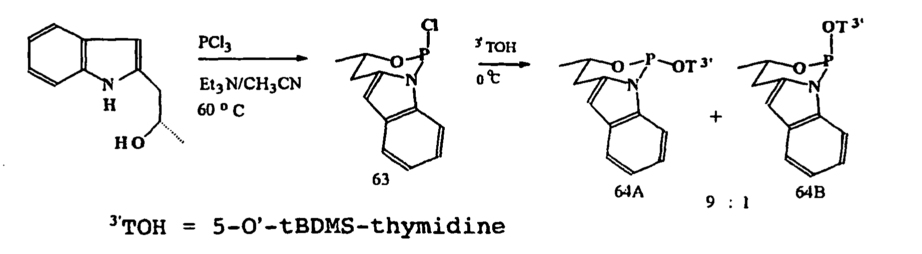

In a scupulously dried 25-ml round-bottom flask was added 10 ml dried CH₃CN, flushed with argon and sealed with a septum. PCl₃ (100 µl, 1.15 mmol) was syringed into the flask. Then the flask was cooled to 0°C, and a solution of (S)-2-indolisopropanol (200 mg, 1.15 mmol) in CH₃CN (0.35 ml) containing triethylamine (525 µl, 3.8 mmol) was syringed into the flask. As soon as (S)-2-indolisopropanol was introduced, a thick white precipitate was observed, corresponding to the formation of triethylammonium chloride. After stirred for 30 min at 0°C, the reaction mixture was warmed to 60°C. The warming was continued until the ³¹P NMR showed a major peak at δ 144 ppm (about one day). The flask was cooled to 0°C again, and a solution of 5'-O-tBDMS-thymidine (410 mg, 1.15 mmol) in CH₂Cl₂ (0.4 ml) was added. The reaction mixture was stirred at 0°C for 30 min. Triethylammonium chloride was filtered out and washed with CH₂Cl₂ (2×10 ml). The filtration was concentrated and purified by silica gel chromatography (CH₂Cl₂ / CH₃CN 1: 10) to afford indol oxazaphosphorine (346 mg, 54%) as a white solid. Two diastereoisomers of indol oxazaphosphorine were obtained (64A and 64B),in the ratio of 9 : 1 (read from ³¹p NMR). The following NMR spectra were assigned for the major component:
³¹P NMR (202.3 MHz, CDCl₃): δ 121.56 (12.4%), 120.67 (87.6%). ¹H NMR (500 MHz, CDCl₃, assigned by COSY): δ 8.81 (br s, 1H, NH), 7.39 (s, 1H, H-6), 7.54, 7.17 (m, 4H, C₆H₄), 6.36 (dd, 1H, ³J = 9.0 Hz, ³J = 5.5 Hz, H-1'), 6.33 (s, 1H, C=CH-Ph), 4.72 (m, 1H, H-3'), 4.41 (m, 1H, CHOP), 3.94 (m, 1H, H-4'), 3.58 (m, 1H, H-5'), 3.06 - 3.10 (m, 3H, H-5", CH₂), 2.36 (m, 1H, H-2'), 1.97 (m, 1H, H-2"), 1.87 (s, 3H, CH₃C-5), 1.48 (d, 3H, ³J = 5.5 Hz, CH₃), 0.84 (s, 9H, SiC(CH₃)₃), -0.05 (d, 6H, Si(CH₃)₂). ¹³C NMR (67.9 MHz, CDCl₃): δ 163.7 (C-4), 150.3 (C-2), 137.6, 129.8, 122.2, 121.5, 120.4, 111.1 (C₆H₄), 136.4 (CCHPh), 135.2 (C-6), 110.6 (C-5), 103.2 (CCHPh), 86.2 (C-4'), 86.1 (CHOP), 84.8 (C-1'), 73.7 (C-3'), 71.5 (C-5'), 62.9 (C-2'), 26.0 (CH2), 25.9 (SiC(CH₃)₃), 23.0 (SiC(CH₃)₃), 18.3 (CH₃), 12.6 (CH₃C-5), -5.54, -5.77 (CH₃SiCH₃). HRMS (FAB, M+H): Cal. 560.234578, found 560.234590). m. p. 80-82 °C.

### Preparation of dinucleotide phosphorothioate triester

Dinucleotide phosphorothioate triester compounds were prepared according to the following scheme:

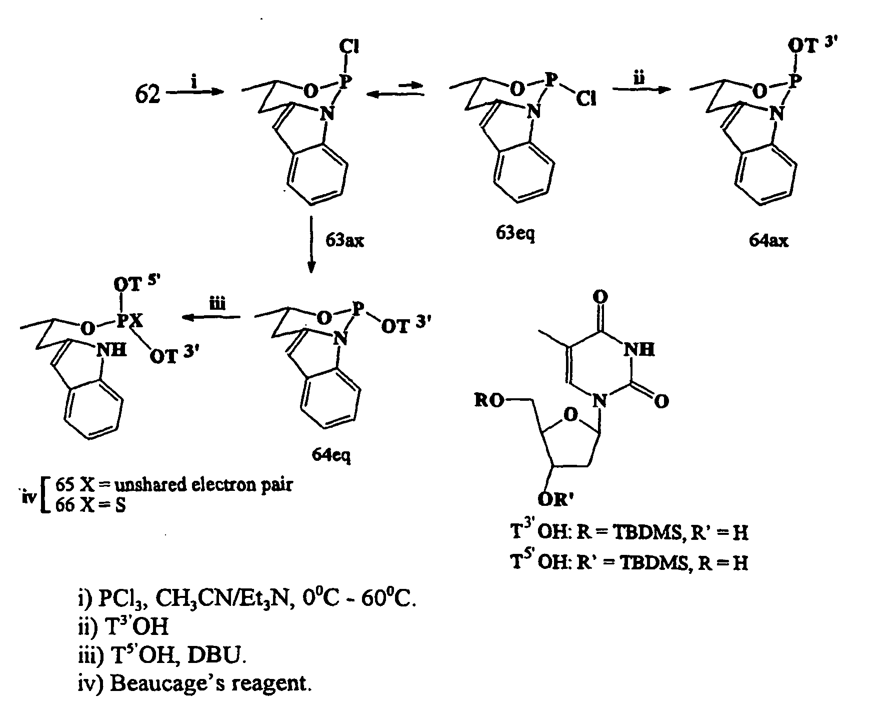

Equimolar acetonitrile solutions of **62** and PCl₃ were allowed to react at 0°C under argon and the reaction was followed by ³¹P NMR. After a few minutes, the total disappearance of the peak corresponding to PCl₃ at 221 ppm was observed, and several peaks appeared around 140 - 150 ppm. The mixture was warmed up to 60°C. The warming was continued (about 10 hours) until the ³¹P NMR showed a major peak at 144 ppm, which indicated the formation of phosphorochloridite **63,** which is believed to exist as a rapidly equilibrating mixture of 63ax and **63eq,** in which **63ax** predominates. The mixture was cooled to 0°C and a solution of 5'-O-tBDMS-thymidine in CH₂Cl₂ was added. Two peaks were observed within 0.5 h, a major one at 120.47 ppm and a minor one at 120.36 ppm, corresponding to the formation of the two diastereoisomers **64eq** and **64ax.** The ratio of two diastereoisomers of 3 was affected by the temperature at which 5'-O-tBDMS-thymidine was added. At 20-60°C, the ratio was 7:1; at lower temperature (0-78°C), the ratio increased to 9:1.

The coupling of **64ax** and **64eq** with 3'-O-tBDPS-thymidine was done in the presence of 1,8-diazabicyclo[5,4,0]undec-7-ene (DBU). The major diastereoisomer **64eq** reacted much faster with 3'-O-tBDPS-thymidine than the minor axially substituted isomer **64ax.** By using 1 eq. of DBU and 1 eq. of 3'-O-tBDPS-thymidine, 95% of **64eq** was converted to phosphite triester **65** after five hours at 50°C, while **64ax** almost did not react, as determined by ³¹P NMR. After filtration through a short silica gel column to remove DBU, triester **65** was treated with Beaucage's reagent to give a 73:1 mixture of phosphorothioates **66,** in which the major isomer is beleived to have the Rp configuration, ³¹P NMR 66.76 ppm (major) and 66.59 ppm. The chiral auxiliary 1 could not be removed with 28% ammonium hydroxide, but may be removed with other reagents.

The thermodynamically more stable axially substituted cyclic indole derivatives reacted much more slowly than their equatorially substituted isomers. While not wishing to be bound by a particualr theory, the displacement leading from **63** to **64,** and **64** to **65** proceed with inversion. The rapidly equilibrating mixture of slow reacting **63ax** and faster reacting **63eq** (ratio ~ 99:1) provides a 7-9:1 mixture of non-equilibrating fast reacting **64eq** and slow reacting **64ax.** The fast reacting indole derivative **64eq** and its slow reacting isomer **64ax** are then transformed with inversion to provide **65** as a mixture of diastereomers, in which one isomer, which is believed to be the isomer havin the Rp configuration, is formed as the major product. Sulfurization provides a mixture of diastereomers **66** in a ratio of ~ 70:1.

### Example 48

### Preparation of dinucleotide phosphorothioate triester (66)

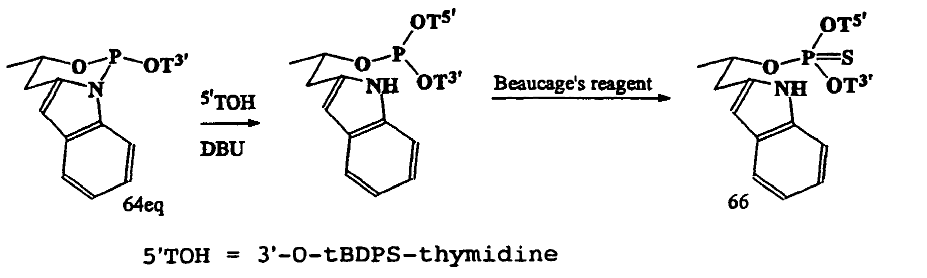

In a dried 5-ml round-bottom flask was added 2 ml dried CHCl₃, indol oxazaphosphorine **64eq** (50 mg, 0.085 mmol) and 3'-O-tBDPS-thymidine (40.8 mg, 0.085 mmol), flushed with argon, and sealed with a septum. DBU (14 µl, 0.094 mmol) was syringed into the flask. The reaction solution was stirred at room temperature overnight. Then the solution passed though a short silica gel column to filter out DBU, and eluted with dried CH₂Cl₂/CH₃CN (1:1). The solvent was evaporated to afford a colorless oil. The oil was redissolved in dried CH₂Cl₂ (5 ml), and Beaucage's reagent (30 mg, 0.15 mmol) was added. Evaporation of the reaction solution followed by flash chromatography (CH₂Cl₂/CH₃COCH₃ 5:1) afforded dinucleotide phosphorothioate triester **66** (71mg, 78%) as white solid.
³¹P NMR (202.3 MHz, CDCl₃): δ 66.76 (98.65%), 66.59 (1.35%). ¹H NMR (500 MHz, CDCl₃, assigned by COSY): δ 9.93 (s, 1H, NH), 9.31 (s, 1H, NH-3-T^{5'}), 8.85 (s, 1H, NH-3-T^{3'}), 7.62 - 6.93 (m, 16H, Si(C₆H₅)₂, C₆H₄, H-6-T^{3'}, H-6-T^{5'}), 6.46 (dd, 1H, ³J=8.0 Hz, ³J=6.0 Hz, H-1'-T^{5'}), 6.26 (s, 1H, CH-Ph), 6.05 (dd, 1H, ³J=9.2 Hz, ³J=5.5 Hz, H-1'-T^{3'}), 4.92 (m, 1H, CHOP), 4.76 (m, 1H, H-3'-T^{3'}), 4.31 (m, 1H, H-3'-T^{5'}), 4.03 (m, 1H, H-4'-T^{5'}), 3.82 (m, 1H, H-4'-T^{3'}), 3.80, 3.50 (m, 2H, H-5', H-5"-T^{5'}), 3.67, 3.58 (m, 2H, H-5', H-5"-T^{3'}), 3.00 (m, 2H, CH₂), 2.31 (m, 1H, H-2'-T^{5'}), 1.94 (s, 3H, CH₃C-5-T^{5'}), 1.90 (s, 3H, CH₃C-5-T^{3'}), 1.85 (m, 1H, H-2"-T^{5'}), 1.60 (m, 1H, H-2'-T^{3'}), 1.26 (d, 3H, ³J=6.0 Hz, CH₃), 1.14 (m, 1H, H-2"-T^{3'}), 1.80 (s, 9H, SiC(CH₃)₃-T^{5'}), 0.89 (s, 9H, SiC(CH₃)₃-T^{3'}), 0.07 (d, 6H, Si(CH₃)₂). ¹³C NMR (125.7 MHz, CDCl₃, assigned by HMQC): S 163.84, 163.80 (C-4-T^{3'}. C-4-T^{5'}), 150.74, 150.34 (C-2-T^{3'}, C-2-T^{5'}), 135.52, 135.49, 135.25, 134.56, 134.16, 132.73, 132.55, -130.15, 130.05, 128.48, 127.91, 127.85, 120.99, 119.59, 119.34, 111.24, 110.41 (C-₆H₅SiC₆H₅, C₆H₅NC, C-6-T^{3'}, C-6-T^{5'}), 100.69 .(PhCH), 85.29, 85.17 (C-4'-T^{3'}, C-4'-T^{5'}), 84.87 (C-1'-T^{5'}), 84.27 (C-1'-T^{3'}), 79.70 (C-3'-T^{3'}), 76.82 (CH), 73.31 (C-3'-T^{5'}), 66.83 (C-5'-T^{5'}), 63.01 (C-5'-T^{3'}), 40.17 (C-2'-T^{5'}), 37.59 (C-2'-T³'), 36.00 (CH₂), 26.67 (C(CH₃)₃-T^{5'}), 25.77 (C(CH₃)₃-T^{3'}), 21.24 (CH₃), 18.81, 18.15 (SiC-T^{3'}, SiC-T^{5'}), -5.61, -5.56 (CH₃SiCH₃). MS (FAB, M+H): 1072. m.p. 115-116 °C.

### Example 49

### (R)-Glycidyl-tert-butyldimethylsilyl ether

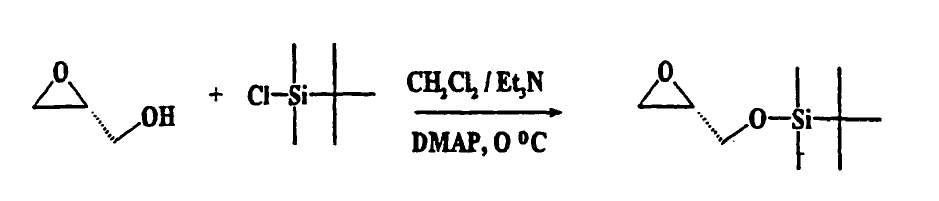

To a solution of (R)-glycidol (5 g, 67.5 mmol) in dry dichloromethane containing triethylamine (10.3 ml, 74 mmol) cooled down to 0°C, was added a solution of tert-butyldimethylsilyl chloride (11.2 g, 74 mmol) in dry dichloromethane (30 ml). Then DMAP (0.33 g, 2.7 mmol) was added. The mixture was allowed to warm up to room temperature and stirred for 5 hours. The triethylammonium chloride crystals were filtered out and washed with dichloromethane (2×10 ml). The organic solution was washed with brine (2×50 ml) and dried over anhydrous sodium sulfate. The solution was concentrated and passed though a short silica gel column to remove polar impurities, and eluted with hexane/ethyl acetate (3:2). After removing the solvent, a colourless oil was collected and dried in *vacuo* to provide pure (S)-glycidyl-tert-butyldimethylsilyl ether (10.3 g, 81.2%).
¹H NMR (270 Mhz, CDCl3): 3.81, 3.61 (m, 2H, CH2OSi), 3.04 (m, 1H, CH), 2.72, 2.59 (m, 2H, CH2O), 0.86 (s, 9H, C(CH3)3), 0.036 (d, 6H, Si(CH3)2). ¹³C NMR (67.9 Mhz, CDCl3): 63.78 (CH2OSi), 52.44 (CH2O), 44.45 (CHO), 25.90 ((CH3)3), 18.38 (Csi), -5.28, -5.32 (CH3SiCH3).

### Preparation of chiral auxiliaries 67 to 69 are shown in the following Scheme:

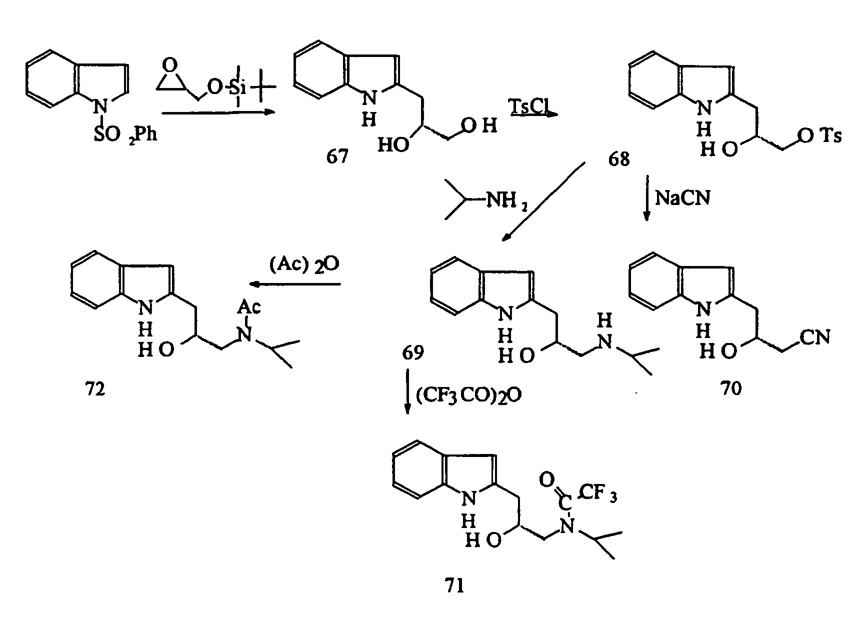

The chiral auxiliaries 70-72 are removable with standard reagents useful for the removal of phosphorus protecting groups.

### Example 50

### (R)-3-indol-2-yl-propane-1,2-diol (67)

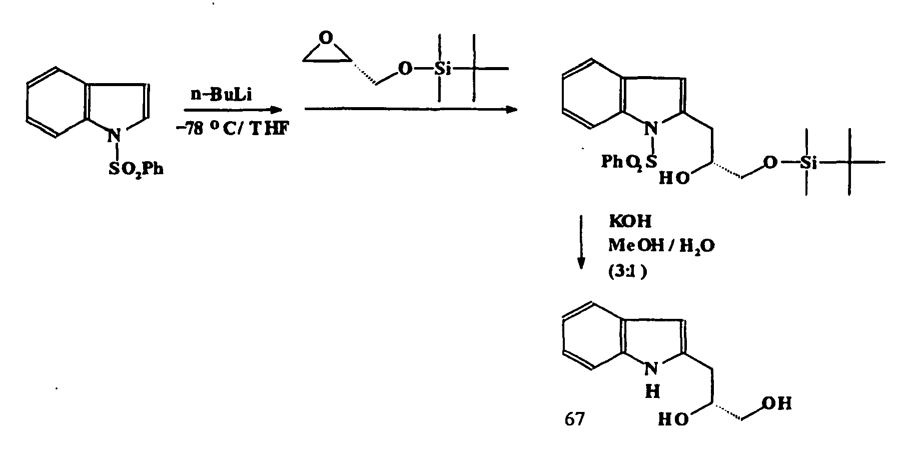

To a solution of 1-phenylsulfonyl-indole (6.2 g, 24 mmol) in dry THF (60 ml) under argon at -78°C was added dropwise via syringe over 10 min a solution of 1.6 M butyllithium (15 ml, 24 mmol). The mixture was stirred for 1.5 h below -70°C and then allowed to warm slowly to 5°C over 1h. The solution was cooled to -78°C and then treated via syringe with a solution of (S)-glycidyl-tert-butyldimethylsilyl ether (4.5 g, 24 mmol) in dry THF (10 ml). The mixture was allowed to warm slowly to room temperature overnight, poured into saturated NH₄Cl solution (80 ml). The solution was extracted with ethyl acetate (3 × 40 ml). The combined extracts were washed with H₂O (2×100 ml), saturated sodium bicarbonate solution (2×100 ml) and brine (2×100 ml), dried over anhydrous sodium sulphate, and rotary evaporated to afford a deep red oil. This oil was purified by silica gel chromatography (ethyl acetate/hexane 1:1) to provide (R)-1-tert-butyldimethylsiloxyl-3-(1-phenylsulfonylindol-2-yl)-propan-2-ol as a light red oil (5.2 g, 46%).
¹H NMR (500 MHz, CDCl₃) : δ 7.19-8.16 (m, 9H, C₆H₅, C₆H₄), 6.57 (s, 1H, NCCH), 4.14 (m, 1H, CHO), 3.74, 3.58 (m, 2H, CH2OSi), 3.23, 3.09 (m, 2H, CH2C), 2.57 (d, 1H, ³J = 4.5 Hz, OH), 0.93 (s, 9H, (CH3)3), 0.10 (d, 6H, CH3SiCH3). ¹³C NMR (75.4 MHz, CDCl₃): δ 138.95, 138.32, 137.30, 133.66, 129.83, 129.22, 126.20, 124.19, 123.73, 120.37, 114.93, 111.25, 70.88, 66.52, 33.03, 25.90, 18.30.

Cleavage of the phenylsulfonyl protecting group was achieved with potassium hydroxide. 4.5 g (R)-1-tert-butyldimethylsiloxyl-3-(1-phenylsulfonylindol-2-yl)- propan-2-ol was dissolved in 50ml methanol/water (3:1) containing 2.8 g KOH. The solution was refluxed for 5 hours and extracted with ethyl acetate (2×50 ml). The combined extracts were washed with H₂O (2×100 ml) and brine (2x100 ml), dried over anhydrous sodium sulphate, and evaporated to afford pure (R)-3-indol-2-yl-propane-1,2-diol (1.68 g, 86.9%) as a light amber oil.
¹H NMR (270 MHz, CDCl₃): δ 8.58 (s, 1H, NH), 7.53-7.00 (m, 4H, C₆H₄), 6.21 (s, 1H, NCCH), 3.88 (m, 1H, CHO), 3.56, 3.40 (m, 2H, CH2O), 3.2 (s, br, 1H, OH), 2.79 (m, 2H, CH2C), 2.00 (s, broad, 1H, OH). ¹³C NMR (67.9 MHz, CDCl₃): δ 136.24, 135.71, 128.46, 121.45, 119.96, 119.78, 110.75 (C6H4NC), 100.93 (CHCN), 71.80 (CHO), 66.06 (CH2C), 31.87 (CH2OH).

### Example 51

### 1-p-toluenesulfonic-3-indol-2-yl-propan-2-ol (68)

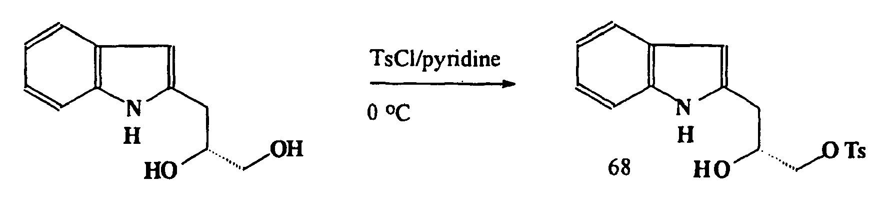

To a solution of (R)-3-indol-2-yl-propane-1,2-diol (1.20 g, 6.28 mmol) in dry pyridine (60 ml) cooled down to 0°C, was added p-toluenesulfonyl chloride (1.20 g, 6.29 mmol). After stiiring for 5 hours at 0°C, the solution was poured into 100 ml cold water and extracted with ether (3×30 ml). The combined extracts were washed with 6 N hydrchloric acid (2×50 ml), brine (2×50 ml), dried over anhydrous sodium sulphate, and evaporated to give 1-p-tolunesulfonic-3-indol-2-yl-propan-2-ol as white solid (1.70 g, 78.6%), which was not purified for the next reactions.
¹H NMR (270 Mhz, CDCl3): 8.63 (s, broad, 1H, NH), 7.0-7.7 (m, 8H, C6H4, C6H4SO2), 4.13 (m, 1H, CHO), 3.99 (m, 2H, CH2O), 2.89 (m, 2H, CH2C), 2.41 (s, 3H, CH3).

### Example 52

### Hydroxycyanopropylindole 70

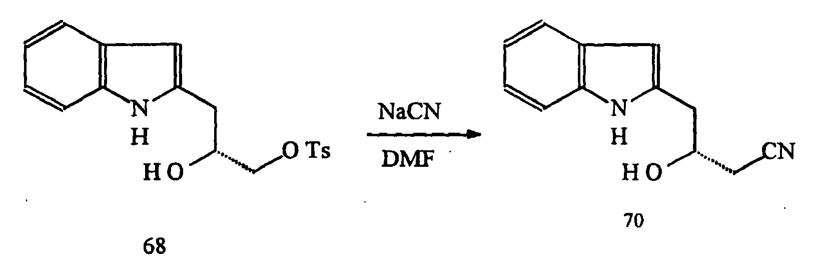

A solution of tosylate 68 (1.62 g, 4.7 mmol) in DMF (30 ml) containing sodium cyanide (0.5 g, 10.2 mmol) was stirred for four hours at 110°C, then cooled down to room temperature, poured into 80 ml ice-water, and extracted with ethyl acetate (330 ml), The combined organic solution was washed with saturated sodium bicarbonate (230 ml), brine (230 ml), dried over anhydrous sodium sulfate, and evaporated to yield a deep red oil. This oil was purified by flash chromatography (hexane:ethyl acetate 2:3) to give nitrile 10 (0.6 g. 64%).
¹H NMR (270 MHz, CDC13): 8.42 (s, broad, 1H, NH), 7.0-7.5 (m, 4H, C6H4), 6.30(d, 1H, ⁴J = 1.48 Hz, CHCN), 4.20 (m, 1H, CHO), 3.01 (m, 2H, CH2), 2.47, 2.49 (m, 2H, CH2CN). ¹³C NMR (67.9 Mhz, CDCl₃): 136.56, 133.58, 1218.55, 121.94, 120.16, 120.06, 110.74, 102.12 (C8H5N), 117.12 (CN), 67.55 (CH2C), 35.10 (CHO), 25.28 (CH2CN).

### Example 53

### Amino compound 69

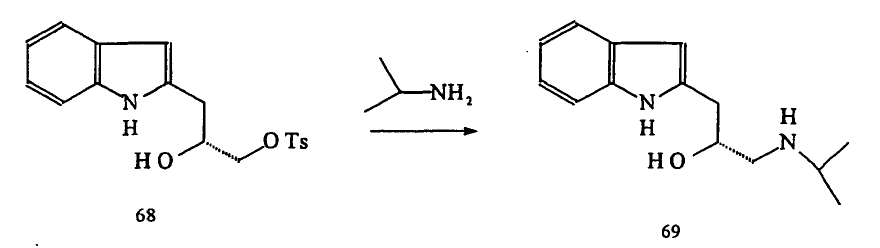

To a pressure vessel was added 2.94 g of 68 and 10 ml isopropylamine. The mixture was stirred overnight at 110°C. Evaporation of the solvent afforded a amber oil which was purified by flash chromatography to provide 69 (1.6 g, 81%).
¹H NMR (270 Mhz, CDCl3): 9.01 (s, broad, 1H, NH), 7.0-7.5 (m, 4H, C6H4), 6.23(s, 1H, CHCN), 3.89 (m, 1H, CHO), 2.72-3.03 (m, 5H, NCH, OH, NH, CH2), 2.44, 2.59 (m, 2H, CH2N), 1.05, 1.04 (d, 6H, ³J = 6.18 Hz, (CH3)2). ¹³C NMR (67.9 MHz, CDCl₃): 136.61, 136.28, 128.43, 121.12, 119.81, 119.46, 110.73, 100.68 (C8H5N), 69.37, 51.80, 49.04, 33.34, 23.12, 22.81.

### Example 54

### Compound 72

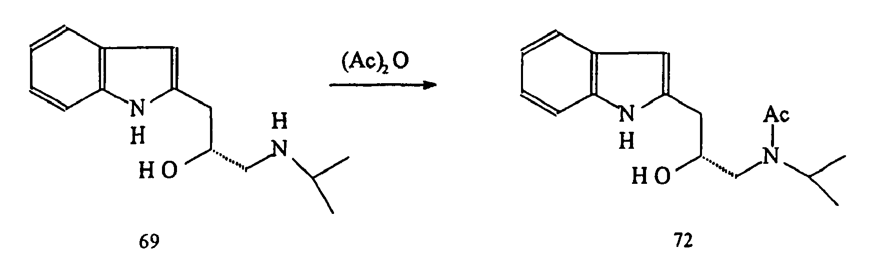

To a solution of **69** (0.2 g, 0.86 mmol) in dry CH₃CN (20 ml) was added acetic anhydride (0.1 ml, 1.06 mmol). The mixture was stirred for four hours at room temperature, then washed with saturated sodium bicarbonate solution (210 ml), brine (210 ml), and dried ove anhydrous sodium sulfate. The solvent was evaporated ant residual solid was purified on flash chromatography (ethyl acetate) to compound **72** as a colourless crystals (0.22 g, 92%).
¹H NMR (270 Mhz, CDCl3): 9.17 (s, broad, 1H, NH), 7.0-7.5 (m, 4H, C6H4), 6.25(s, 1H, CHCN), 3.98 (m, 2H, CHO, CHN), 3.50, 3.10 (m, 2H, CH2), 2.94 (m, 2H, CH2N), 2.15 (s, 3H, CH3CO),1.14, 1.12 (d, 6H, ³J = 6.42 Hz, (CH3)2). ¹³C NMR (67.9 MHz, CDCl₃): 173.58 (CO), 136.34, 136.16, 128.24, 121.17, 119.70, 119.41, 110.89, 100.85 (C₈H₅N), 73.41, 50.15, 47.92, 34.12, 21.32, 20.73.

Those skilled in the art will appreciate that numerous changes and modifications may be made to the preferred embodiments of the invention and that such changes and modifications may be made without departing from the scope of the invention. It is therefore intended that the appended claims cover all equivalent variations as fall within the scope of the invention.

## Claims

1. A method for synthesizing an oligomer having a phosphorothioate linkage comprising the steps of:
- reacting a first synthon of Formula I: 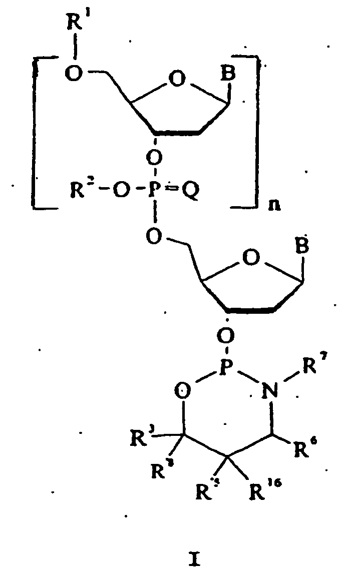 wherein:
Q is independently O or S;
R¹ is a hydroxyl protecting group;
R² is a chiral auxiliary of formula
-C(R⁸)R³-C (R¹⁶)R⁵-CHR⁶-NHR⁷;
R³ is hydrogen, alkyl having 1 to 6 carbon atoms, cyanomethyl, monohalomethyl, dihalomethyl, trihalomethyl, -CH₂R₄, -CH₂Si(R⁴)₃, or -CH₂-SOₖR⁴ where k is 0, 1 or 2;
R⁴ is independently alkyl, aryl, aralkyl or alkaryl having up to 15 carbon atoms, -N(R₇₀)-C(=O)-R₇₁,
-S-C (=O) -R₇₀, or -O-C (=O)-O-N (R₇₀)(R₇₁);
R₇₀ and R₇₁ are each independently alkyl having 1 to 6 carbon atoms, ∝-halo substituted alkyl having 1 to 6 carbon atoms, aralkyl having 4 to 20 carbon atoms, ∝-halo substituted aralkyl having 4 to 20 carbon toms, or aryl, having 3 to 14 carbon atoms, substituted with up to three electron withdrawing groups;
R⁵ is H, -CN, -Si(R⁴)₃, SOₖR⁴ or halogen;
or R⁸ and R¹⁶ are each H, and R³and R⁵, together, form one of the structures 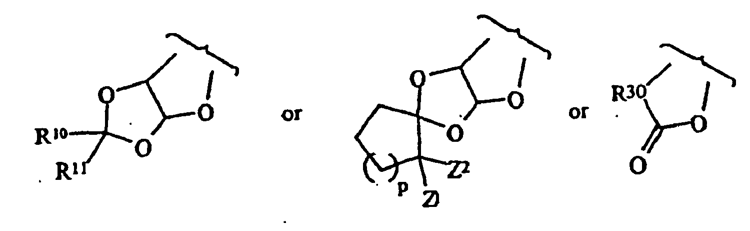 wherein:
R¹⁰ and R¹¹ are H, alkyl having from 1 to 10 carbons, -CH₂C(=O)OR²², -CH₂CN, -CH₂Si(CH₃)₃, or *o*- or *p*-C₆H₄-R²¹;
R²¹ is hydrogen, -O-C(=O)CH₃, alkoxy having from 1 to 10 carbons, -NO₂, or -N(R²²)₂;
R²² is independently H or alkyl having from one to 10 carbon atoms;
p is 1 or 2;
Z¹ and Z² are independently halogen, CN, -Si(CH₃)₃, and -C(=O)OR²²;
R³⁰ is hydrogen, -O-C(=O)CH₃, alkoxy having from 1 to 10 carbons, or -O-Si(R₄)₃;
R⁶ is H, alkyl or aralkyl having up to 15 carbon atoms;
or R⁵ and R⁶, together with the atoms to which they are attached, form a 5 or 6 membered ring;
R⁷ is alkyl or aralkyl having up to 15 carbon atoms;
or R⁶ and R⁷, together, form one of the structures 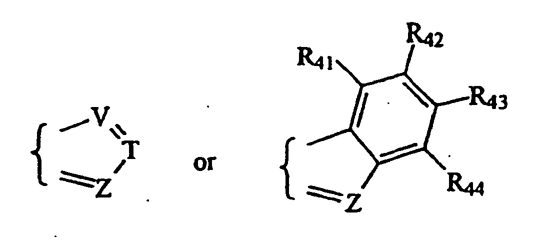
wherein V, T, and Z are independently CH or N;
R₄₁, R₄₂, R₄₃, and R₄₄ are each independently H or an electron withdrawing group;
R⁸ is H or methyl;
R¹⁶ is H, alkyl or aralkyl having up to 15 carbon atoms;
B is a nucleobase; and
n is an integer from 0 to 50;
with a second synthon of Formula II: 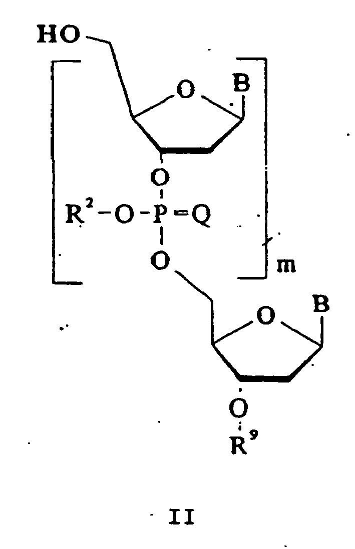 wherein:
R⁹ is a hydroxyl protecting group or a linker connected to a solid support; and
m is an integer from 0 to 50;
for a time and under reaction conditions effective to form a third synthon of Formula III: 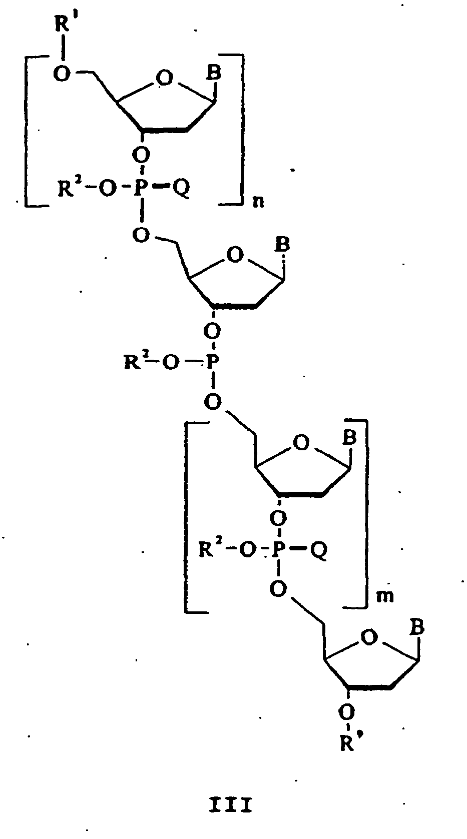 and
- contacting said third synthon with a sulfurizing agent to form an oligomer of Formula IV: 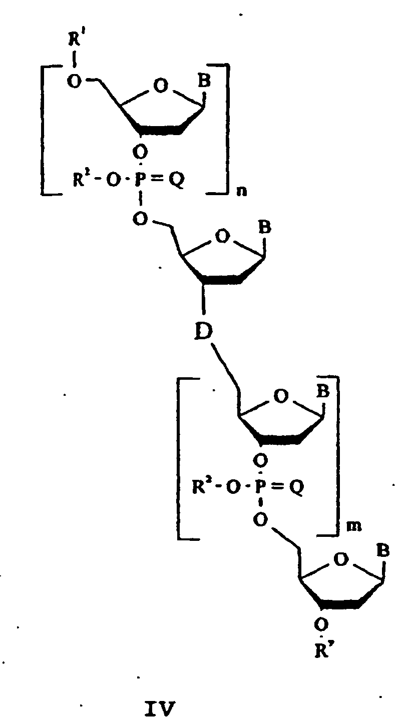
wherein D is said phosphorothioate linkage having the formula:

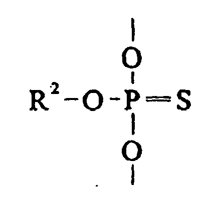

2. The method of claim 1 wherein said phosphorothioate linkage is diastereomerically enriched.

3. The method of claim 1 wherein 75% of said phosphorothioate linkage is in a single stereoisomeric form.

4. The method of claim 1 wherein 85% of said phosphorothioate linkage is in a single stereoisomeric form.

5. The method of claim 1 wherein 95% of said phosphorothioate linkage is in a single stereoisomeric form.

6. The method of claim 1 wherein said phosphorothioate linkage is in a single stereoisomeric form, substantially free of other stereoisomeric forms.

7. The method of claim 1 wherein n is 0.

8. The method of claim 1 wherein said first synhon is in a single stereoisomeric form, substantially free of other stereoisomeric forms.

9. The method of claim 1 further comprising removing said R¹ group from said phosphorothioate.

10. The method of claim 1 further comprising removing said chiral auxiliaries.

11. The method of claim 10 wherein the removing said chiral auxiliaries is via β-elimination

12. The method of claim 10 wherein the removing said chiral auxiliaries is by treatment with acidic reagents.

13. The method of claim 1 wherein said compound of Formula IV contains a plurality of said phosphorothioate linkages.

14. The method of claim 1 wherein said first and second synthons are reacted at a temperature of from -20°C to 40°C.

15. The method of claim 1 wherein said first and second synthons are reacted at a temperature of from -15°C to 0°C.

16. The method of claim 1 wherein said first synthon is formed by reacting a compound of Formula V: 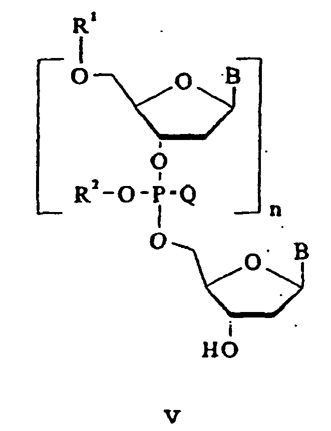 with an azaphospholane of Formula VIa: 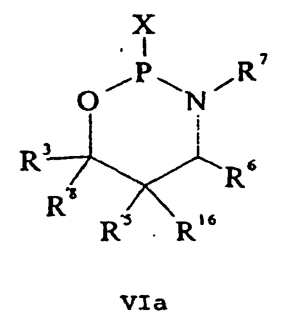 wherein R³-R⁸ are as defined above; and X is halogen, dialkylamino, imidazole, triazole or substituted phenoxy wherein said substituents are electron withdrawing.

17. The method of claim 16 wherein said electron withdrawing substituents are halogen or nitro.

18. The method of claim 16 wherein said azaphospholane is produced by reacting a reagent of formula HO-C(R⁸)R³-C(R¹⁶)R⁵-CHR⁶-NHR⁷ and a phosphorus trihalide, phosphorus tri(dialkylamide), phosphorus triphenoxide, or phosphorus tri(imidazolides).

19. The method of claim 1 wherein said first synthon is formed by reacting a compound of Formula VII: 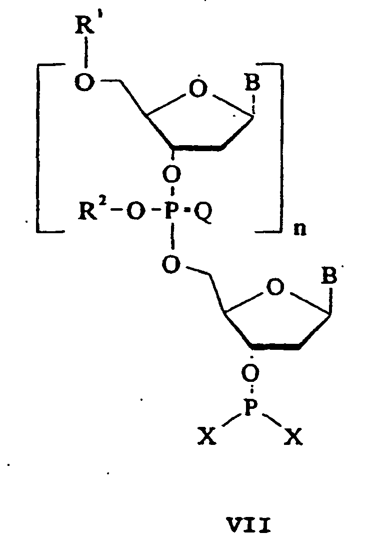 and a γ-amino alcohol of formula HO-C(R⁸)R³-C(R¹⁶)R⁶-CHR⁶⁻NHR⁷; wherein X and R¹-R⁸ and R¹⁶ are as defined above.

20. The method of claim 19 wherein X is chlorine.

21. The method of claim 19 wherein said reaction is stereoselective.

22. The method of claim 20 wherein said first synthon is in a single stereoisomeric form, substantially free of other stereoisomeric forms.

23. The method of claim 1 wherein said reaction is performed in the presence of a catalyst.

24. The method of claim 23 wherein said catalyst has one of the Formulas VIII or IX: 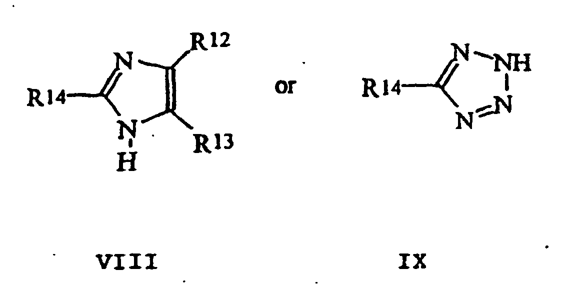 wherein:
R¹² and R¹³ are independently hydrogen., halogen, cyano, nitro, alkyl having from one to 10 carbons, substituted alkyl having from one to 10 carbons, an ester group, or R¹² and R¹³ together with the carbon atoms to which they are attached, form a substituted or unsubstituted phenyl ring where said substituents are electron withdrawing; and
R¹⁴ is hydrogen, halogen, cyano, nitro, thio, alkyl having from one to 10 carbons, substituted alkyl having from one to 10 carbons, norbornyl, substituted norbornyl, aryl having 3 to 14 carbon atoms, substituted aryl having 3 to 14 carbon atoms wherein said substituents are electron withdrawing, or has the formula: 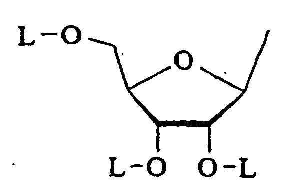
wherein L is protecting group.

25. The method of claim 23 wherein R¹⁴ is halogen or nitro, and wherein R¹² and R¹³ are each halogen or each cyano.

26. The method of claim 24 wherein R¹⁴ is bromine, and R¹² and R¹³ are each cyano.

27. The method of claim 24 wherein R¹⁴, R¹² and R¹³ are each bromo.

28. The method of claim 23 wherein R¹⁴ has one of the formulas: 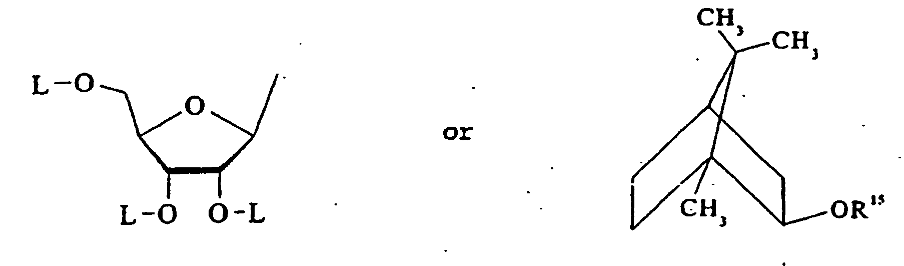 wherein R¹⁵ is H, methyl, trialkylsilyl or acetyl.

29. The method of claim 1 wherein R³ is cyanomethyl or -CH₂-SOₖR⁴ where k is 0, 1 or 2.

30. The method of claim 29 wherein R⁷ is alkyl having 1 to 6 carbon atoms or aralkyl having up to 15 carbon atoms.

31. The method of claim 1 wherein said first synthon has one of the Formulas Xa, XIa, XIIa, XIIIa or XXa: 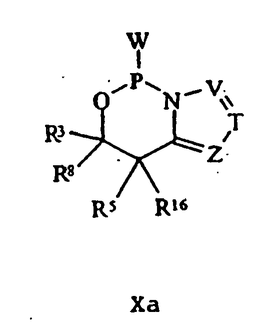 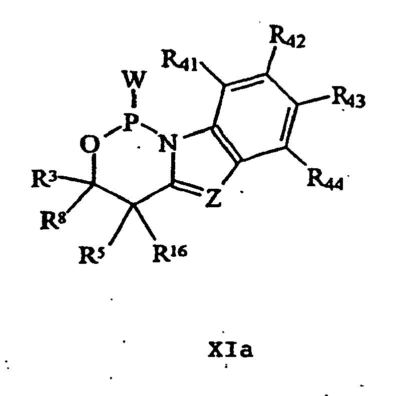 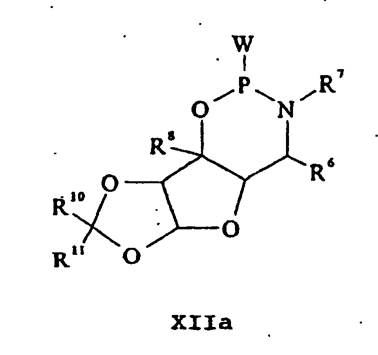 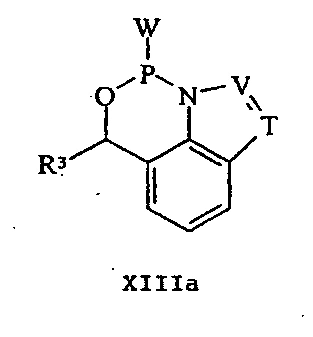 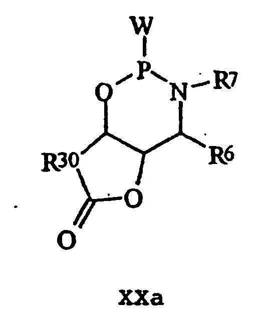 wherein W has the formula: 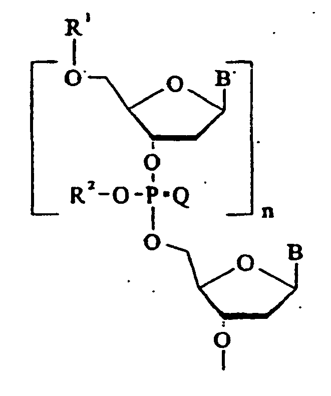 and R¹-R¹⁶, V, T and Z are as defined above.

32. The method of claim 31 wherein said first synthon has the Formula XIa; R³ is -CH₂R₄; and R⁴ is independently -N(R₇₀)-C(=O)-R₇₁, -S-C(=O)-R₇₀, or -O-C(=O)-O-N(R₇₀)(R₇₁).

33. The method of claim 32 wherein R₇₀ and R₇₁ are independently alkyl having 1 to 6 carbon atoms or α-halo substituted alkyl having 1 to 6 carbon atoms.

34. The method of claim 31 wherein said first synthon has the Formula Xb or Xc:

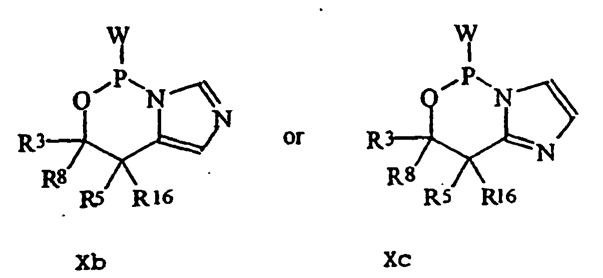

35. The method of claim 31 wherein said first synthon has the Formula XIVa: 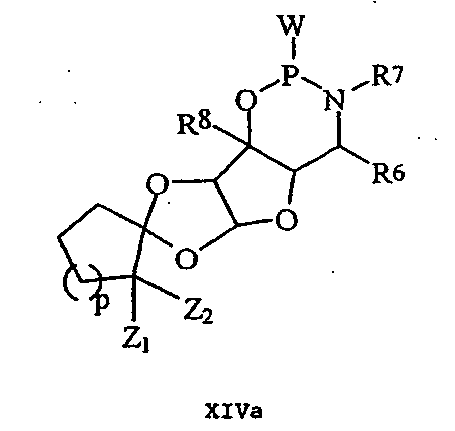 wherein p, Z¹ and Z² are as defined above.

36. The method of claim 35 wherein said first synthon has the Formula XVa or XVIa: 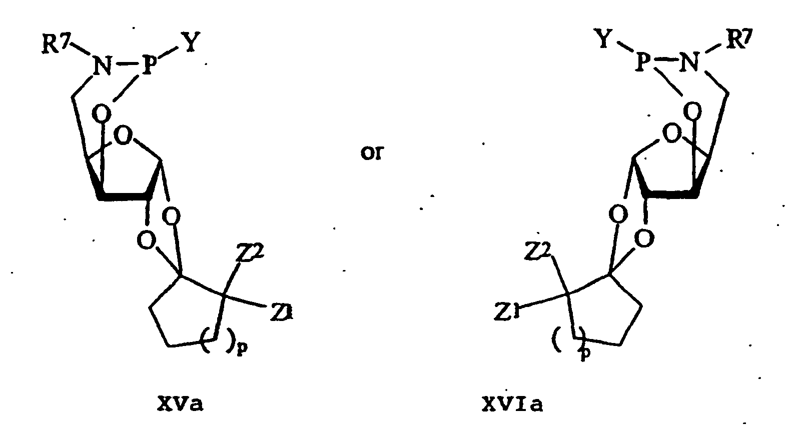 wherein Y, R⁷, Z¹, Z², and p are as defined above.

37. The method of claim 31 wherein said first synthon has the Formula XVIIa or XVIIIa: 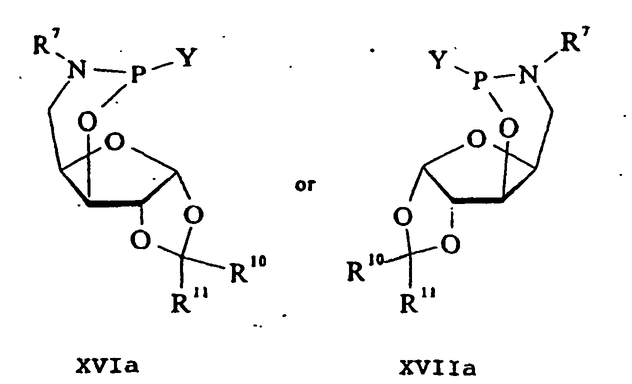 wherein W, R⁷, R¹⁰ and R¹¹ are as defined above.

38. The method of claim 1 further comprising removing said R⁹ groups.

39. The method of claim 1 further comprising removing said R² groups.

40. A method for the preparation of an internucleotide linkage comprising the steps of:
selecting a first synthon, said first synthon being selected from the group consisting of nucleoside phosphoramidites and nucleoside azaphospholanes;
selecting a second nucleoside synthon having a free 5'-hydroxyl group; and
reacting said first synthon and said second synthon in the presence of a catalyst to form said internuclotide linkage;
said catalyst having the formula: 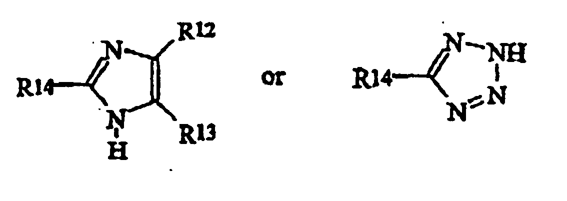 wherein:
R¹² and R¹³ are independently hydrogen, halogen, cyano, nitro, alkyl having from one to 10 carbons, substituted alkyl having from one to 10 carbons, an ester group, or R¹² and R¹³ together with the carbon atoms to which they are attached, form a substituted or unsubstituted phenyl ring where said substituents are electron withdrawing; and
R¹⁴ is hydrogen, halogen, cyano, nitro, thio, alkyl having from one to 10 carbons, substituted alkyl having from one to 10 carbons, norbornyl, substituted norbornyl, aryl, substituted aryl wherein said substituents are electron withdrawing, or has the formula: 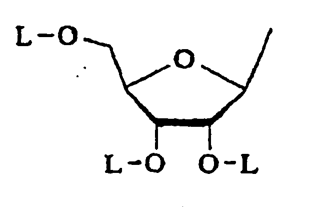
wherein L is protecting group.

41. The method of claim 40 wherein said internucleotide linkage is a phosphite linkage.

42. The method of claim 40 further comprising the step of oxidizing said internucleotide linkage to form a phosphodiester linkage, a phosphorothioate linkage, or a phosphorodithioate linkage.

43. The method of claim 23 or claim 40, wherein said catalyst is 2-bromo-4,5-dicyanoimidazole.

44. An oligomer of Formula IV according to claim 1: 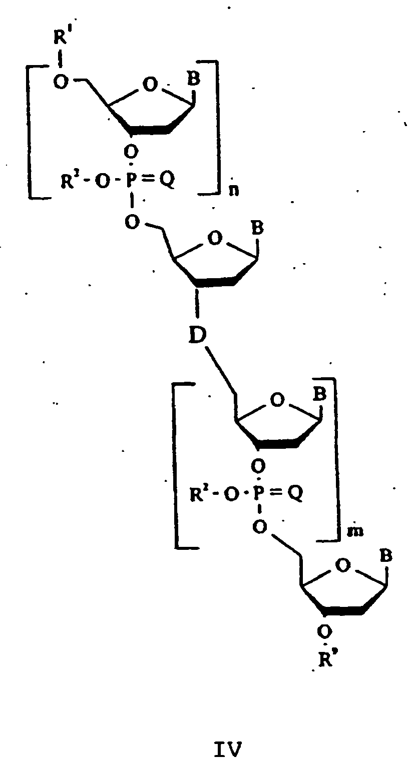 wherein D is a phosphorothioate linkage having the formula:

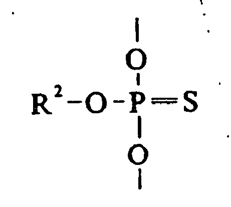

45. An azaphospholane of Formula VIb: 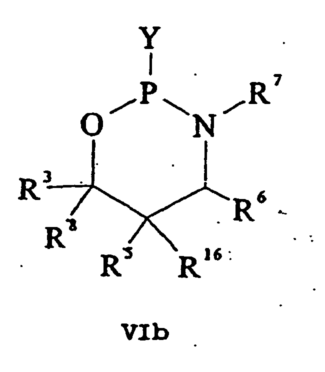 wherein:
Y is X or W wherein X is imidazole, triazole or substituted phenoxy wherein said substituents are electron withdrawing, and W has the formula: 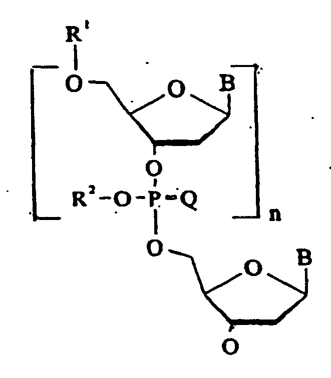
wherein:
Q is independently O or S;
R¹ is a hydroxyl protecting group;
R² is a chiral auxiliary of formula
-C(R⁸)R³-C(R¹⁶)R5-CHR⁶-NHR⁷;
R³ is hydrogen, alkyl having 1 to 6 carbon atoms, cyanomethyl, monohalomethyl, dihalomethyl, trihalomethyl, -CH₂R₄, -CH₂Si(R⁴)₃, or -CH₂-SOₖR⁴ where k is 0, 1 or 2;
R⁴ is independently alkyl, aryl, aralkyl or alkaryl having up to 15 carbon atoms, -N(R₇₀)-C(=O)-R₇₁, -S-C(=O)-R₇₀, or -O-C(=O)-O-N(R₇₀) (R₇₁);
R₇₀ and R₇₁ are each independently alkyl having 1 to 6 carbon atoms, α-halo substituted alkyl having 1 to 6 carbon atoms, aralkyl having 4 to 20 carbon atoms, α-halo substituted aralkyl having 4 to 20 carbon atoms, or aryl having 3 to 14 carbon atoms substituted with up to three electron whithdrawing groups;
R⁵ is H, -CN, -Si(R⁴)₃, SOₖR⁴ or halogen;
or R⁸ and R¹⁶ are each H, and R³ and R⁵, together, form one of the structures 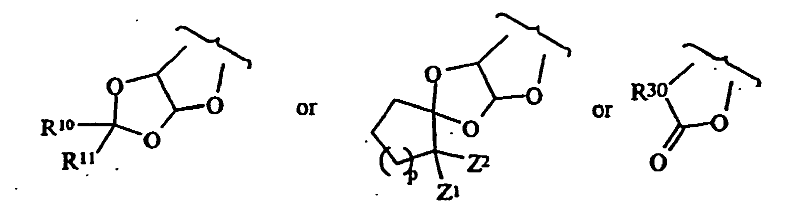 wherein:
_{R}¹⁰ and R¹¹ are H, alkyl having from 1 to 10 carbons, -CH₂C(=O)OR²², -CH₂CN, -CH₂Si(CH₃)₃, or o- or *p*-C₆H₄-R²¹;
R²¹ is hydrogen, -O-C(=O)CH₃, alkoxy having from 1 to 10 carbons, -NO₂, or -N(R²²)₂;
R²² is independently H or alkyl having from one to 10 carbon atoms;
p is 1 or 2;
Z¹ and Z² are independently halogen, CN, -Si(CH₃)₃, and -C(=O)OR²²;
R³⁰ is hydrogen, -O-C(=O)CH₃, alkoxy having from 1 to 10 carbons, or -O-Si(R₄)₃;
R⁶ is H, alkyl or aralkyl having up to 15 carbon atoms;
or R⁵ and R⁶, together with the atoms to which they are attached, form a 5 or 6 membered ring;
R⁷ is alkyl or aralkyl having up to 15 carbon atoms;
or R⁶ and R¹, together, form one of the structures 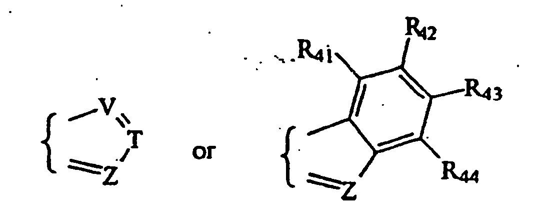
wherein X, T, and Z are independently CH or NH; or Z and R⁵, together with the atoms to which they are attached, form a phenyl ring;
R⁸ is H or methyl;
R⁹ is a hydroxyl protecting group or a solid support;
R¹⁶ is H, alkyl or aralkyl having up to 15 carbon atoms;
B is a nucleobase; and
n is an integer from 0 to 50.

46. The azaphospholane of claim 45 wherein Y is W and n is 0.

47. The azaphospholane of claim 45 wherein R⁷ is alkyl having up to 15 carbon atoms.

48. The azaphospholane of claim 45 wherein R⁷, R⁶ and the atoms to which they are attached form a 5 or 6 membered ring.

49. The azaphospholane of claim 46 wherein R³ is cyanomethyl or -CH₂-SOₖR⁴ where k is 0, 1 or 2.

50. The azaphospholane of claim 45 having one of the Formulas XVIc or XVId:

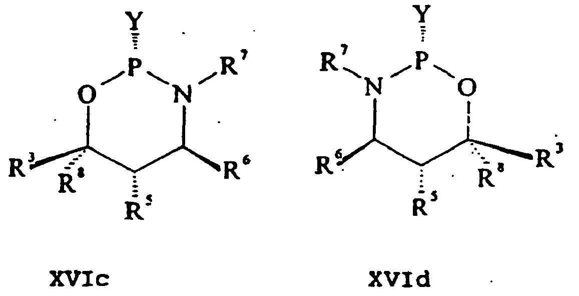

51. The azaphospholane of claim 45, having one of the Formulas Xb, XIb, XIIb, XIIIb or XXb: 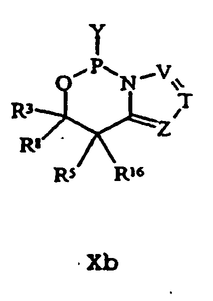 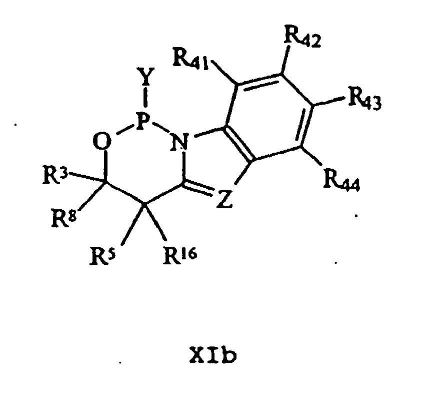 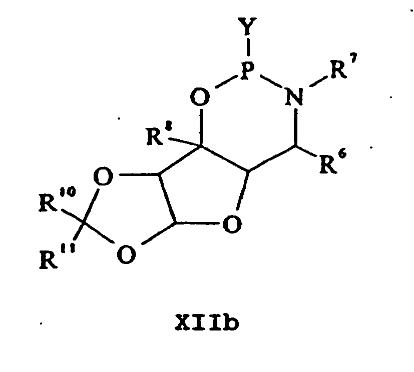 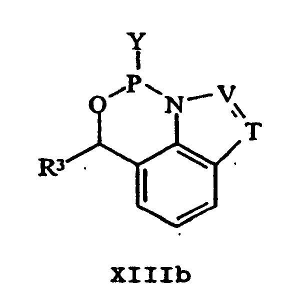 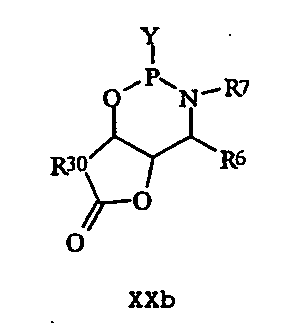 wherein R³-R¹⁶, Y, V, T, Z, Z₁, Z₂ and p are as defined above.

52. The azaphosphalane of claim 51 having the Formula XIa.

53. The azaphosphalane of claim 52 wherein R³ is -CH₂R₄; and R⁴ is independently -N(R₇₀)-C(=O)-R₇₁, -S-C(=O)-R₇₀, or -O-C(=O)-O-N(R₇₀) (R₇₁).

54. The azaphosphalane of claim 52 wherein R₇₀ and R₇₁ are independently alkyl having 1 to 6 carbon atoms or α-halo substituted alkyl having 1 to 6 carbons atoms.

55. The azaphosphalane of claim 51 having the Formula XIVb: 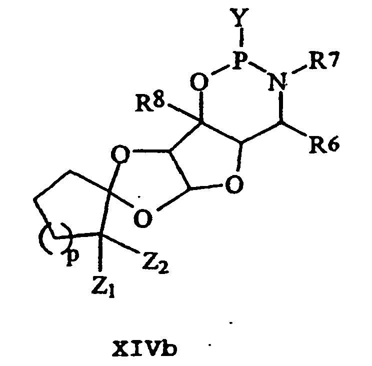 wherein Y, R⁶, R⁷ R⁸, p, Z¹ and Z² are as defined above.

56. The azaphosphalane of claim 50 having the Formula Xd or Xe: 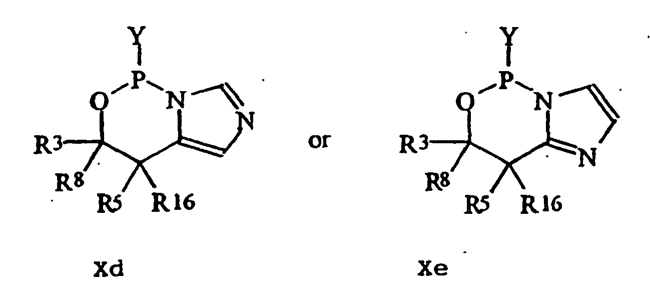 wherein Y, R³, R⁵, R⁸, and R¹⁶ are as defined above.

57. The azaphospholane of claim 51 having the Formula XVIIb or XVIIIb: 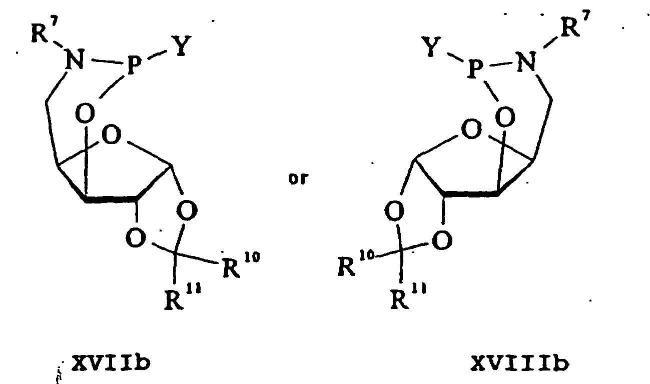 wherein Y, R⁷, R¹⁰ and R¹¹ are as defined above.

58. The azaphospholane of claim 49 having the Formula XVb or XVIb: 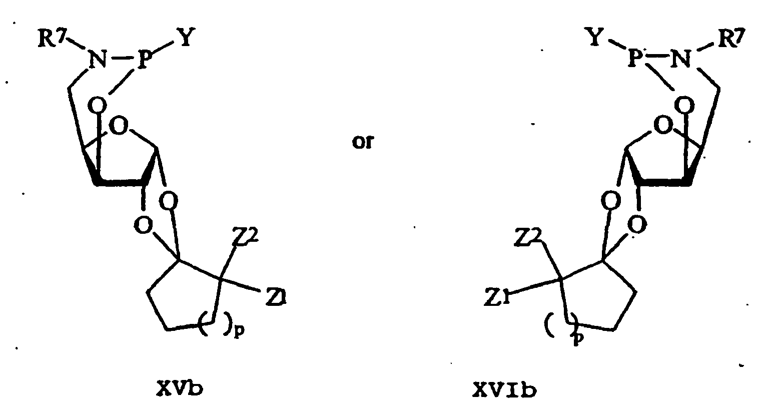 wherein Y, R⁷, Z¹, Z², and p are as defined above.

59. The azaphospholane of claim 45 wherein said azaphospholane is diastereomerically enriched.

60. The azaphospholane of claim 45 wherein 75% of said azaphospholane is in a single stereoisomeric form.

61. The azaphospholane of claim 45 wherein 85% of said azaphospholane is in a single stereoisomeric form.

62. The azaphospholane of claim 45 wherein 95% of said azaphospholane is in a single stereoisomeric form.

63. The azaphospholane of claim 45 wherein said azaphospholane is in a single stereoisomeric form, substantially free of other stereoisomeric forms.

64. An oligomeric compound of Formula III, according to claim 1, comprising a moiety having a phosphite linkage of the Formula XXX: 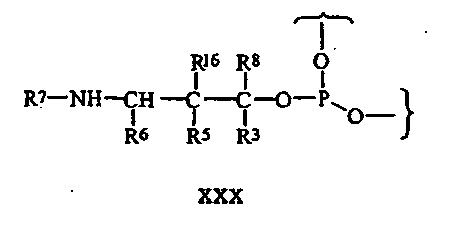 wherein:
R₃, R₅-R₈ and R₁₆ are as defined according to claim 1.

65. The oligomeric compound of claim 64 wherein 75% of said phosphite linkage is in a single stereoisomeric form.

66. The oligomeric compound of claim 64 wherein 85% of said phosphite linkage is in a single stereoisomeric form.

67. The oligomeric compound of claim 64 wherein 95% of said phosphite linkage is in a single stereoisomeric form.

68. The oligomeric compound of claim 64 wherein said phosphosphite linkage is in a single stereoisomeric form, substantially free of other stereoisomeric forms.

## Patentansprüche

1. Verfahren zur Synthese eines Oligomers mit einer Phosphorothioatverkrüpfung, umfassend die Schritte:
- Umsetzen eines ersten Synthons der Formel I: 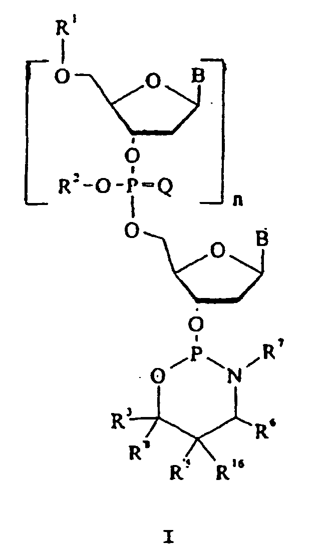 wobei
Q unabhängig O oder S ist;
R¹ eine Hydroxyl-Schutzgruppe ist;
R² eine chirale Hilfsgruppe der Formel -C(R⁸)R³-C(R¹⁶)R⁵-CHR⁶-NHR⁷ ist;
R³ Wasserstoff, Alkyl mit 1 bis 6 Kohlenstoffatomen, Cyanomethyl, Monohalogenmethyl, Dihalogenmethyl, Trihalogenmethyl, -CH₂R₄, -CH₂Si(R⁴)₃ oder -CH₂-SOₖR⁴, wobei k 0, 1 oder 2 ist, ist;
R⁴ unabhängig Alkyl, Aryl, Aralkyl oder Alkaryl mit bis zu 15 Kohlenstoffatomen, -N(R₇₀)-C(=O)-R₇₁, -S-C(=O)-R₇₀ oder -O-C(=O)-O-N(R₇₀)(R₇₁) ist;
R₇₀ und R₇₁ jeweils unabhängig Alkyl mit 1 bis 6 Kohlenstoffatomen, α-Halogen-substituiertes Alkyl mit 1 bis 6 Kohlenstoffatomen, Aralkyl mit 4 bis 20 Kohlenstoffatomen, α-Halogen-substituiertes Aralkyl mit 4 bis 20 Kohlenstoffatomen oder Aryl mit 3 bis 14 Kohlenstoffatomen, die mit bis zu drei elektronenziehenden Gruppen substituiert sind;
R⁵ H, -CN, -Si(R⁴)₃, SOₖR⁴ oder Halogen ist;
oder R⁸ und R¹⁶ jeweils H sind und R³ und R⁵ zusammen eine der Strukturen 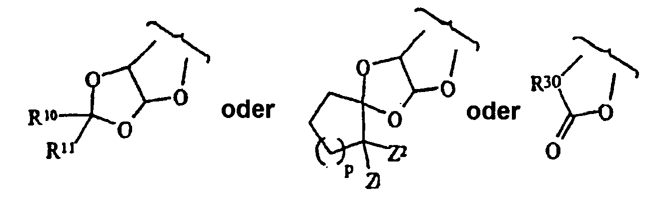 bilden, wobei:
R¹⁰ und R¹¹ H, Alkyl mit 1 bis 10 Kohlenstoffen, -CH₂C(=O)OR²², -CH₂CN, -CH₂Si(CH₃)₃ oder o- oder P-C₆H₄-R²¹ sind;
R²¹ Wasserstoff, -O-C(=O)CH₃, Alkoxy mit 1 bis 10 Kohlenstoffen, -NO₂ oder -N(R²²)₂ ist;
R²² unabhängig H oder Alkyl mit 1 bis 10 Kohlenstoffatomen ist;
p 1 oder 2 ist;
Z¹ und Z² unabhängig Halogen, CN, -Si(CH₃)₃ und -C(=O)OR²² sind;
R³⁰ Wasserstoff, -O-C(=O)CH₃, Alkoxy mit 1 bis 10 Kohlenstoffen oder -O-Si(R₄)₃ ist;
R⁶ H, Alkyl oder Aralkyl mit bis zu 15 Kohlenstoffatomen ist;
oder R⁵ und R⁶ zusammen mit den Atomen, an die sie gebunden sind, einen 5- oder 6-gliedrigen Ring bilden;
R⁷ Alkyl oder Aralkyl mit bis zu 15 Kohlenstoffatomen ist; oder R⁶ und R⁷ zusammen eine der Strukturen 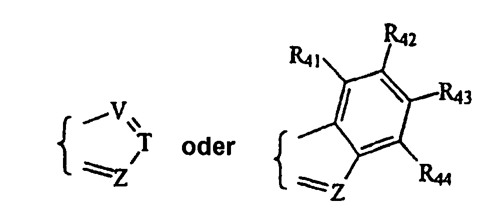 bilden, wobei V, T und Z unabhängig CH oder N sind;
R₄₁, R₄₂, R₄₃ und R₄₄ jeweils unabhängig H oder eine elektronenziehende Gruppe sind;
R⁸ H oder Methyl ist;
R¹⁶ H, Alkyl oder Aralkyl mit bis zu 15 Kohlenstoffatomen ist;
B eine Nucleobase ist; und
n eine ganze Zahl von 0 bis 50 ist;
mit einem zweiten Synthon der Formel II: 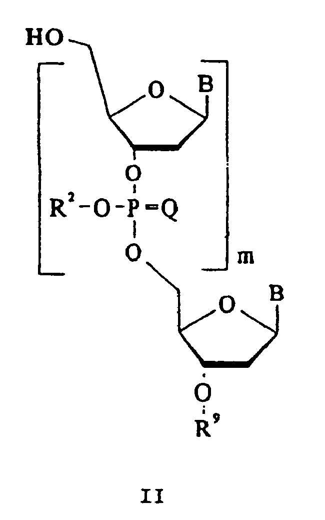 wobei R⁹ eine Hydroxyl-Schutzgruppe oder ein Linker ist, der an einem festen Täger gebunden ist; und
m eine ganze Zahl von 0 bis 50 ist;
fr eine Dauer und unter Reaktionsbedingungen, die zur Bildung eines dritten Synthons der Formel III wirksam sind: 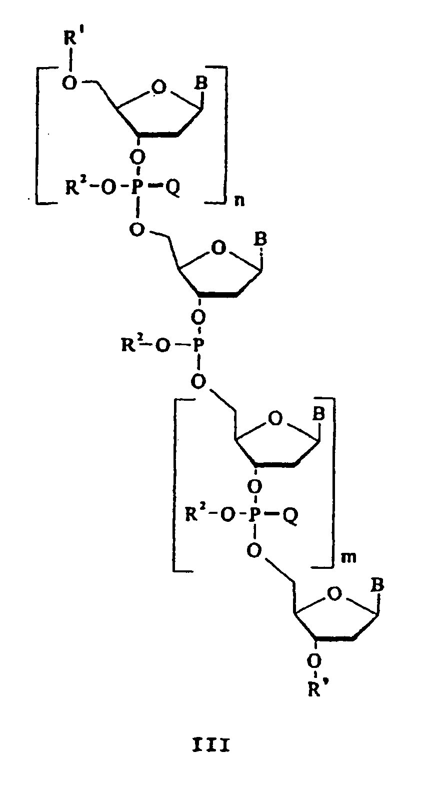
und
- Zusammenbringen des dritten Synthons mit einem Sulfurierungsmittels unter Bildung eines Oligomers der Formel IV: 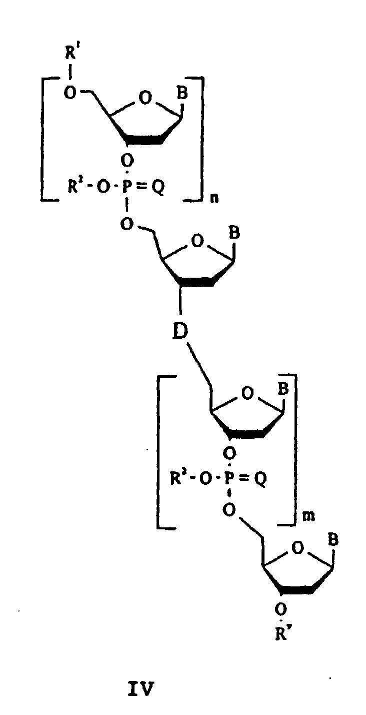
wobei D die Phosphorothioatverkrüpfung mit der Formel 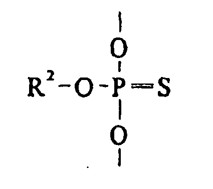 ist.

2. Verfahren nach Anspruch 1, wobei die Phosphorothioatverkrüpfung diastereomerisch angereichert ist.

3. Verfahren nach Anspruch 1, wobei 75 % der Phosphorothioatverkrüpfung in einer einzigen stereoisomeren Form vorliegen.

4. Verfahren nach Anspruch 1, wobei 85 % der Phosphorothioatverkrüpfung in einer einzigen stereoisomeren Form vorliegen.

5. Verfahren nach Anspruch 1, wobei 95 % der Phosphorothioatverkrüpfung in einer einzigen stereoisomeren Form vorliegen.

6. Verfahren nach Anspruch 1, wobei die Phosphorothioatverkrüpfung in einer einzigen stereoisomeren Form, im Wesentlichen frei von anderen stereoisomeren Formen, vorliegt.

7. Verfahren nach Anspruch 1, wobei n 0 ist.

8. Verfahren nach Anspruch 1, wobei das erste Synthon in einer einzigen stereoisomeren Form, im Wesentlichen frei von anderen stereoisomeren Formen, vorliegt.

9. Verfahren nach Anspruch 1, das außerdem die Entfernung der R¹-Gruppe aus dem Phosphorothioat umfasst.

10. Verfahren nach Anspruch 1, das außerdem die Entfernung der chiralen Hilfsgruppen umfasst.

11. Verfahren nach Anspruch 10, wobei die Entfernung der chiralen Hilfsgruppenüber β-Eliminierung erfolgt.

12. Verfahren nach Anspruch 10, wobei die Entfernung der chiralen Hilfsgruppen mit Behandlung durch Säurereagentien erfolgt.

13. Verfahren nach Anspruch 1, wobei die Verbindung der Formel IV eine Vielzahl der Phosphorothioatverkrüpfungen enthält.

14. Verfahren nach Anspruch 1, wobei das erste und zweite Synthon bei einer Temperatur von -20°C bis 40°C umgesetzt werden.

15. Verfahren nach Anspruch 1, wobei das erste und zweite Synthon bei einer Temperatur von -15°C bis 0°C umgesetzt werden.

16. Verfahren nach Anspruch 1, wobei das erste Synthon durch Umsetzen einer Verbindung der Formel V: 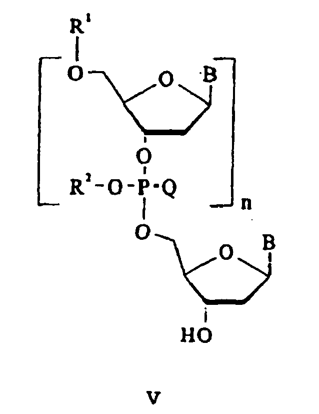 mit einem Azaphospholan der Formel VIa 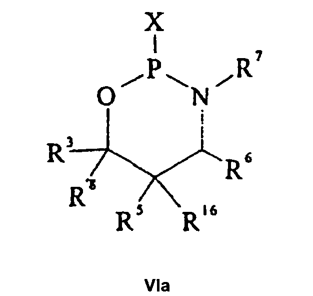 wobei R³-R⁸ wie vorstehend definiert sind; und X Halogen, Dialkylamino, Imidazol, Triazol oder substituiertes Phenoxy ist, wobei die Substituenten elektronenziehend sind.

17. Verfahren nach Anspruch 16, wobei die elektronenziehenden Substituenten Halogen oder Nitro sind.

18. Verfahren nach Anspruch 16, wobei das Azaphospholan durch Umsetzung eines Reagens der Formel HO-C(R⁸)R³-C(R¹⁶)R⁵-CHR⁶⁻NHR⁷ und von Phosphortrihalogenid, Phosphor-tri(dialkylamid), Phosphortriphenoxid oder Phosphor-tri(imidazoliden) hergestellt wird.

19. Verfahren nach Anspruch 1, wobei das erste Synthon durch Umsetzung einer Verbindung der Formel VII 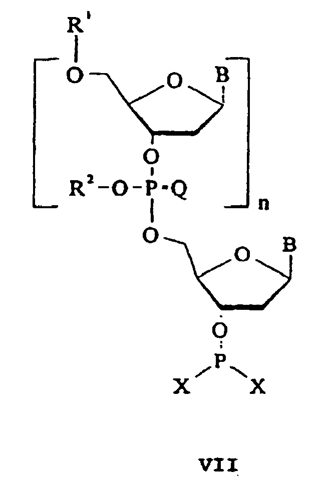 und eines γ-Aminoalkohols der Formel HO-C(R⁸)R³-C(R¹⁶)R⁵-CHR⁶⁻NHR⁷; wobei X und R¹-R⁸ und R¹⁶ wie vorstehend definiert sind, gebildet wird.

20. Verfahren nach Anspruch 19, wobei X Chlor ist.

21. Verfahren nach Anspruch 19, wobei die Umsetzung stereoselektiv ist.

22. Verfahren nach Anspruch 20, wobei das erste Synthon in einer einzigen stereoisomeren Form, im Wesentlichen frei von anderen stereoisomeren Formen, vorliegt.

23. Verfahren nach Anspruch 1, wobei die Umsetzung in Gegenwart eines Katalysators durchgefhrt wird.

24. Verfahren nach Anspruch 23, wobei der Katalysator eine der Formeln VIII oder IX aufweist: 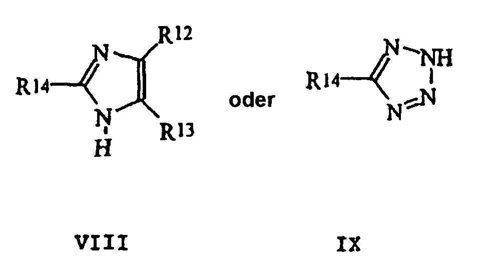 wobei R¹² und R¹³ unabhängig Wasserstoff, Halogen, Cyano, Nitro, Alkyl mit 1 bis 10 Kohlenstoffen, substituiertes Alkyl mit 1 bis 10 Kohlenstoffen, eine Estergruppe sind oder R¹² und R¹³ zusammen mit den Kohlenstoffatomen, an die sie gebunden sind, einen substituierten oder unsubstituierten Phenylring bilden, wobei die Substituenten elektronenziehend sind; und
R¹⁴ Wasserstoff, Halogen, Cyano, Nitro, Thio, Alkyl mit 1 bis 10 Kohlenstoffen, substituiertes Alkyl mit 1 bis 10 Kohlenstoffen, Norbornyl, substituiertes Norbornyl, Aryl mit 3 bis 14 Kohlenstoffatomen, substituiertes Aryl mit 3 bis 14 Kohlenstoffatomen, wobei die Substituenten elektronenziehend sind, ist oder die Formel 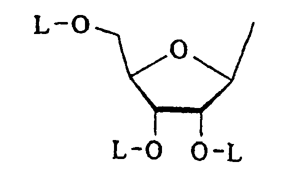 aufweist, wobei L eine Schutzgruppe ist.

25. Verfahren nach Anspruch 23, wobei R¹⁴ Halogen oder Nitro ist und wobei R¹² und R¹³ jeweils Halogen oder jeweils Cyano sind.

26. Verfahren nach Anspruch 24, wobei R¹⁴ Brom ist und R¹² und R¹³ jeweils Cyano sind.

27. Verfahren nach Anspruch 24, wobei R¹⁴, R¹² und R¹³ jeweils Brom sind.

28. Verfahren nach Anspruch 23, wobei R¹⁴ eine der Formeln 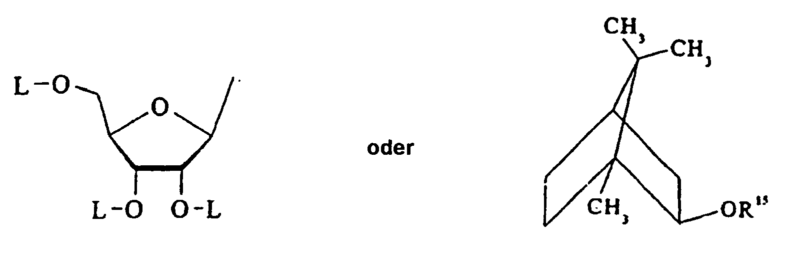 aufweist, wobei R¹⁵ H, Methyl, Trialkylsilyl oder Acetyl ist.

29. Verfahren nach Anspruch 1, wobei R³ Cyanomethyl oder -CH₂-SOₖR⁴, wobei k 0, 1 oder 2 ist, ist.

30. Verfahren nach Anspruch 29, wobei R⁷ Alkyl mit 1 bis 6 Kohlenstoffatomen oder Aralkyl mit bis zu 15 Kohlenstoffatomen ist.

31. Verfahren nach Anspruch 1, wobei das erste Synthon eine der Formeln Xa, XIa, XIIa, XIIIa oder XXa ist: 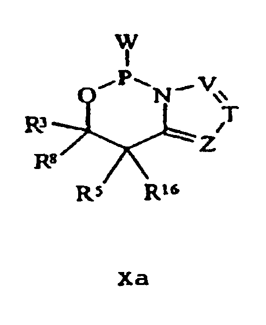 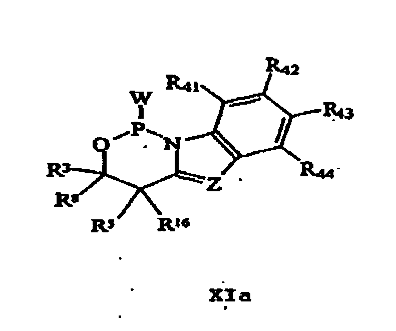 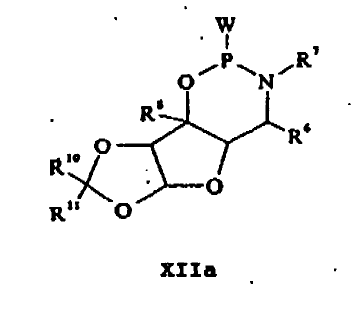 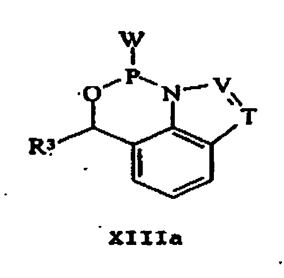 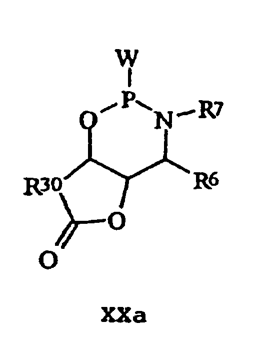 wobei W die Formel 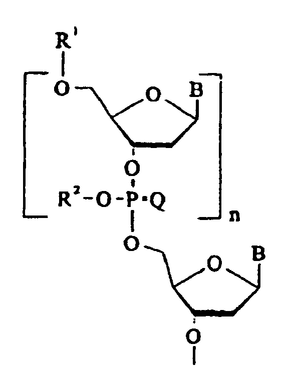 aufweist und R¹-R¹⁶, V, T und Z wie vorstehend definiert sind.

32. Verfahren nach Anspruch 31, wobei das erste Synthon die Formel Xla aufweist; R³-CH₂R₄ ist; und R⁴ unabhängig -N(R₇₀)-C(=O)-R₇₁, -S-C(=O)-R₇₀ oder-O-C(=O)-O-N(R₇₀)(R₇₁) ist.

33. Verfahren nach Anspruch 32, wobei R₇₀ und R₇₁ unabhängig Alkyl mit 1 bis 6 Kohlenstoffatomen oder ein α-Halogen-substituiertes Alkyl mit 1 bis 6 Kohlenstoffatomen sind.

34. Verfahren nach Anspruch 31, wobei das erste Synthon die Formel Xb oder Xc aufweist:

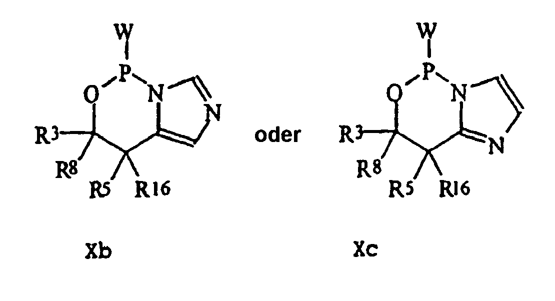

35. Verfahren nach Anspruch 31, wobei das erste Synthon die Formel XIVa aufweist: 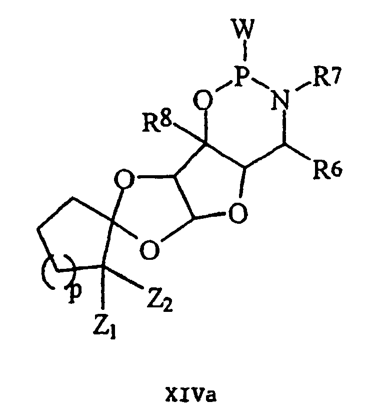 wobei p, Z¹ und Z² wie vorstehend definiert sind.

36. Verfahren nach Anspruch 35, wobei das erste Synthon die Formel XVa oder XVIa aufweist: 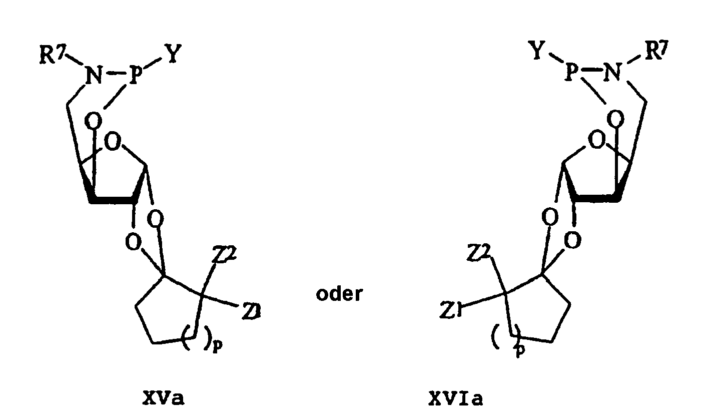 wobei Y, R⁷, Z¹, Z² und p wie vorstehend definiert sind.

37. Verfahren nach Anspruch 31, wobei das erste Synthon die Formel XVIIa oder XVIIIa aufweist: 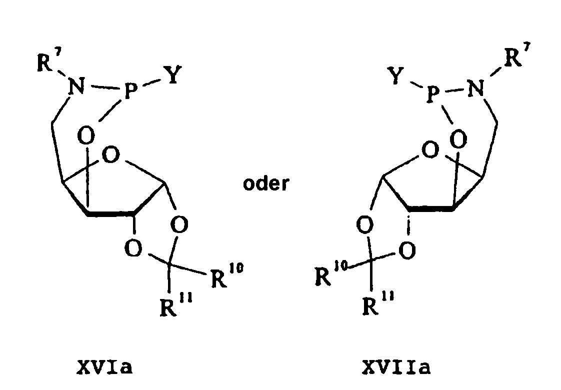 wobei W, R⁷, R¹⁰ und R¹¹ wie vorstehend definiert sind.

38. Verfahren nach Anspruch 1, das außerdem die Entfernung der R⁹-Gruppen umfasst.

39. Verfahren nach Anspruch 1, das außerdem die Entfernung der R²-Gruppen umfasst.

40. Verfahren zur Herstellung einer Internucleotidverkrüpfung, umfassend die Schritte:
Auswählen eines ersten Synthons, wobei das erste Synthon aus der Gruppe ausgewählt ist, bestehend aus Nucleosidphosphoramiditen und Nucleosidazaphospholanen;
Auswählen eines zweiten Nucleosid-Synthons mit einer freien 5'-Hydroxylgruppe; und
Umsetzen des ersten und zweiten Synthons in Gegenwart eines Katalysators unter Bildung der Internucleotidverkrüpfung;
wobei der Katalysator die Formel 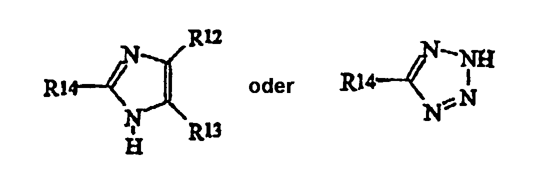 aufweist, wobei
R¹² und R¹³ unabhängig Wasserstoff, Halogen, Cyano, Nitro, Alkyl mit 1 bis 10 Kohlenstoffen, substituiertes Alkyl mit 1 bis 10 Kohlenstoffen, eine Estergruppe sind oder R¹² und R¹³ zusammen mit den Kohlenstoffatomen, an die sie gebunden sind, einen substituierten oder unsubstituierten Phenylring, wobei die Substituenten elektronenziehend sind, bilden; und
R¹⁴ Wasserstoff, Halogen, Cyano, Nitro, Thio, Alkyl mit 1 bis 10 Kohlenstoffen, substituiertes Alkyl mit 1 bis 10 Kohlenstoffen, Norbornyl, substituiertes Norbornyl, Aryl, substituiertes Aryl, wobei die Substituenten elektronenziehend sind, ist oder die Formel 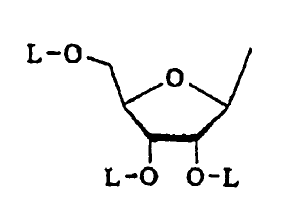 aufweist, wobei L eine Schutzgruppe ist.

41. Verfahren nach Anspruch 40, wobei die Internucleotidverkrüpfung eine Phosphitverkrüpfung ist.

42. Verfahren nach Anspruch 40, das außerdem den Schritt der Oxidation der Internucleotidverkrüpfung unter Bildung einer Phosphordiesterverkrüpfung, einer Phosphorothioatverkrüpfung oder einer Phosphorodithioatverkrüpfung umfasst.

43. Verfahren nach Anspruch 23 oder Anspruch 40, wobei der Katalysator 2-Brom-4,5-dicyanoimidazol ist.

44. Oligomer der Formel IV nach Anspruch 1: 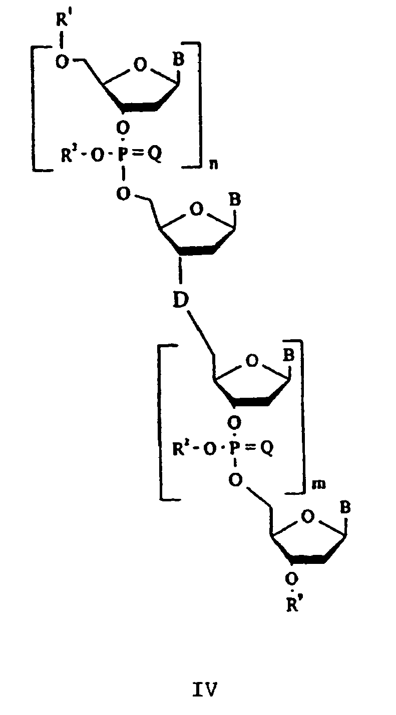 wobei D eine Phosphorothioatverkrüpfung der Formel 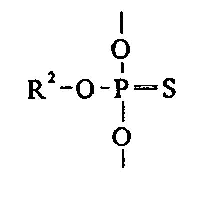 ist.

45. Azaphospholan der Formel Vlb 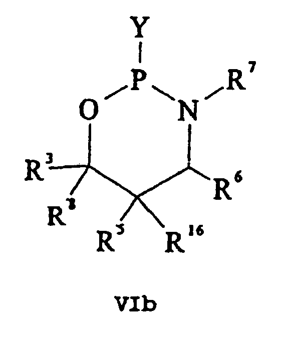 wobei:
Y X oder W ist, wobei X Imidazol, Triazol oder substituiertes Phenoxy ist, wobei die Substituenten elektronenziehend sind, und W die Formel 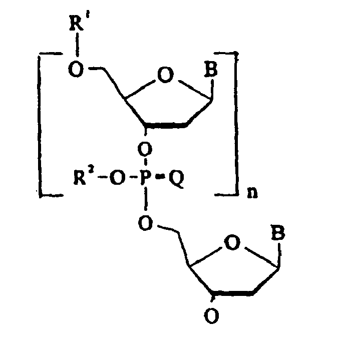 aufweist, wobei
Q unabhängig O oder S ist;
R¹ eine Hydroxyl-Schutzgruppe ist;
R² eine chirale Hilfsgruppe der Formel -C(R⁸)R³-C(R¹⁶)R⁵⁻CHR⁶-NHR⁷ ist;
R³ Wasserstoff, Alkyl mit 1 bis 6 Kohlenstoffatomen, Cyanomethyl, Monohalogenmethyl, Dihalogenmethyl, Trihalogenmethyl, -CH₂R₄, -CH₂Si(R⁴)₃ oder -CH₂-SOₖR⁴, wobei k 0, 1 oder 2 ist, ist;
R⁴ unabhängig Alkyl, Aryl, Aralkyl oder Alkaryl mit bis zu 15 Kohlenstoffatomen, -N(R₇₀)-C(=O)-R₇₁, -S-C(=O)-R₇₀ oder -O-C(=O)-O-N(R₇₀)(R₇₁) ist;
R₇₀ und R₇₁ jeweils unabhängig Alkyl mit 1 bis 6 Kohlenstoffatomen, α-Halogen-substituiertes Alkyl mit 1 bis 6 Kohlenstoffatomen, Aralkyl mit 4 bis 20 Kohlenstoffatomen, α-Halogen-substituiertes Aralkyl mit 4 bis 20 Kohlenstoffatomen oder Aryl mit 3 bis 14 Kohlenstoffatomen, substituiert mit bis zu drei elektronenziehenden Gruppen, sind;
R⁵ H, -CN, -Si(R⁴)₃, SOₖR⁴ oder Halogen ist;
oder R⁸ und R¹⁶ jeweils H sind und R³ und R⁵ zusammen eine der Strukturen 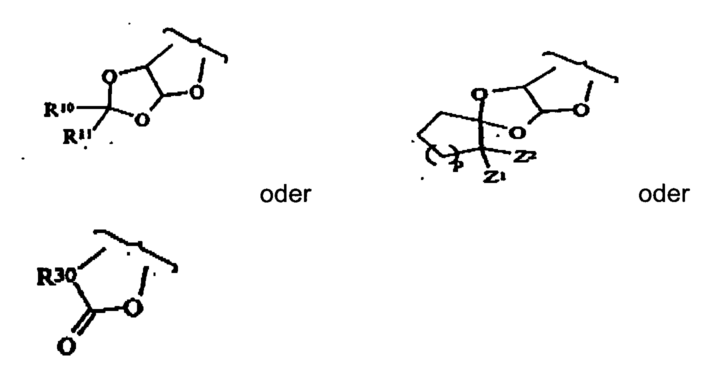 bilden, wobei R¹⁰ und R¹¹ H, Alkyl mit 1 bis 10 Kohlenstoffen -CH₂C(=O)OR²², -CH₂CN, -CH₂Si(CH₃)₃ oder o- oder p-C₆H₄-R²¹ sind;
R²¹ Wasserstoff -O-C(=O)CH₃, Alkoxy mit 1 bis 10 Kohlenstoffen, -NO₂ oder -N(R²²)₂ ist;
R²² unabhängig H oder Alkyl mit 1 bis 10 Kohlenstoffatomen ist; p 1 oder 2 ist;
Z¹ und Z² unabhängig Halogen, CN, -Si(CH₃)₃ und -C(=O)OR²² sind;
R³⁰ Wasserstoff, -O-C(=O)CH₃, Alkoxy mit 1 bis 10 Kohlenstoffen oder -O-Si(R₄)₃ ist;
R⁶ H, Alkyl oder Aralkyl mit bis zu 15 Kohlenstoffatomen ist;
oder R⁵ und R⁶ zusammen mit den Atomen, an die sie gebunden sind, einen 5- oder 6-gliedrigen Ring bilden;
R⁷Alkyl oder Aralkyl mit bis zu 15 Kohlenstoffatomen ist;
oder R⁶ und R⁷ zusammen eine der Strukturen 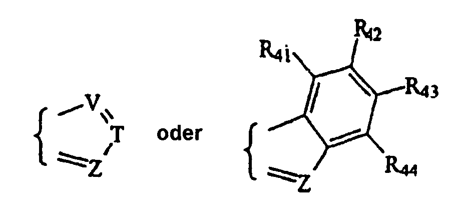 bilden, wobei X, T und Z unabhängig CH oder NH sind;
oder Z und R⁵ zusammen mit den Atomen, an die sie gebunden sind, einen Phenylring bilden;
R⁸ H oder Methyl ist;
R⁹ eine Hydroxyl-Schutzgruppe oder ein fester Täger ist;
R¹⁶ H, Alkyl oder Aralkyl mit bis zu 15 Kohlenstoffatomen ist;
B eine Nucleobase ist; und
n eine ganze Zahl von 0 bis 50 ist.

46. Azaphospholan nach Anspruch 45, wobei Y W ist und n 0 ist.

47. Azaphospholan nach Anspruch 45, wobei R⁷ Alkyl mit bis zu 15 Kohlenstoffatomen ist.

48. Azaphospholan nach Anspruch 45, wobei R⁷, R⁶ und die Atome, an die sie gebunden sind, einen 5- oder 6-gliedrigen Ring bilden.

49. Azaphospholan nach Anspruch 46, wobei R³ Cyanomethyl oder -CH₂-SOₖR⁴, wobei k 0, 1 oder 2 ist, ist.

50. Azaphospholan nach Anspruch 45 mit einer der Formeln XVIc oder XVId:

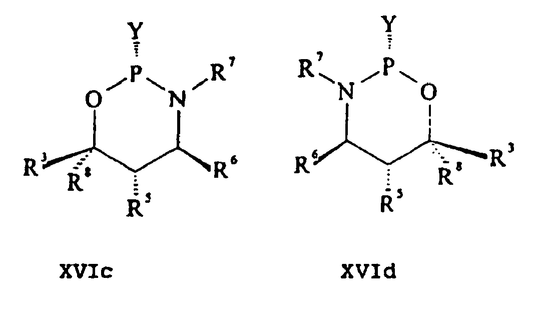

51. Azaphospholan nach Anspruch 45, mit einer der Formeln Xb, Xlb, Xllb, Xlllb oder XXb: 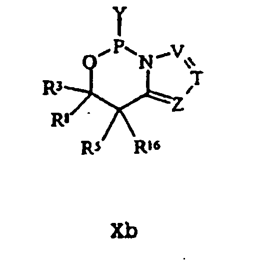 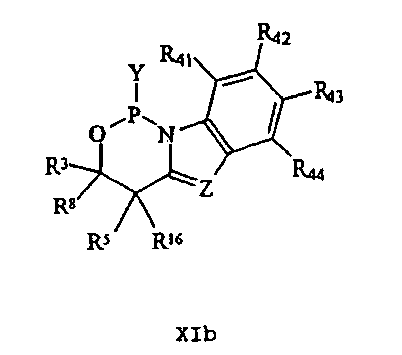 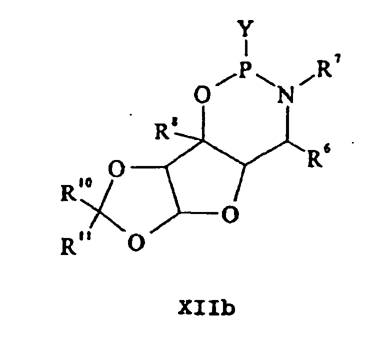 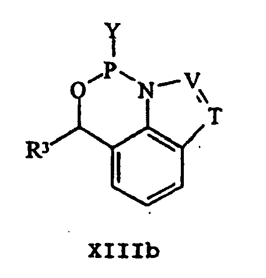 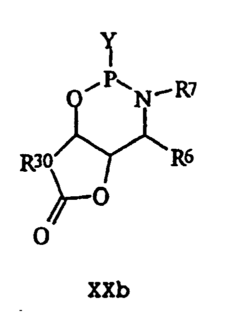 wobei R³-R¹⁶, Y, V, T, Z, Z₁, Z₂ und p wie zuvor definiert sind.

52. Azaphospholan nach Anspruch 51 mit der Formel XIa.

53. Azaphospholan nach Anspruch 52, wobei R³ -CH₂R₄ ist; und R⁴ unabhängig -N(R₇₀)-C(=O)-R₇₁, -S-C(=O)-R₇₀ oder -O-C(=O)-O-N(R₇₀)(R₇₁) ist.

54. Azaphospholan nach Anspruch 52, wobei R₇₀ und R₇₁ unabhängig Alkyl mit 1 bis 6 Kohlenstoffatomen oder ein α-Halogen-substituiertes Alkyl mit 1 bis 6 Kohlenstoffatomen sind.

55. Azaphospholan nach Anspruch 51 mit der Formel XIVb: 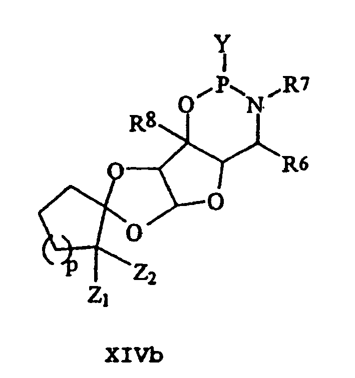 wobei Y, R⁶, R⁷, R⁸, p, Z¹ und Z² wie zuvor definiert sind.

56. Azaphospholan nach Anspruch 50 mit der Formel Xd oder Xe: 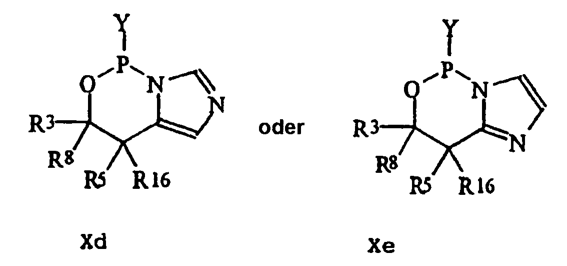 wobei Y R³, R⁵, R⁸ und R¹⁶ wie zuvor definiert sind.

57. Azaphospholan nach Anspruch 51 mit der Formel XVllb oder XVlllb: 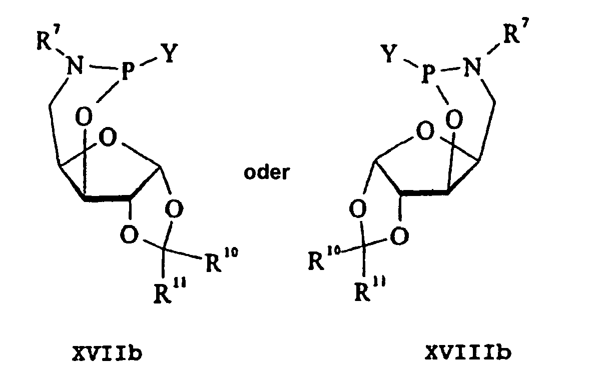 wobei Y, R⁷, R¹⁰ und R¹¹ wie zuvor definiert sind.

58. Azaphospholan nach Anspruch 49 mit der Formel XVb oder XVIb: 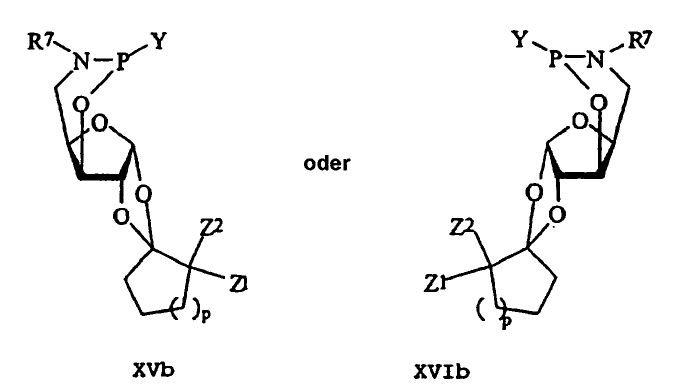 wobei Y, R⁷, Z¹, Z² und p wie zuvor definiert sind.

59. Azaphospholan nach Anspruch 45, wobei das Azaphospholan diastereomerisch angereichert ist.

60. Azaphospholan nach Anspruch 45, wobei 75 % des Azaphospholans in einer einzigen stereoisomeren Form vorliegen.

61. Azaphospholan nach Anspruch 45, wobei 85 % des Azaphospholans in einer einzigen stereoisomeren Form vorliegen.

62. Azaphospholan nach Anspruch 45, wobei 95 % des Azaphospholans in einer einzigen stereoisomeren Form vorliegen.

63. Azaphospholan nach Anspruch 45, wobei das Azaphospholan in einer einzigen stereoisomeren Form, im Wesentlichen frei von anderen stereoisomeren Formen, vorliegt.

64. Oligomere Verbindung der Formel III nach Anspruch 1, umfassend eine Gruppierung mit einer Phosphitverkrüfung der Formel XXX 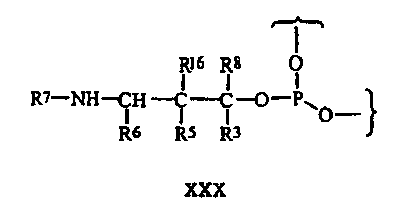 wobei R³, R⁵-R⁸ und R¹⁶ wie nach Anspruch 1 definiert sind.

65. Oligomere Verbindung nach Anspruch 64, wobei 75 % der Phosphitverkrüpfung in einer einzigen stereoisomeren Form vorliegen.

66. Oligomere Verbindung nach Anspruch 64, wobei 85 % der Phosphitverkrüpfung in einer einzigen stereoisomeren Form vorliegen.

67. Oligomere Verbindung nach Anspruch 64, wobei 95 % der Phosphitverkrüpfung in einer einzigen stereoisomeren Form vorliegen.

68. Oligomere Verbindung nach Anspruch 64, wobei die Phosphitverkrüpfung in einer einzigen stereoisomeren Form, im Wesentlichen frei von anderen stereoisomeren Formen, vorliegt.

## Revendications

1. Procédé de synthèse d'un oligomère ayant une liaison phosphorothioate comprenant les stades dans lesquels :
- on fait réagir un premier synthon de Formule I : 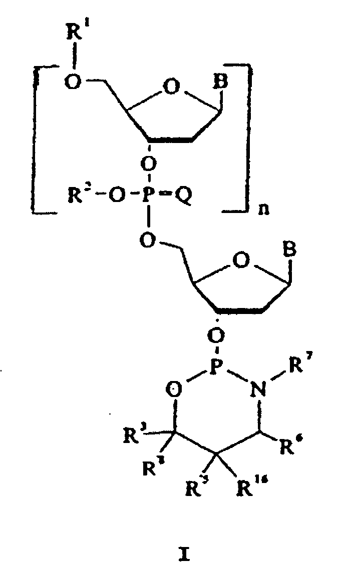 dans laquelle :
Q est indépendamment O ou S ;
R¹ est un groupe de protection d'un hydroxyle ;
R² est un auxiliaire chiral de formule
-C(R⁸)R³-C(R¹⁶)R⁵-CHR⁶-NHR⁷ ;
R³ est hydrogène, alcoyle ayant de 1 à 6 atomes de carbone, cyanométhyle, monohalométhyle, dihalométhyle, trihalométhyle, -CH₂R₉, -CH₂Si(R⁴)₃ ou -CH₂-SOₖR⁴ dans laquelle k est 0, 1 ou 2 ;
R⁴ est indépendamment alcoyle, aryle, aralcoyle ou alcoylaryle ayant jusqu'à 15 atomes de carbone, -N(R₇₀) -C(=O)-R₇₁, -S-C (=O) -R₇₀ ou -O-C(=O)-O-N(R₇₀) (R₇₁) ;
R₇₀ et R₇₁ sont chacun indépendamment alcoyle ayant de 1 à 6 atomes de carbone, alcoyle substitué en position α par un halogène et ayant de 1 à 6 atomes de carbone, aralcoyle ayant de 4 à 20 atomes de carbone, aralcoyle substitué en position α par un halogène et ayant de 4 à 20 atomes de carbone, ou aryle ayant de 3 à 14 atomes de carbone, substitué par jusqu'à trois groupes attirant les électrons ;
R⁵ est H, -CN, -Si(R⁴)₃, SOₖR⁴ ou halogène ;
ou R⁸ et R¹⁶ sont chacun H et R³ et R⁵ forment ensemble l'une des structures 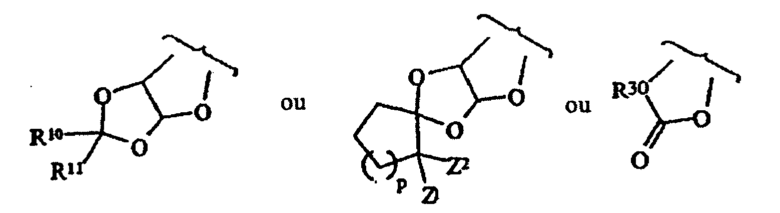 dans lesquelles :
R¹⁰ et R¹¹ sont H, alcoyle ayant de 1 à 10 atomes de carbone, -CH₂C(=O)OR²², -CH₂CN, -CH₂Si (CH₃)₃ ou *o*-C₆H₄-R²¹ ou *p*-C₆H₄-R²¹;
R²¹ est hydrogène, -O-C(=O)CH₃, alcoxy ayant de 1 à 10 atomes de carbone, -NO₂ ou -N(R²²)₂;
R²² est indépendamment H ou alcoyle ayant de 1 à 10 atomes de carbone ;
p est 1 ou 2 ;
Z¹ et Z² sont indépendamment halogène, CN, -Si(CH₃)₃ et -C(=O)OR²² ;
R³⁰ est hydrogène, -O-C(=O)CH₃, alcoxy ayant de 1 à 10 atomes de carbone ou -O-Si(R₄)₃ ;
R⁶ est H, alcoyle ou aralcoyle ayant jusqu'à 15 atomes de carbone ;
ou R⁵ et R⁶ forment ensemble avec les atomes auxquels ils sont fixés un cycle à 5 ou 6 chaînons ;
R⁷ est alcoyle ou aralcoyle ayant jusqu'à 15 atomes de carbone ;
ou R⁶ et R⁷ forment ensemble l'une des structures 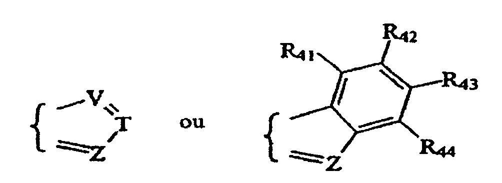
dans lesquelles V, T et Z sont indépendamment CH ou N ;
R₄₁, R₄₂, R₄₃ et R₄₄ sont chacun indépendamment H ou un groupe attirant les électrons ;
R⁸ est H ou méthyle ;
R¹⁶ est H, alcoyle ou aralcoyle ayant jusqu'à 15 atomes de carbone ;
B est une nucléobase ; et
n est un nombre entier de 0 à 50 ;
sur un deuxième synthon de Formule II : 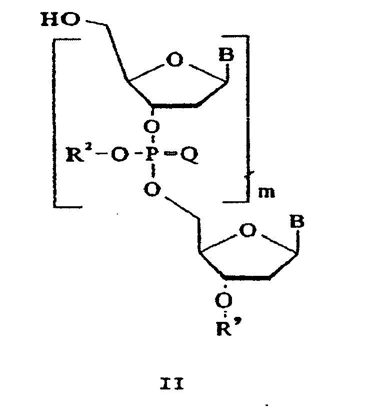
dans laquelle :
R⁹ est un groupe de protection d'un hydroxyle ou un élément de liaison relié à un support solide ; et
m est un nombre entier allant de 0 à 50 ;
pendant une durée et dans des conditions de réaction efficaces pour former un troisième synthon de Formule III : 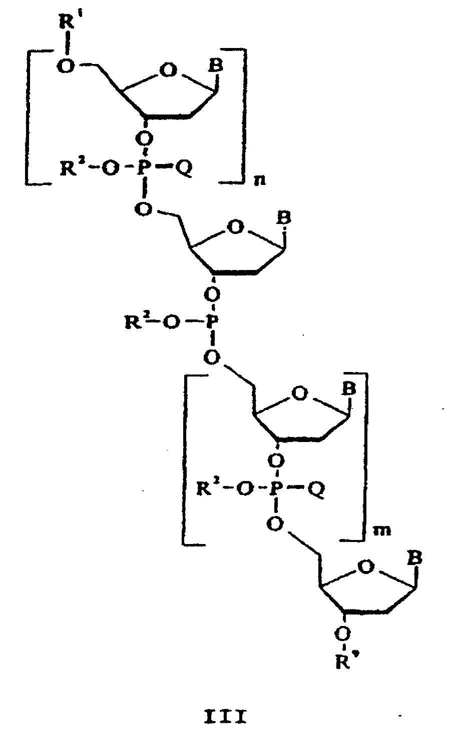 et
- on met le troisième synthon en contact avec un agent de sulfuration pour former un oligomère de Formule IV : 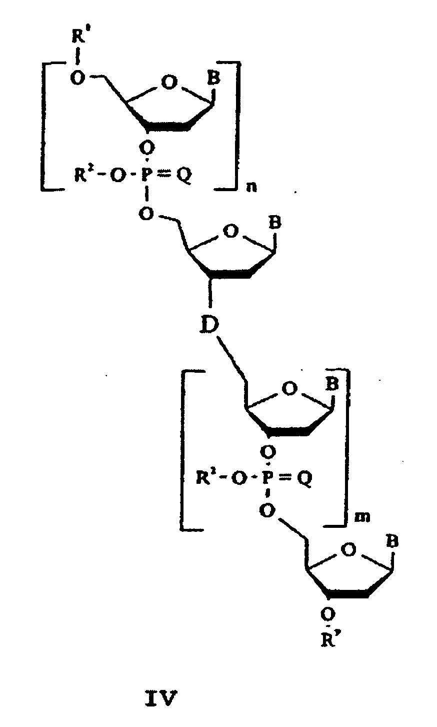
dans laquelle D est une liaison phosphorothioate de formule :

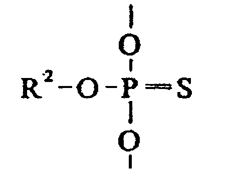

2. Procédé suivant la revendication 1, dans laquelle la liaison phosphorothioate est enrichie diastéréomériquement.

3. Procédé suivant la revendication 1, dans lequel 75 % de la liaison phosphorothioate est sous une forme stéréoisomère unique.

4. Procédé suivant la revendication 1, dans lequel 85 % de la liaison phosphorothioate est sous une forme stéréoisomère unique.

5. Procédé suivant la revendication 1, dans lequel 95 % de la liaison phosphorothioate est sous une forme stéréoisomère unique.

6. Procédé suivant la revendication 1, dans lequel la liaison phosphorothioate est une forme stéréoisomère unique, sensiblement exempte d'autres formes stéréoisomères.

7. Procédé suivant la revendication 1, dans lequel n est 0.

8. Procédé suivant la revendication 1, dans lequel le premier synthon est une forme stéréoisomère unique, sensiblement exempte d'autres formes stéréoisomères.

9. Procédé suivant la revendication 1, comprenant en outre l'élimination du groupe R¹ du phosphorothioate.

10. Procédé suivant la revendication 1, comprenant en outre l'élimination des auxiliaires chiraux.

11. Procédé suivant la revendication 10, dans lequel l'élimination des auxiliaires chiraux s'effectue par l'élimination β.

12. Procédé suivant la revendication 10, dans lequel l'élimination des auxiliaires chiraux s'effectue par un traitement par des réactifs acides.

13. Procédé suivant la revendication 1, dans lequel le composé de Formule IV comporte une pluralité de liaisons de phosphorothioates.

14. Procédé suivant la revendication 1, dans lequel on fait réagir le premier et le deuxième synthon à une température de -20°C à 40°C.

15. Procédé suivant la revendication 1, dans lequel on fait réagir les premier et deuxième synthons à une température de -15°C à 0°C.

16. Procédé suivant la revendication 1, dans lequel on forme le premier synthon en faisant réagir un composé de Formule V : 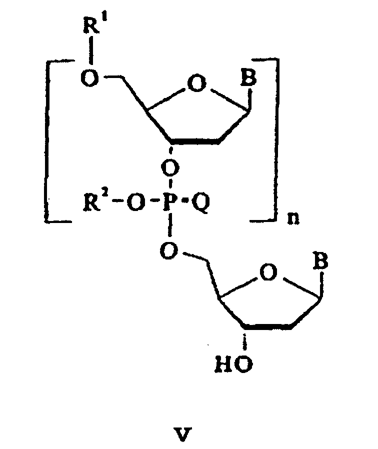 sur un azaphospholane de Formule VIa : 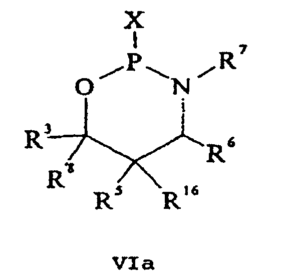 dans lesquelles R³ à R⁸ sont tels que définis ci-dessus ; et X est halogène, dialcoylamino, imidazole, triazole ou phénoxy substitué dont les substituants sont attracteurs d'électrons.

17. Procédé suivant la revendication 16, dans lequel les substituants attirant les électrons sont halogène ou nitro.

18. Procédé suivant la revendication 16, dans lequel on prépare l'azaphospholane en faisant réagir un réactif de formule HO-C(R⁸)R³-C(R¹⁶)R⁵-CHR⁶-NHR⁷ et un trihalogénure de phosphore, un tri(dialcoylamide) de phosphore, un triphénolate de phosphore ou des tri(imidazolides) de phosphore.

19. Procédé suivant la revendication 1, dans lequel on forme le premier synthon en faisant réagir un composé de Formule VII : 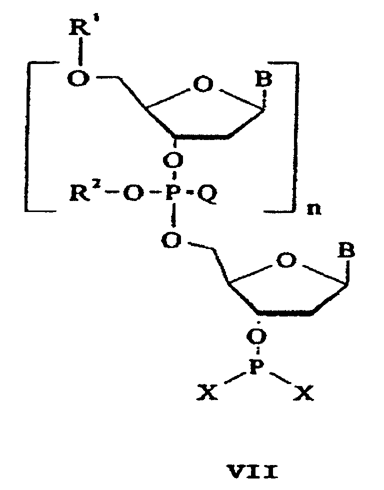 sur un γ-amino alcool de formule HO-C(R⁸)R³-C(R¹⁶)R⁵-CHR⁶-NHR⁷ dans laquelle X et R¹ à R⁸ et R¹⁶ sont tels que définis ci-dessus.

20. Procédé suivant la revendication 19, dans lequel X est le chlore.

21. Procédé suivant la revendication 19, dans lequel la réaction est stéréosélective.

22. Procédé suivant la revendication 20, dans lequel le premier synthon est sous une forme stéréomère unique, sensiblement exempte d'autres formes stéréoisomères.

23. Procédé suivant la revendication 1, dans lequel on effectue la réaction en la présence d'un catalyseur.

24. Procédé suivant la revendication 23, dans lequel le catalyseur a l'une des formules VIII ou IX : 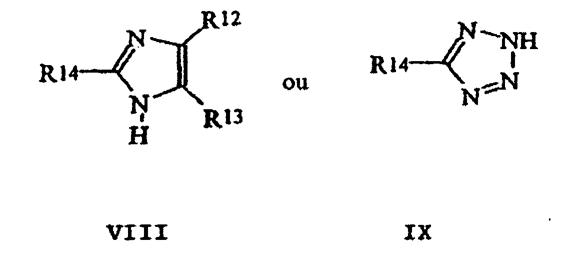 dans lesquelles :
R¹² et R¹³ sont indépendamment hydrogène, halogène, cyano, nitro, alcoyle ayant de 1 à 10 atomes de carbone, alcoyle substitué ayant de 1 à 10 atomes de carbone, un groupe ester, ou R¹² et R¹³ forment ensemble avec les atomes de carbone auxquels ils sont fixés un cycle phényle substitué ou non substitué dont les substituants sont attracteurs d'électrons ; et
R¹⁴ est hydrogène, halogène, cyano, nitro, thio, alcoyle ayant de 1 à 10 atomes de carbone, alcoyle substitué ayant de 1 à 10 atomes de carbone, norbornyle, norbornyle substitué, aryle ayant de 3 à 14 atomes de carbone, aryle substitué ayant de 3 à 14 atomes de carbone, les substituants étant attracteurs d'électrons, ou a la formule : 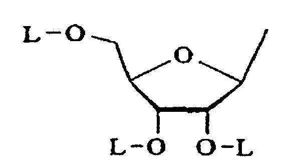
dans laquelle L est un groupe de protection.

25. Procédé suivant la revendication 23, dans lequel R¹⁴ est halogène ou nitro, et dans lequel R¹² et R¹³ sont chacun halogène ou chacun cyano.

26. Procédé suivant la revendication 24, dans lequel R¹⁴ est le brome, et R¹² et R¹³ sont chacun cyano.

27. Procédé suivant la revendication 24, dans lequel R¹⁹, R¹² et R¹³ sont chacun bromo.

28. Procédé suivant la revendication 23, dans lequel R¹⁴ a l'une des formules : 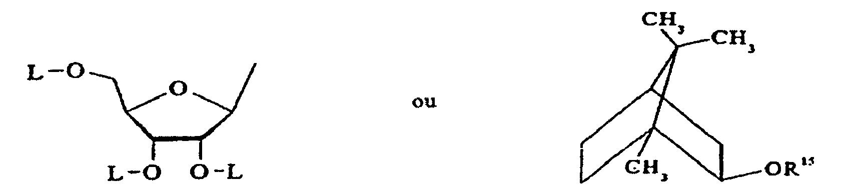 dans lesquelles R¹⁵ est H, méthyle, trialcoylsilyle ou acétyle.

29. Procédé suivant la revendication 1, dans lequel R³ est cyanométhyle ou -CH₂-SOₖR⁴ dans laquelle k est 0, 1 ou 2.

30. Procédé suivant la revendication 29, dans lequel R⁷ est alcoyle ayant de 1 à 6 atomes de carbone ou aralcoyle ayant jusqu'à 15 atomes de carbone.

31. Procédé suivant la revendication 1, dans lequel le premier synthon a l'une des formules Xa, XIa, XIIa, XIIIa ou XXa : 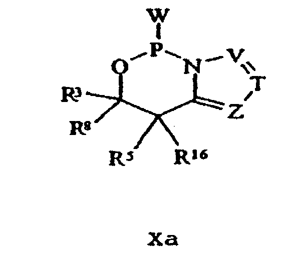 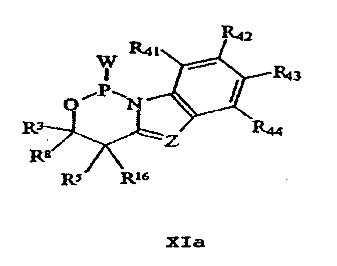 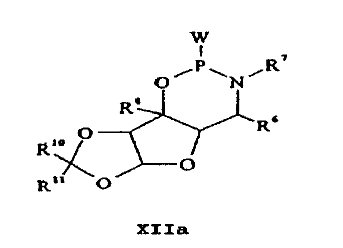 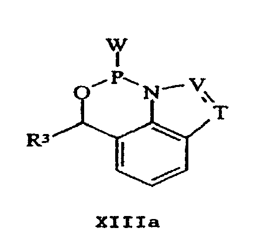 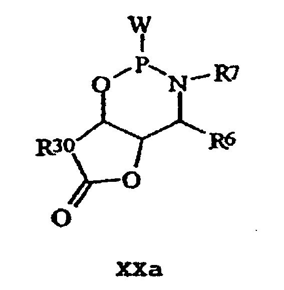 dans laquelle W a la formule : 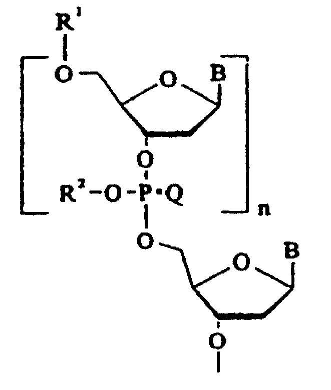 et R¹ à R¹⁶, V, T et Z sont tels que définis ci-dessus.

32. Procédé suivant la revendication 31, dans lequel le premier synthon a la formule XIa ; R³ est -CH₂R₄ ; et R⁴ est indépendamment -(N(R₇₀)-C(=O)-R₇₁, -S-C (=O)-R₇₀ ou -O-C(=O)-O-N(R₇₀) (R₇₁).

33. Procédé suivant la revendication 32, dans lequel R₇₀ et R₇₁ sont indépendamment alcoyle ayant de 1 à 6 atomes de carbone ou alcoyle substitué par un halogène en position α ayant de 1 à 6 atomes de carbone.

34. Procédé suivant la revendication 31, dans lequel le premier synthon a la formule Xb ou Xc :

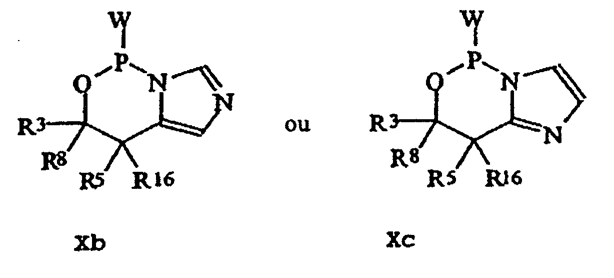

35. Procédé suivant la revendication 31, dans lequel le premier synthon a la Formule XIVa : 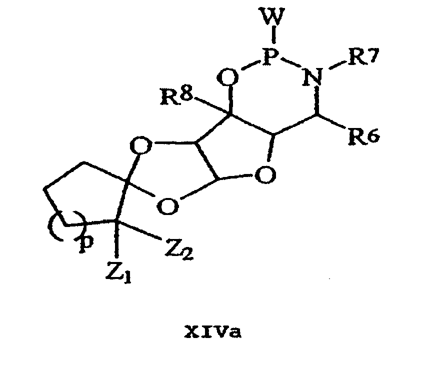 dans laquelle p, Z¹ et Z² sont tels que définis ci-dessus.

36. Procédé suivant la revendication 35, dans lequel le premier synthon a la formule XVa ou XVIa : 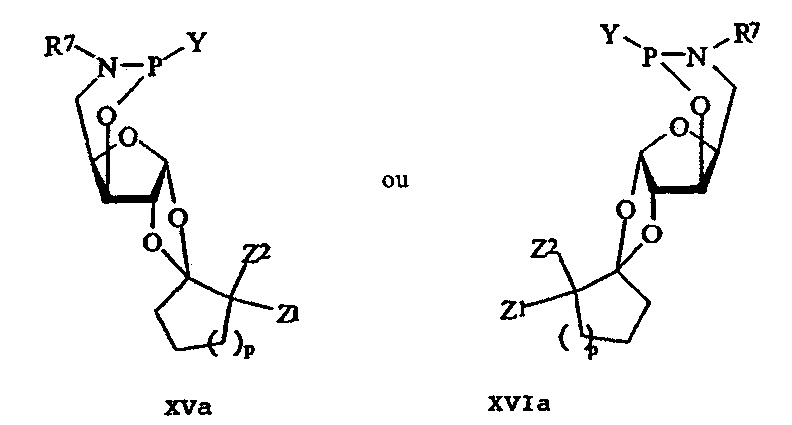 dans laquelle Y, R⁷, Z¹, Z² et p sont tels que définis ci-dessus.

37. Procédé suivant la revendication 31, dans laquelle le premier synthon a la Formule XVIIa ou XVIIIa : 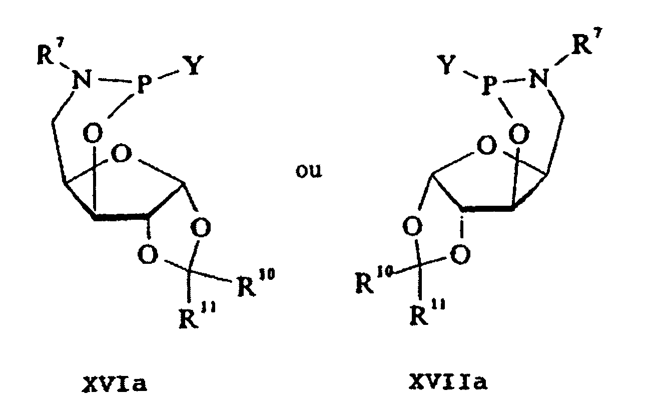 dans lesquelles W, R⁷, R¹⁰ et R¹¹ sont tels que définis ci-dessus.

38. Procédé suivant la revendication 1, comprenant en outre l'élimination des groupes R⁹.

39. Procédé suivant la revendication 1, comprenant en outre l'élimination des groupes R².

40. Procédé de préparation d'une liaison internucléotide comprenant les stades dans lesquels :
on choisit un premier synthon, le premier synthon étant choisi dans le groupe consistant en des nucléosides phosphoramidites et en des nucléosides azaphospholanes ;
on choisit un deuxième synthon de nucléoside ayant un groupe hydroxyle 5' libre ; et
on fait réagir le premier synthon et le deuxième synthon en la présence d'un catalyseur pour former la liaison internucléotide ;
le catalyseur ayant la formule : 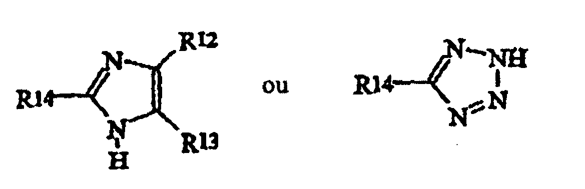
dans lesquelles :
R¹² et R¹³ sont indépendamment hydrogène, halogène, cyano, nitro, alcoyle ayant de 1 à 10 atomes de carbone, alcoyle substitué ayant de 1 à 10 atomes de carbone, un groupe ester, ou R¹² et R¹³ forment ensemble avec les atomes de carbone auxquels ils sont fixés un cycle phényle substitué ou non substitué dont les substituants sont attracteurs d'électrons ; et
R¹⁴ est hydrogène, halogène, cyano, nitro, thio, alcoyle ayant de 1 à 10 atomes de carbone, alcoyle substitué ayant de 1 à 10 atomes de carbone, norbornyle, norbornyle substitué, aryle, aryle substitué dont les substituants sont attracteurs d'électrons, ou a la formule : 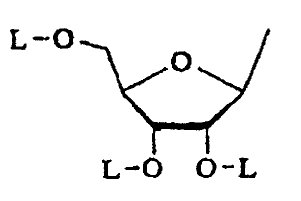
dans laquelle L est un groupe de protection.

41. Procédé suivant la revendication 40, dans lequel la liaison d'internucléotide est une liaison phosphite.

42. Procédé suivant la revendication 40, comprenant en outre le stade d'oxydation de la liaison d'internucléotide pour former une liaison phosphodiester, une liaison phosphorothioate ou une liaison phosphorodithioate.

43. Procédé suivant la revendication 23 ou la revendication 40, dans lequel le catalyseur est le 2-bromo-4,5-dicyanoimidazole.

44. Oligomère de Formule IV suivant la revendication 1 : 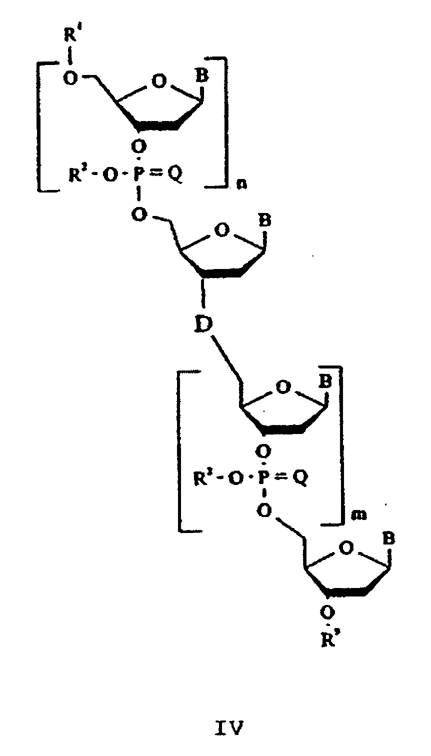 dans laquelle D est une liaison phosphorothioate de formule :

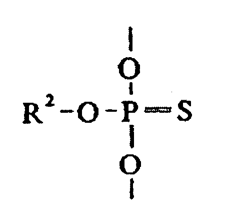

45. Azaphospholane de Formule VIb : 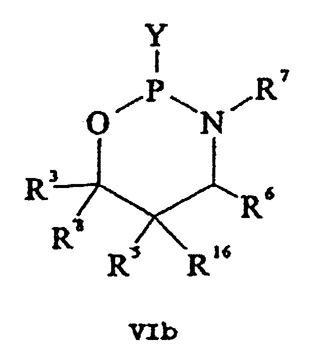 dans laquelle :
Y est X ou W, X étant imidazole, triazole ou phénoxy substitué dont les substituants sont attracteurs d'électrons, et W a la formule : 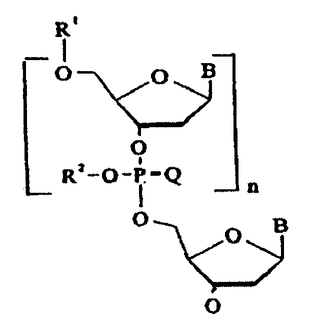 dans laquelle :
Q est indépendamment O ou S ;
R¹ est un groupe de protection d'un hydroxyle ;
R² est un auxiliaire chiral de formule -C(R⁸)R³-C(R¹⁶)R⁵-CHR⁶-NHR⁷ ;
R³ est hydrogène, alcoyle ayant de 1 à 6 atomes de carbone, cyanométhyle, monohalométhyle, dihalométhyle, trihalométhyle, -CH₂R₄, -CH₂Si (R⁴) ₃ ou -CH₂-SOₖR⁴ dans laquelle k est 0, 1 ou 2 ;
R⁴ est indépendamment alcoyle, aryle, aralcoyle ou alcoylaryle ayant jusqu'à 15 atomes de carbone, -N(R₇₀)-C(=O)-R₇₁, -S-C(=O)-R₇₀ ou -O-C(=O)-O-N(R₇₀) (R₇₁);
R₇₀ et R₇₁ sont chacun indépendamment alcoyle ayant de 1 à 6 atomes de carbone, alcoyle substitué en position α par un halogène et ayant de 1 à 6 atomes de carbone, aralcoyle ayant de 4 à 20 atomes de carbone, aralcoyle substitué en position α par un halogène et ayant de 4 à 20 atomes de carbone, ou aryle ayant de 3 à 14 atomes de carbone, substitué par jusqu'à trois groupes attracteurs d'électrons ;
R⁵ est H, -CN, -Si(R⁴)₃, SOₖR⁴ ou halogène ;
ou R⁸ et R¹⁶ sont chacun H, et R³ et R⁵ forment ensemble l'une des structures 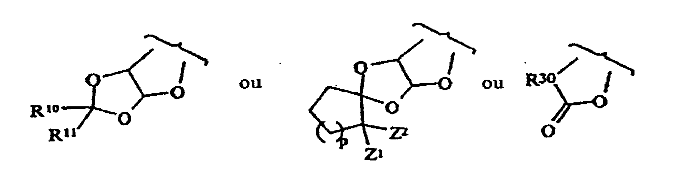
dans lesquelles :
R¹⁰ et R¹¹ sont H, alcoyle ayant de 1 à 10 atomes de carbone, -CH₂C (=O) OR²², -CH₂CN, -CH₂Si (CH₃) ou *o*-C₆H₄-R²¹ ou *p*-C₆H₄-R²¹;
R²¹ est hydrogène, -O-C (=O) CH₃, alcoxy ayant de 1 à 10 atomes de carbone, -NO₂ ou -N(R²²)₂ ;
R²² est indépendamment H ou alcoyle ayant de 1 à 10 atomes de carbone ;
p est 1 ou 2;
Z¹ et Z² sont indépendamment halogène, CN, -Si(CH₃)₃ et -C(=O)OR²²;
R³⁰ est hydrogène, -O-C(=O)CH₃, alcoxy ayant de 1 à 10 atomes de carbone ou -O-Si(R₄)₃ ;
R⁶ est H, alcoyle ou aralcoyle ayant jusqu'à 15 atomes de carbone ;
ou R⁵ et R⁶ forment ensemble avec les atomes auxquels ils sont fixés un cycle à 5 ou 6 chaînons ;
R⁷ est alcoyle ou aralcoyle ayant jusqu'à 15 atomes de carbone ;
ou R⁶ et R⁷ forment ensemble l'une des structures 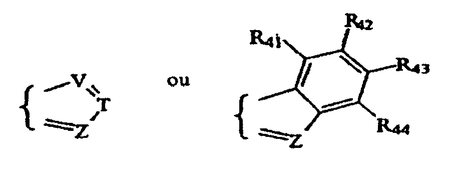
dans lesquelles X, T et Z sont indépendamment CH ou NH ; ou Z et R⁵ forment ensemble avec les atomes auxquels ils sont fixés un cycle phényle ;
R⁸ est H ou méthyle ;
R⁹ est un groupe de protection d'un hydroxyle ou un support solide ;
R¹⁶ est H, alcoyle ou aralcoyle ayant jusqu'à 15 atomes de carbone ;
B est une nucléobase ; et
n est un nombre entier allant de 0 à 50.

46. Azaphospholane suivant la revendication 45, dans lequel Y est W et n est 0.

47. Azaphospholane suivant la revendication 45, dans lequel R⁷ est alcoyle ayant jusqu'à 15 atomes de carbone.

48. Azaphospholane suivant la revendication 45, dans lequel R⁷, R⁶ et les atomes auxquels ils sont fixés forment un cycle à 5 ou 6 chaînons.

49. Azaphospholane suivant la revendication 46, dans lequel R³ est cyanométhyle ou -CH₂-SOₖR⁴ dans laquelle k est 0, 1 ou 2.

50. Azaphospholane suivant la revendication 45 ayant l'une des Formules XVIc ou XVId :

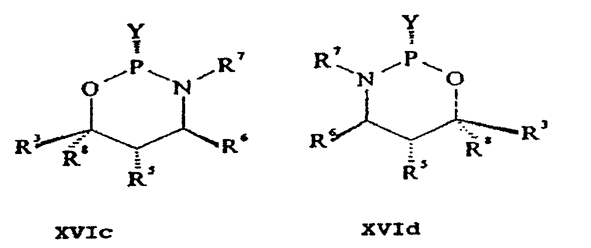

51. Azaphospholane suivant la revendication 45 ayant l'une des Formules Xb, XIb, XIIb, XIIIb ou XXb : 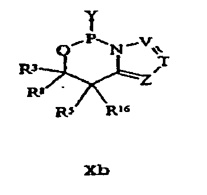 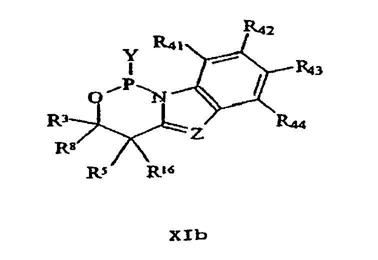 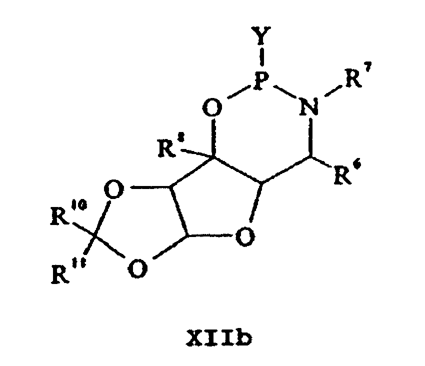 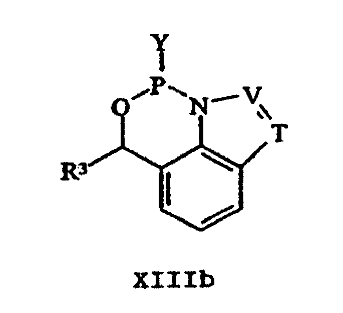 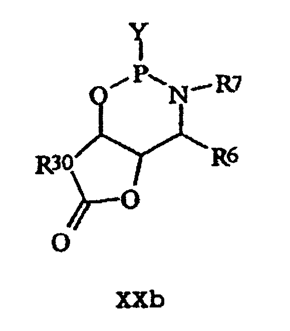 dans lesquelles R³ à R¹⁶, Y, V, T, Z, Z₁, Z₂ et p sont tels que définis ci-dessus.

52. Azaphosphalane suivant la revendication 51 ayant la Formule XIa.

53. Azaphosphalane suivant la revendication 52, dans lequel R³ est -CH₂R₄ ; et R⁴ est indépendamment -N(R₇₀)-C(=O)-R₇₁,-S-C(=O)-R₇₀ ou -O-C(=O)-O-N(R₇₀)(R₇₁);

54. Azaphosphalane suivant la revendication 52, dans lequel R₇₀ et R₇₁ sont indépendamment alcoyle ayant de 1 à 6 atomes de carbone ou alcoyle substitué en position α par un halogène ayant de 1 à 6 atomes de carbone.

55. Azaphosphalane suivant la revendication 51 ayant la Formule XIVb : 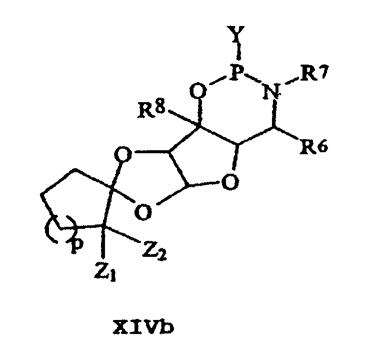 dans laquelle Y, R⁶, R⁷ R⁸, p, Z¹ et Z² sont tels que définis ci-dessus.

56. Azaphosphalane suivant la revendication 50 ayant la Formule Xd ou Xe : 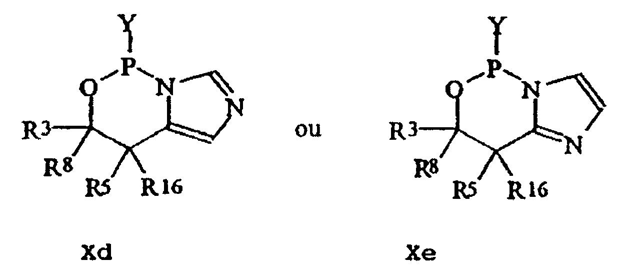 dans lesquelles Y, R³, R⁵, R⁸ et R¹⁶ sont tels que définis ci-dessus.

57. Azaphospholane suivant la revendication 51 ayant la Formule XVIIb ou XVIIIb : 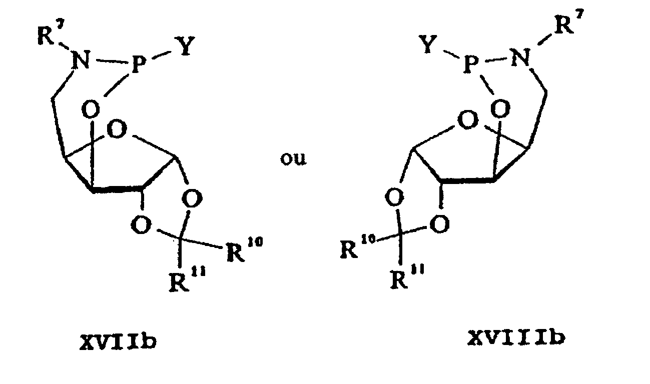 dans lesquelles Y, R⁷, R¹⁰ et R¹¹ sont tels que définis ci-dessus.

58. Azaphospholane suivant la revendication 49 ayant la Formule XVb ou XVIb : 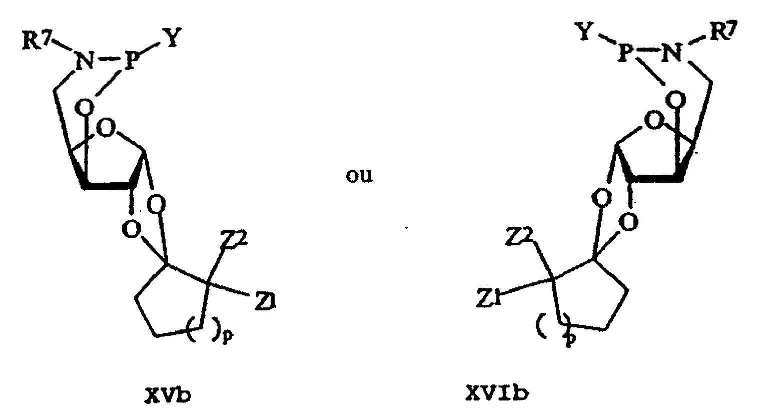 dans lesquelles Y, R⁷, Z¹, Z² et p sont tels que définis ci-dessus.

59. Azaphospholane suivant la revendication 45, dans lequel l'azaphospholane est enrichi diastéréomériquement.

60. Azaphospholane suivant la revendication 45, dans lequel 75 % de l'azaphospholane est sous une forme stéréoisomère unique.

61. Azaphospholane suivant la revendication 45, dans lequel 85 % de l'azaphospholane est sous une forme stéréoisomère unique.

62. Azaphospholane suivant la revendication 45, dans lequel 95 % de l'azaphospholane est sous une forme stéréoisomère unique.

63. Azaphospholane suivant la revendication 45, dans lequel l'azaphospholane est une forme stéréoisomère unique, sensiblement exempte d'autres formes stéréoisomères.

64. Composé oligomère de Formule III suivant la revendication 1, ayant une liaison phosphite de Formule XXX : 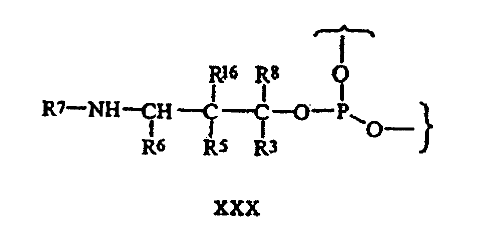 dans laquelle :
R³, R⁵ à R⁸ et R¹⁶ sont tels que définis suivant la revendication 1.

65. Composé oligomère suivant la revendication 64, dans lequel 75 % de la liaison phosphite est une forme stéréoisomère unique.

66. Composé oligomère suivant la revendication 64, dans lequel 85 % de la liaison phosphite est une forme stéréoisomère unique.

67. Composé oligomère suivant la revendication 64, dans lequel 95 % de la liaison phosphite est une forme stéréoisomère unique.

68. Composé oligomère suivant la revendication 64, dans lequel la liaison phosphite est une forme stéréoisomère unique, sensiblement exempte d'autres formes stéréoisomères.
